# EUROPEAN PATENT SPECIFICATION

(11) **EP 2 326 722 B1**
(45) Date of publication and mention of the grant of the patent: **06.08.2014**
(21) Application number: 09785101.8
(22) Date of filing: 14.09.2009
(51) Int. Cl.: C12N 15/82, C12N 15/54

(54) **HERBICIDE TOLERANT PLANTS**
HERBIZIDTOLERANTE PFLANZEN
PLANTES TOLÉRANTES AUX HERBICIDES

(30) Priority: 15.09.2008 GB 0816880
(43) Date of publication of application: 01.06.2011
(60) Divisional application: 14173972.2
(73) Proprietor: Syngenta Limited, Guildford Surrey GU2 7YH (GB)
(72) Inventor: HAWKES, Timothy, Robert, Bracknell Berkshire (GB); DRAYTON, Paul, Richard, Bracknell Berkshire (GB); DALE, Richard, Bracknell Berkshire (GB)
(74) Representative: Syngenta International AG
(86) International application number: PCT/GB2009/002188
(87) International publication number: WO 2010/029311

(56) References cited:
- WO-A-03/047547
- WO-A-2004/044173
- WO-A-2005/054453
- WO-A-2007/024782
- WO-A2-03/080647
- US-A1- 2008 146 447
- TIAN LI ET AL: "The pds2 mutation is a lesion in the Arabidopsis homogentisate solanesyltransferase gene involved in plastoquinone biosynthesis" PLANTA (BERLIN), vol. 226, no. 4, September 2007 (2007-09), pages 1067-1073, XP002561518 ISSN: 0032-0935
- NORRIS S R BARRETTE T R DELLAPENNA D: "Genetic dissection of carotenoid synthesis in arabidopsis defined plastoquinone as an essential component of phytoene desaturation" PLANT CELL, AMERICAN SOCIETY OF PLANT PHYSIOLOGISTS, ROCKVILLE, MD, US, vol. 7, 1 December 1995 (1995-12-01), pages 2139-2149, XP002953798 ISSN: 1040-4651
- SADRE R ET AL: "Characterization of homogentisate prenyltransferases involved in plastoquinone-9 and tocochromanol biosynthesis" FEBS LETTERS, ELSEVIER, AMSTERDAM, NL, vol. 580, no. 22, 2 October 2006 (2006-10-02), pages 5357-5362, XP025232598 ISSN: 0014-5793 [retrieved on 2006-10-02]
- TYAMAGONDLU V VENKATESH ET AL: "Identification and characterization of an Arabidopsis homogentisate phytyltransferase paralog" PLANTA ; AN INTERNATIONAL JOURNAL OF PLANT BIOLOGY, SPRINGER, BERLIN, DE, vol. 223, no. 6, 12 January 2006 (2006-01-12), pages 1134-1144, XP019427417 ISSN: 1432-2048
- MIRELA RACLARU ET AL: "Increase of the tocochromanol content in transgenic Brassica napus seeds by overexpression of key enzymes involved in prenylquinone biosynthesis", MOLECULAR BREEDING, KLUWER ACADEMIC PUBLISHERS, DO, vol. 18, no. 2, 29 August 2006 (2006-08-29), pages 93-107, XP019403953, ISSN: 1572-9788, DOI: 10.1007/S11032-006-9014-5

## Description

The present invention relates to methods for selectively controlling weeds at a locus. The invention further relates to recombinant DNA technology, and in particular to the production of transgenic plants which exhibit substantial resistance or substantial tolerance to herbicides when compared with non transgenic like plants. Plants which are substantially "tolerant" to a herbicide when they are subjected to it provide a dose/response curve which is shifted to the right when compared with that provided by similarly subjected non tolerant like plants. Such dose/response curves have "dose" plotted on the x-axis and "percentage kill", "herbicidal effect" etc. plotted on the y-axis. Tolerant plants will typically require at least twice as much herbicide as non tolerant like plants in order to produce a given herbicidal effect. Plants which are substantially "resistant" to the herbicide exhibit few, if any, necrotic, lytic, chlorotic or other lesions when subjected to the herbicide at concentrations and rates which are typically employed by the agricultural community to kill weeds in the field.

More particularly, the present invention relates to the production of plants that are resistant to herbicides that inhibit hydroxyphenyl pyruvate dioxygenase (HPPD) and/ or herbicides that inhibit the subsequent, homogentisate solanesyl transferase (HST) step in the pathway to plastoquinone.

Herbicides that act by inhibiting HPPD are well known in the art. Inhibition of HPPD blocks the biosynthesis of plastoquinone (PQ) from tyrosine. PQ is an essential cofactor in the biosynthesis of carotenoid pigments which are essential for photoprotection of the photosynthetic centres. HPPD-inhibiting herbicides are phloem-mobile bleachers which cause the light-exposed new meristems and leaves to emerge white where, in the absence of carotenoids, chlorophyll is photo-destroyed and becomes itself an agent of photo-destruction via the photo-generation of singlet oxygen. Methods for production of transgenic plants which exhibit substantial resistance or substantial tolerance to HPPD-inhibiting herbicides have been reported-for example WO02/46387.

The enzyme catalysing the following step from HPPD in the plastoquinone biosynthesis pathway is HST. The HST enzyme is a prenyl tranferase that both decarboxylates homogentisate and also transfers to it the solanesyl group from solanesyl diphosphate and thus forms 2-methyl-6-solanesyl-1,4-benzoquinol (MSBQ), an intermediate along the biosynthetic pathway to plastoquinone. HST enzymes are membrane bound and the genes that encode them include a plastid targeting sequence. Methods for assaying HST have recently been disclosed.

Over expression of HST in transgenic plants has been reported, for example in WO03047547 and WO03080647, and said plants are said to exhibit slightly higher concentrations of α-tocopherol. However, it has not hitherto been recognised that HST is the target site for certain classes of herbicidal compounds - which act wholly or in part by inhibiting HST. Furthermore, it has now been found, *inter alia,* that over expression of HST in a transgenic plant provides tolerance to HST-inhibiting and/or HPPD-inhibiting herbicides.

Thus, according to the present invention there is provided a method of selectively controlling weeds at a locus comprising crop plants and weeds, wherein the method comprises application to the locus of a weed controlling amount of a pesticide composition comprising an homogentisate solanesyltransferase (HST) inhibiting herbicide selected from the group consisting of Formula (IIa), (IIb), (IIc), (IId), (IIe) and (IIf) as defined below and/or hydroxyphenyl pyruvate dioxygenase (HPPD) inhibiting herbicide, wherein the crop plants comprise at least one heterologous polynucleotide which comprises a region which encodes an HST and which provides over expression of said HST in said crop plants. In a preferred embodiment of the method the crop plants further comprise an additional heterologous polynucleotide which comprises a region which encodes a hydroxyphenyl pyruvate dioxygenase (HPPD).

The invention still further provides a method of selectively controlling weeds at a locus comprising crop plants and weeds, wherein the method comprises application to the locus of a weed controlling amount of a pesticide composition comprising an HST-inhibiting herbicide, wherein the crop plants comprise at least one heterologous polynucleotide which comprises a region which encodes a HPPD enzyme.

In a preferred embodiment the pesticide composition referred to in the aforementioned methods comprises both an HST-inhibiting herbicide and an HPPD-inhibiting herbicide

For the purposes of the present invention - an HST inhibiting herbicide is one which itself, or as a procide generates a molecule that inhibits *Arabidopsis* HST exhibits an IC50 less than 150 ppm, preferably less than 60 ppm using the "total extract" assay method as set out herein. It should be appreciated that the HST inhibiting herbicides may also act as a HPPD inhibitors (possible to identify using, for example, HPPD enzyme assays and/or the differential responses of HPPD or HST over expressing transgenic plant lines) and, therefore, as shown below, self-synergise the effect of their inhibition of HST. Preferably the HST inhibiting herbicide is selected from the group consisting of a compound of formula (IIa) 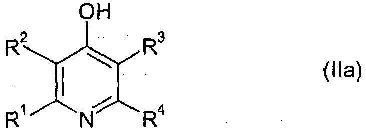 wherein
R¹, R², R³ and R⁴ are independently hydrogen or halogen; provided that at least three of R¹, R², R³ and R⁴ are halogen; or salts thereof;
a compound of formula (IIb) 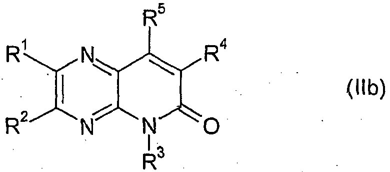 wherein
R¹ and R² are independently hydrogen, C₁-C₄alkyl, C₁-C₄haloalkyl, halo, cyano, hydroxy, C₁-C₄alkoxy, C₁-C₄alkylthio, aryl or aryl substituted by one to five R⁶, which may be the same or different, or heteroaryl or heteroaryl substituted by one to five R⁶, which may be the same or different;
R³ is hydrogen, C₁-C₁₀alkyl, C₂-C₁₀alkenyl, C₂-C₁₀alkynyl, C₃-C₁₀cycloalkyl, C₃-C₁₀cycloalkyl-C₁-C₆alkyl-, C₁-C₁₀alkoxy-C₁-C₆alkyl-, C₁-C₁₀cyanoalkyl-, C₁-C₁₀alkoxycarbonyl-C₁-C₆alkyl-, *N*-C₁-C₃alkyl-aminocarbonyl-C₁-C₆alkyl-, *N*,*N*-di-(C₁-C₃alkyl)-aminocarbonyl-C₁-C₆alkyl-, aryl-C₁-C₆alkyl- or aryl-C₁-C₆alkyl-wherein the aryl moiety is substituted by one to three R⁷, which may be the same or different, or heterocyclyl-C₁-C₆alkyl- or heterocyclyl-C₁-C₆alkyl- wherein the heterocyclyl moiety is substituted by one to three R⁷, which may be the same or different;
R⁴ is aryl or aryl substituted by one to five R⁸, which may be the same or different, or heteroaryl or heteroaryl substituted by one to four R⁸, which may be the same or different;
R⁵ is hydroxy, R⁹-oxy-, R¹⁰-carbonyloxy-, tri-R¹¹-silyloxy- or R¹²-sulfonyloxy-, each R⁶, R⁷ and R⁸ is independently halo, cyano, nitro, C₁-C₁₀alkyl, C₁-C₄haloalkyl, C₂-C₁₀alkenyl, C₂-C₁₀alkynyl, hydroxy, C₁-C₁₀alkoxy, C₁-C₄haloalkoxy, C₁-C₁₀alkoxy-C₁-C₄alkyl-, C₃-C₇cycloalkyl, C₃-C₇cycloalkoxy, C₃-C₇cycloalkyl-C₁-C₄alkyl-, C₃-C₇cycloalkyl-C₁-C₄alkoxy-, C₁-C₆alkylcarbonyl-, fonnyl, C₁-C₄alkoxy-carbonyl-, C₁-C₄alkylcarbonyloxy-, C₁-C₁₀alkylthio-, C₁-C₄haloalkylthio-, C₁-C₁₀alkylsulfinyl-, C₁-C₄haloalkylsulfinyl-, C₁-C₁₀alkylsulfonyl-, C₁-C₄haloalkylsulfonyl-, amino, C₁-C₁₀alkylamino-, di-C₁-C₁₀alkylamino-, C₁-C₁₀alkylcarbonylamino-, aryl or aryl substituted by one to three R¹³, which may be the same or different, heteroaryl or heteroaryl substituted by one to three R¹³, which may be the same or different, aryl-C₁-C₄alkyl- or aryl-C₁-C₄alkyl- wherein the aryl moiety is substituted by one to three R¹³, which maybe the same or different, heteroaryl-C₁-C₄alkyl- or heteroaryl-C₁-C₄alkyl- wherein the heteroaryl moiety is substituted by one to three R¹³, which may be the same or different, aryloxy- or aryloxy- substituted by one to three R¹³, which may be the same or different, heteroaryloxy- or heteroaryloxy-substituted by one to three R¹³, which may be the same or different, arylthio- or arylthio- substituted by one to three R¹³, which may be the same or different, or heteroarylthio- or heteroarylthio- substituted by one to three R¹³, which may be the same or different;
R⁹ is C₁-C₁₀alkyl, C₂-C₁₀alkenyl, C₂-C₁₀alkynyl or aryl-C₁-C₄alkyl- or aryl-C₁-C₄alkyl- wherein the aryl moiety is substituted by one to five substituents independently selected from halo, cyano, nitro, C₁-C₆alkyl, C₁-C₆haloalkyl or C₁-C₆alkoxy;
R¹⁰ is C₁-C₁₀alkyl, C₃-C₁₀cycloalkyl, C₃-C₁₀cycloalkyl-C₁-C₁₀alkyl-, C₁-C₁₀haloalkyl, C₂-C₁₀alkenyl, C₂-C₁₀alkynyl, C₁-C₄alkoxy-C₁-C₁₀alkyl-, C₁-C₄alkylthio-C₁-C₄alkyl-, C₁-C₁₀alkoxy, C₂-C₁₀alkenyloxy, C₂-C₁₀alkynyloxy, C₁-C₁₀alkylthio-, *N*-C₁-C₄alkyl-amino-, *N,N*-di-(C₁-C₄alkyl)-amino-, aryl or aryl substituted by one to three R¹⁴, which may be the same or different, heteroaryl or heteroaryl substituted by one to three R¹⁴, which may be the same or different, aryl-C₁-C₄alkyl- or aryl-C₁-C₄alkyl-wherein the aryl moiety is substituted by one to three R¹⁴, which may be the same or different, heteroaryl-C₁-C₄alkyl- or heteroaryl-C₁-C₄alkyl- wherein the heteroaryl moiety is substituted by one to three R¹⁴, which may be the same or different, aryloxy-or aryloxy- substituted by one to three R¹⁴, which may be the same or different, heteroaryloxy- or heteroaryloxy- substituted by one to three R¹⁴, which may be the same or different, arylthio- or arylthio- substituted by one to three R¹⁴*,* which may be the same or different, or heteroarylthio- or heteroarylthio- substituted by one to three R¹⁴, which may be the same or different;
each R¹¹ is independently C₁-C₁₀alkyl or phenyl or phenyl substituted by one to five substituents independently selected from halo, cyano, nitro, C₁-C₆alkyl, C₁-C₆haloalkyl or C₁-C₆alkoxy;
R¹² is C₁-C₁₀alkyl or phenyl or phenyl substituted by one to five substituents independently selected from halo, cyano, nitro, C₁-C₆alkyl, C₁-C₆haloalkyl or C₁-C₆alkoxy;
each R¹³ is independently halo, cyano, nitro, C₁-C₆alkyl, C₁-C₆haloalkyl or C₁-C₆alkoxy; and
each R¹⁴ is independently halo, cyano, nitro, C₁-C₁₀alkyl, C₁-C₄haloalkyl, C₁-C₁₀alkoxy, C₁-C₄alkoxycarbonyl-, C₁-C₄haloalkoxy, C₁-C₁₀alkylthio-, C₁-C₄haloalkylthio-, C₁-C₁₀alkylsulfinyl-, C₁-C₄haloalkylsulfinyl-, C₁-C₁₀alkylsulfonyl-, C₁-C₄haloalkylsulfonyl-, aryl or aryl substituted by one to five substituents independently selected from halo, cyano, nitro, C₁-C₆alkyl, C₁-C₆haloalkyl or C₁-C₆alkoxy, or heteroaryl or heteroaryl substituted by one to four substituents independently selected from halo, cyano, nitro, C₁-C₆alkyl, C₁-C₆haloalkyl or C₁-C₆alkoxy; or salts or *N*-oxides thereof;
a compound of formula (IIc) 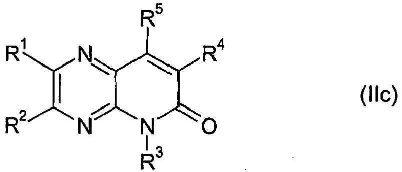 wherein
R¹ and R² are independently hydrogen, C₁-C₄alkyl, C₁-C₄haloalkyl, halo, cyano, hydroxy, C₁-C₄alkoxy, C₁-C₄alkylthio, aryl or aryl substituted by one to five R⁶, which may be the same or different, or heteroaryl or heteroaryl substituted by one to five R⁶, which may be the same or different;
R³ is C₁-C₄haloalkyl, C₂-C₄haloalkenyl or C₂-C₄haloalkynyl;
R⁴ is aryl or aryl substituted by one to five R⁸, which may be the same or different, or heteroaryl or heteroaryl substituted by one to four R⁸, which may be the same or different;
R⁵ is hydroxy or a group which can be metabolised to the hydroxy group;
each R⁶ and R⁸ is independently halo, cyano, nitro, C₁-C₁₀alkyl, C₁-C₄haloalkyl, C₂-C₁₀alkenyl, C₂-C₁₀alkynyl, hydroxy, C₁-C₁₀alkoxy, C₁-C₄haloalkoxy, C₁-C₁₀alkoxy-C₁-C₄alkyl-, C₃-C₇cycloalkyl, C₃-C₇cycloalkoxy, C₃-C₇cycloalkyl-C₁-C₄alkyl-, C₃-C₇cycloalkyl-C₁-C₄alkoxy-, C₁-C₆alkylcarbonyl-, formyl, C₁-C₄alkoxycarbonyl-, C₁-C₄alkylcarbonyloxy-, C₁-C₁₀alkylthio-, C₁-C₄haloalkylthio-, C₁-C₁₀alkylsulfinyl-, C₁-C₄haloalkylsulfinyl-, C₁-C₁₀alkylsulfonyl-, C₁-C₄haloalkylsulfonyl-, amino, C₁-C₁₀alkylamino-, di-C₁-C₁₀alkylamino-, C₁-C₁₀alkylcarbonylamino-, aryl or aryl substituted by one to three R¹³, which maybe the same or different, heteroaryl or heteroaryl substituted by one to three R¹³, which maybe the same or different, aryl-C₁-C₄alkyl- or aryl-C₁-C₄alkyl- wherein the aryl moiety is substituted by one to three R¹³, which may be the same or different, heteroaryl-C₁-C₄alkyl- or heteroaryl-C₁-C₄alkyl- wherein the heteroaryl moiety is substituted by one to three R¹³, which may be the same or different, aryloxy- or aryloxy- substituted by one to three R¹³, which may be the same or different, heteroaryloxy- or heteroaryloxy- substituted by one to three R¹³, which may be the same or different, arylthio- or arylthio- substituted by one to three R¹³, which may be the same or different, or heteroarylthio- or heteroarylthio-substituted by one to three R¹³, which may be the same or different; and each R¹³ is independently halo, cyano, nitro, C₁-C₆alkyl, C₁-C₆haloalkyl or C₁-C₆alkoxy; or a salt or *N*-oxide thereof;
a compound of formula (IId) 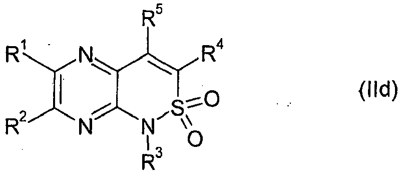 wherein
R¹ and R² are independently hydrogen, C₁-C₄alkyl, C₁-C₄haloalkyl, halo, cyano, hydroxy, C₁-C₄alkoxy, C₁-C₄alkylthio, aryl or aryl substituted by one to five R⁶, which may be the same or different, or heteroaryl or heteroaryl substituted by one to five R⁶, which may be the same or different;
R³ is hydrogen, C₁-C₁₀alkyl, C₁-C₄haloalkyl, C₂-C₁₀alkenyl, C₂-C₄haloalkenyl, C₂-C₁₀alkynyl, C₂-C₄haloalkynyl, C₃-C₁₀cycloalkyl, C₃-C₁₀cycloalkyl-C₁-C₆alkyl-, C₁-C₁₀alkoxy-C₁-C₆alkyl-, C₁-C₁₀cyanoalkyl-, C₁-C₁₀alkoxycarbonyl-C₁-C₆alkyl-, *N-*C₁-C₃alkyl-aminocarbonyl-C₁-C₆alkyl-, *N,N*-di-(C₁-C₃alkyl)-aminocarbonyl-C₁-C₆alkyl-, aryl-C₁-C₆alkyl- or aryl-C₁-C₆alkyl- wherein the aryl moiety is substituted by one to three R⁷, which may be the same or different, or heterocyclyl-C₁-C₆alkyl- or heterocyclyl-C₁-C₆alkyl- wherein the heterocyclyl moiety is substituted by one to three R⁷, which may be the same or different;
R⁴ is aryl or aryl substituted by one to five R⁸, which may be the same or different, or heteroaryl or heteroaryl substituted by one to four R⁸, which may be the same or different;
R⁵ is hydroxy or a group which can be metabolised to the hydroxy group;
each R⁶, R⁷ and R⁸ is independently halo, cyano, nitro, C₁-C₁₀alkyl, C₁-C₄haloalkyl, C₂-C₁₀alkenyl, C₂-C₁₀alkynyl, hydroxy, C₁-C₁₀alkoxy, C₁-C₄haloalkoxy, C₁-C₁₀alkoxy-C₁-C₄alkyl-, C₃-C₇cycloalkyl, C₃-C₇cycloalkoxy, C₃-C₇cycloalkyl-C₁-C₄alkyl-, C₃-C₇cycloalkyl-C₁-C₄alkoxy-, C₁-C₆alkylcarbonyl-, formyl, C₁-C₄alkoxy-carbonyl-, C₁-C₄alkylcarbonyloxy-, C₁-C₁₀alkylthio-, C₁-C₄haloalkylthio-, C₁-C₁₀alkylsulfinyl-, C₁-C₄haloalkylsulfinyl-, C₁-C₁₀alkylsulfonyl-, C₁-C₄haloalkylsulfonyl-, amino, C₁-C₁₀alkylamino-, di-C₁-C₁₀alkylamino-, C₁-C₁₀alkylcarbonylamino-, aryl or aryl substituted by one to three R¹³, which may be the same or different, heteroaryl or heteroaryl substituted by one to three R¹³, which may be the same or different, aryl-C₁-C₄alkyl- or aryl-C₁-C₄alkyl- wherein the aryl moiety is substituted by one to three R¹³, which may be the same or different, heteroaryl-C₁-C₄alkyl- or heteroaryl-C₁-C₄alkyl- wherein the heteroaryl moiety is substituted by one to three R¹³, which may be the same or different, aryloxy- or aryloxy- substituted by one to three R¹³, which may be the same or different, heteroaryloxy- or heteroaryloxy-substituted by one to three R¹³, which may be the same or different, arylthio- or arylthio- substituted by one to three R¹³, which may be the same or different, or heteroarylthio- or heteroarylthio- substituted by one to three R¹³, which may be the same or different; and
each R¹³ is independently halo, cyano, nitro, C₁-C₆alkyl, C₁-C₆haloalkyl or C₁-C₆alkoxy; or a salt or *N*-oxide thereof;
IIe) a compound of formula (IIe) 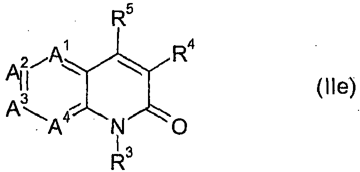 wherein
A¹, A², A³ and A⁴ are independently C-R¹ or N, provided at least one of A¹, A², A³ and A⁴ is N, and provided that if A¹ and A⁴ are both N, A² and A³ are not both C-R¹; each R¹ is independently hydrogen, C₂-C₄alkyl, C₁-C₄haloalkyl, halo, cyano, hydroxy, C₁-C₄alkoxy, C₁-C₄alkylthio, aryl or aryl substituted by one to five R⁶, which may be the same or different, or heteroaryl or heteroaryl substituted by one to five R⁶, which may be the same or different;
R³ is hydrogen, C₁-C₁₀alkyl, C₁-C₄haloalkyl, C₂-C₁₀alkenyl, C₂-C₄haloalkenyl, C₂-C₁₀alkynyl, C₂-C₄haloalkynyl, C₃-C₁₀cycloalkyl, C₃-C₁₀cycloalkyl-C₁-C₆alkyl-, C₁-C₁₀alkoxy-C₁-C₆alkyl-, C₁-C₁₀cyanoalkyl-, C₁-C₁₀alkoxycarbonyl-C₁-C₆alkyl-, *N-*C₁-C₃alkyl-aminocarbonyl-C₁-C₆alkyl-, *N,N-*di-(C₁-C₃alkyl)-aminocarbonyl-C₁-C₆alkyl-, aryl-C₁-C₆alkyl- or aryl-C₁-C₆alkyl- wherein the aryl moiety is substituted by one to three R⁷, which may be the same or different, or heterocyclyl-C₁-C₆alkyl- or heterocyclyl-C₁-C₆alkyl- wherein the heterocyclyl moiety is substituted by one to three R⁷, which may be the same or different;
R⁴ is aryl or aryl substituted by one to five R⁸, which may be the same or different, or heteroaryl or heteroaryl substituted by one to four R⁸, which may be the same or different;
R⁵ is hydroxy or a group which can be metabolised to a hydroxy group;
each R⁶, R⁷ and R⁸ is independently halo, cyano, nitro, C₁-C₁₀alkyl, C₁-C₄haloalkyl, C₂-C₁₀alkenyl, C₂-C₁₀alkynyl, hydroxy, C₁-C₁₀alkoxy, C₁-C₄haloalkoxy, C₁-C₁₀alkoxy-C₁-C₄alkyl-, C₃-C₇cycloalkyl, C₃-C₇cycloalkoxy, C₃-C₇cycloalkyl-C₁-C₄alkyl-, C₃-C₇cycloalkyl-C₁-C₄alkoxy-, C₁-C₆alkylcarbonyl-, formyl, C₁-C₄alkoxy-carbonyl-, C₁-C₄alkylcarbonyloxy-, C₁-C₁₀alkylthio-, C₁-C₄haloalkylthio-, C₁-C₁₀alkylsulfinyl-, C₁-C₄haloalkylsulfinyl-, C₁-C₁₀alkylsulfonyl-, C₁-C₄haloalkylsulfonyl-, amino, C₁-C₁₀alkylamino-, di-C₁-C₁₀alkylamino-, C₁-C₁₀alkylcarbonylamino-, aryl or aryl substituted by one to three R¹³, which may be the same or different, heteroaryl or heteroaryl substituted by one to three R¹³, which may be the same or different, aryl-C₁-C₄alkyl- or aryl-C₁-C₄alkyl- wherein the aryl moiety is substituted by one to three R¹³; which may be the same or different, heteroaryl-C₁-C₄alkyl- or heteroaryl-C₁-C₄alkyl- wherein the heteroaryl moiety is substituted by one to three R¹³, which may be the same or different, aryloxy- or aryloxy- substituted by one to three R¹³, which may be the same or different, heteroaryloxy- or heteroaryloxy-substituted by one to three R¹³, which may be the same or different, arylthio- or arylthio- substituted by one to three R¹³, which may be the same or different, or heteroarylthio- or heteroarylthio- substituted by one to three R¹³, which may be the same or different; and
each R¹³ is independently halo, cyano, nitro, C₁-C₆alkyl, C₁-C₆haloalkyl or C₁-C₆alkoxy; or a salt or *N*-oxide thereof; and
a compound of formula (IIf) 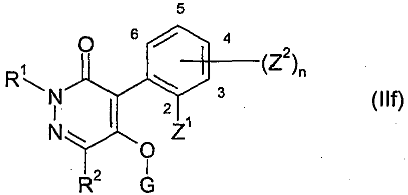 wherein
R¹ is C₁-C₆ alkyl or C₁-C₆alkyloxy-C₁-C₆alkyl;
R² is hydrogen or C₁-C₆alkyl;
G is a hydrogen, -(C=L)R³, -(SO₂)R⁴, or -(P=L)R⁵R⁶, wherein
L is oxygen or sulfur;
R³ is C₁-C₆alkyl, C₃-C₈cycloalkyl, C₂-C₆alkenyl, C₂-C₆alkynyl, C₆-C₁₀aryl, C₆-C₁₀aryl-C₁-C₆alkyl-, C₁-C₆alkyloxy, C₃-C₈cycloalkyloxy, C₂-C₆alkenyloxy, C₂-C₆alkynyloxy, C₆-C₁₀aryloxy, C₆-C₁₀aryl-C₁-C₆alkyloxy-, amino, C₁-C₆alkylamino, C₂-C₆alkenylamino, C₆-C₁₀arylamino, di(C₁-C₆alkyl)amino, di(C₂-C₆alkenyl)amino, (C₁-C₆alkyl)(C₆-C₁₀aryl)amino or a three- to eight-membered nitrogen containing heterocyclic ring,
R⁴ is C₁-C₆alkyl, C₆-C₁₀aryl, C₁-C₆alkylamino group or di(C₁-C₆ alkyl)amino; and R⁵ and R⁶ may be same or different and are independently C₁-C₆alkyl, C₃₋₈cycloalkyl, C₂-C₆alkenyl, C₆-C₁₀aryl, C₁-C₆alkyloxy, C₃-C₈cycloalkyloxy, C₆-C₁₀aryloxy, C₆-C₁₀aryl-C₁-C₆alkyloxy, C₁-C₆alkylthio, C₁-C₆alkylamino or di(C₁-C₆alkyl)amino, whereby any R³, R⁴, R⁵ and R⁶ group may be substituted with halogen, C₃-C₈cycloalkyl, C₆-C₁₀aryl, C₆-C₁₀aryl-C₁-C₆alkyl-, C₃-C₈cycloalkyloxy, C₆-C₁₀aryloxy, C₆-C₁₀aryl-C₁-C₆alkyloxy-, C₆-C₁₀arylamino, (C₁-C₆ alkyl)(C₆-C₁₀aryl)amino and a three- to eight-membered nitrogen containing heterocyclic ring which may be substituted with at least one C₁-C₆alkyl;
Z¹ is C₁-C₆alkyl;
Z² is C₁-C₆alkyl;
n is 0, 1, 2, 3 or 4;
and each of Z² may be same or different when n represents an integer of 2 or more, and a sum of the number of carbon atoms in the group represented by Z¹ and that in the group represented by Z² is equal to 2 or more.

The HST inhibitors of formula (IIa) are known, for example haloxydine and pyriclor. The HST inhibitors of formula (IIb) are known from, for example WO 2008/009908. The HST inhibitors of formula (IIc) are known from, for example WO 2008/071918. The HST inhibitors of formula (IId) are known from, for example WO 2009/063180. The HST inhibitors of formula (IIe) are known from, for example WO2009/090401 and WO2009/090402. The HST inhibitors of formula (IIf) are known from, for example WO 2007/119434.

Preferred are the compounds of formula (IIa) 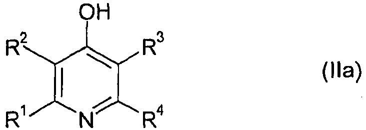 wherein
R¹, R², R³ and R⁴ are independently hydrogen, bromo, chloro or fluoro; provided that at least three of R¹, R², R³ and R⁴ are either bromo, chloro or fluoro, most preferred is the compound of formula (IIa) wherein R¹ and R⁴ are fluoro and R² and R³ are chloro (haloxydine) or wherein R¹, R² and R³ are chloro and R⁴ is hydrogen (pyriclor).

The term "HPPD inhibiting herbicide" refers to herbicides that act either directly or as procides to inhibit HPPD and that, in their active form, exhibit a Ki value of less than 5 nM, preferably 1 nM versus *Arabidopsis* HPPD when assayed using the on and off rate methods described in WO 02/46387. Within the context of the present invention the terms hydroxy phenyl pyruvate (or pyruvic acid) dioxygenase (HPPD), 4-hydroxy phenyl pyruvate (or pyruvic acid) dioxygenase (4-HPPD) and p-hydroxy phenyl pyruvate (or pyruvic acid) dioxygenase (p-HPPD) are synonymous.

Preferably, the HPPD-inhibiting herbicide is selected from the group consisting of
a compound of formula (Ia) 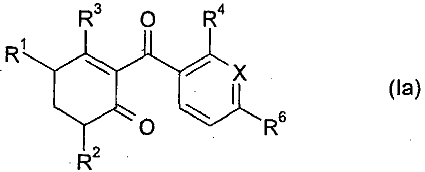
wherein R¹ and R² are hydrogen or together form an ethylene bridge;
R³ is hydroxy or phenylthio-; R⁴ is halogen, nitro, C₁-C₄alkyl, C₁-C₄alkoxy-C₁-C₄alkyl-, C₁-C₄alkoxy-C₁-C₄alkoxy-C₁-C₄alkyl-;
X is methine, nitrogen, or C-R⁵ wherein R⁵ is hydrogen, C₁-C₄haloalkoxy-C₁-C₄alkyl-, or a group 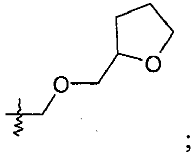 and
R⁶ is C₁-C₄alkylsulfonyl- or C₁-C₄haloalkyl;
a compound of formula (Ib) 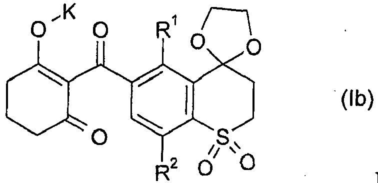 R¹ and R² are independently C₁-C₄alkyl; and the free acids thereof;
a compound of formula (Ic) 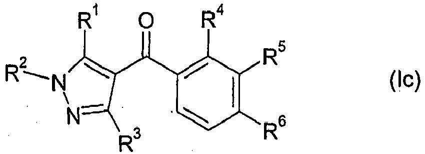
wherein R¹ is hydroxy, phenylcarbonyl-C₁-C₄alkoxy- or phenylcarbonyl-C₁-C₄alkoxy- wherein the phenyl moiety is substituted in para-position by halogen or C₁-C₄alkyl, or phenylsulfonyloxy- or phenylsulfonyloxy- wherein the phenyl moiety is substituted in para-position by halogen or C₁-C₄alkyl;
R² is C₁-C₄alkyl;
R³ is hydrogen or C₁-C₄alkyl; R⁴ and R⁶ are independently halogen, C₁-C₄alkyl, C₁-C₄haloalkyl, or C₁-C₄alkylsulfonyl-; and
R⁵ is hydrogen, C₁-C₄alkyl, C₁-C₄alkoxy-C₁-C₄alkoxy-, or a group 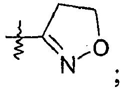
a compound of formula (Id) 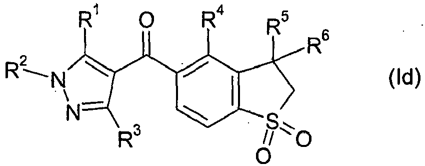
wherein R¹ is hydroxy;
R² is C₁-C₄alkyl;
R³ is hydrogen; and R⁴, R⁵ and R⁶ are independently C₁-C₄alkyl;
a compound of formula (Ie) 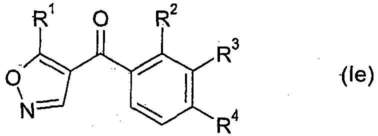
wherein R¹ is cyclopropyl;
R² and R⁴ are independently halogen, C₁-C₄haloalkyl, or C₁-C₄alkylsulfonyl-; and
R³ is hydrogen; and
a compound of formula (If) 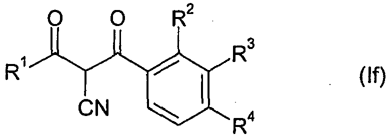
wherein R¹ is cyclopropyl;
R² and R⁴ are independently halogen, C₁-C₄haloalkyl, or C₁-C₄alkylsulfonyl-; and
R³ is hydrogen.

Example HPPD-inhibitors are also disclosed in WO2009/016841. In a preferred embodiment the HPPD inhibitor is selected from the group consisting of benzobicyclon, Mesotrione, sulcotrione, tefuryltrione, tembotrione, 4-hydroxy-3-[[2-(2-methoxyethoxy)methyl]-6-(trifluoromethyl)-3-pyridinyl]carbonyl]-bicyclo[3.2.1]-oct-3-en-2-one (bicyclopyrone), ketospiradox or the free acid thereof, benzofenap, pyrasulfotole, pyrazolynate, pyrazoxyfen, topramezone, [2-chloro-3-(2-methoxyethoxy)-4-(methylsulfonyl)phenyl](1-ethyl-5-hydroxy-1*H*-pyrazol-4-yl)-methanone, (2,3-dihydro-3,3,4-trimethyl-1,1-dioxidobenzo[b]thien-5-yl)(5-hydroxy-1-methyl-1*H*-pyrazol-4-yl)-methanone, isoxachlortole, isoxaflutole, α-(cyclopropylcarbonyl)-2-(methylsulfonyl)-β-oxo-4-chloro-benzenepropanenitrile, and α-(cyclopropylcarbonyl)-2-(methylsulfonyl)-β-oxo-4-(trifluoromethyl)-benzenepropanenitrile.

These HPPD inhibitors are known and have the following Chemical Abstracts registration numbers: benzobicyclon (CAS RN 156963-66-5), mesotrione (CAS RN 104206-82-8), sulcotrione (CAS RN 99105-77-8), tefuryltrione (CAS RN 473278-76-1), tembotrione (CAS RN 335104-84-2), 4-hydroxy-3-[[2-(2-methoxyethoxy)methyl]-6-(trifluoromethyl)-3-pyridinyl]carbonyl]-bicyclo[3.2.1]oct-3-en-2-one (CAS RN 352010-68-5), ketospiradox (CAS RN 192708-91-1) or its free acid (CAS RN 187270-87-7), benzofenap (CAS RN 82692-44-2), pyrasulfotole (CAS RN 365400-11-9), pyrazolynate (CAS RN 58011-68-0), pyrazoxyfen (CAS RN 71561-11-0), topramezone (CAS RN 210631-68-8), [2-chloro-3-(2-methoxyethoxy)-4-(methylsulfonyl)phenyl](1-ethyl-5-hydroxy-1*H-*pyrazol-4-yl)-methanone (CAS RN 128133-27-7), (2,3-dihydro-3,3,4-trimethyl-1,1-dioxidobenzo[b]thien-5-yl)(5-hydroxy-1-methyl-1*H-*pyrazol-4-yl)-methanone (CAS RN 345363-97-5), isoxachlortole (GAS RN 141112-06-3), isoxaflutole (CAS RN 141112-29-0), α-(cyclopropylcarbonyl)-2-(methylsulfonyl)-β-oxo-4-chloro-benzenepropanenitrile (CAS RN 143701-66-0), and α-(cyclopropylcarbonyl)-2-(methylsulfonyl)-β-oxo-4-(trifluoromethyl)-benzenepropanenitrile (CAS RN 143701-75-1).

The following definitions apply to those terms used in respect of Formula I and Formula II.

Alkyl moiety (either alone or as part of a larger group, such as alkoxy, alkoxy-carbonyl, alkylcarbonyl, alkylaminocarbonyl, dialkylaminocarbonyl) is a straight or branched chain and is, for example, methyl, ethyl, *n*-propyl, *n*-butyl, *n*-pentyl, *n-*hexyl, *iso*-propyl, *n*-butyl, *sec*-butyl, *iso*-butyl, *tert*-butyl or *neo*-pentyl. The alkyl groups are preferably C₁ to C₆ alkyl groups, more preferably C₁-C₄ and most preferably methyl groups.

Alkenyl and alkynyl moieties (either alone or as part of a larger group, such as alkenyloxy or alkynyloxy) can be in the form of straight or branched chains, and the alkenyl moieties, where appropriate, can be of either the (E)- or (Z)-configuration. Examples are vinyl, allyl and propargyl. The alkenyl and alkynyl groups are preferably C₂ to C₆ alkenyl or alkynyl groups, more preferably C₂-C₄ and most preferably C₂-C₃ alkenyl or alkynyl groups.

Alkoxyalkyl groups preferably have a chain length of from 2 to 8 carbon or oxygen atoms. An example of an alkoxyalkyl group is 2-methoxy-ethyl-.

Halogen is generally fluorine, chlorine, bromine or iodine, preferably fluorine or chlorine. The same is true of halogen in conjunction with other meanings, such as haloalkyl.

Haloalkyl groups preferably have a chain length of from 1 to 4 carbon atoms. Haloalkyl is, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl or 2,2,2-trichloroethyl; preferably trichloromethyl, difluorochloromethyl, difluoromethyl, trifluoromethyl or dichlorofluoromethyl. Haloalkoxyalkyl groups preferably have a chain length of from 2 to 8 carbon or oxygen atoms. An example of an alkoxyalkyl group is 2,2,2-trifluoroethoxymethyl-Alkoxyalkoxy groups preferably have a chain length of from 2 to 8 carbon or oxygen atoms. Examples of alkoxyalkoxy are: methoxymethoxy, 2-methoxy-ethoxy, methoxypropoxy, ethoxymethoxy, ethoxyethoxy, propoxymethoxy and butoxybutoxy. Alkoxyalkyl groups have a chain length of preferably from 1 to 6 carbon atoms. Alkoxyalkyl is, for example, methoxymethyl, methoxyethyl, ethoxymethyl, ethoxyethyl, n-propoxymethyl, n-propoxyethyl, isopropoxymethyl or isopropoxyethyl.

Alkoxyalkoxyalkyl groups preferably have a chain length of from 3 to 8 carbon or oxygen atoms. Examples of alkoxy-alkoxy-alkyl are: methoxymethoxymethyl, methoxyethoxymethyl, ethoxymethoxymethyl and methoxyethoxyethyl.

Cyanoalkyl groups are alkyl groups which are substituted with one or more cyano groups, for example, cyanomethyl or 1,3-dicyanopropyl.

Cycloalkyl groups can be in mono- or bi-cyclic form and may optionally be substituted by one or more methyl groups. The cycloalkyl groups preferably contain 3 to 8 carbon atoms, more preferably 3 to 6 carbon atoms. Examples of monocyclic cycloalkyl groups are cyclopropyl, 1-methylcyclopropyl, 2-methylcyclopropyl, cyclobutyl, cyclopentyl and cyclohexyl.

In the context of the present specification the term "aryl" refers to a ring system which may be mono-, bi- or tricyclic. Examples of such rings include phenyl, naphthalenyl, anthracenyl, indenyl or phenanthrenyl. A preferred aryl group is phenyl.

The term "heteroaryl" refers to an aromatic ring system containing at least one heteroatom and consisting either of a single ring or of two or more fused rings. Preferably, single rings will contain up to three and bicyclic systems up to four heteroatoms which will preferably be chosen from nitrogen, oxygen and sulfur. Examples of such groups include pyridyl, pyridazinyl, pyrimidinyl, pyrazinyl, triazinyl, furanyl, thiophenyl, oxazolyl, isoxazolyl, oxadiazolyl, thiazolyl, isothiazolyl, thiadiazolyl, pynolyl, pyrazolyl, imidazolyl, triazolyl and tetrazolyl. A preferred heteroaryl group is pyridine. Examples of bicyclic groups are benzothiophenyl, benzimidazolyl, benzothiadiazolyl, quinolinyl, cinnolinyl, quinoxalinyl and pyrazolo[1,5-a]pyrimidinyl.

The term "heterocyclyl" is defined to include heteroaryl and in addition their unsaturated or partially unsaturated analogues such as 4,5,6,7-tetrahydro-benzothiophenyl, chromen-4-onyl, 9H-fluorenyl, 3,4-dihydro-2H-benzo-1,4-dioxepinyl, 2,3-dihydro-benzofuranyl, piperidinyl, 1,3-dioxolanyl, 1,3-dioxanyl, 4,5-dihydro-isoxazolyl, tetrahydrofuranyl and morpholinyl.

It should be understood that in the aforementioned methods the herbicide composition may be applied to the locus pre-emergence of the crop and/or post-emergence of the crop. In a preferred embodiment the herbicide composition is applied post-emergence of the crop - a so-called "over-the-top" application. Single or indeed multiple applications may be applied as necessary to obtain the desired weed control.

The term "weeds" relates to any unwanted vegetation and includes, for example, carry-over or "rogue" or "volunteer" crop plants in a field of soybean crop plants.

Typically, the heterologous polynucleotide will comprise (i) a plant operable promoter operably linked to (ii) the region encoding the HST enzyme and (iii) a transcription terminator. Typically, the heterologous polynucleotide will further comprise a region which encodes a polypeptide capable of targeting the HST enzyme to subcellular organelles such as the chloroplast or mitochondria - preferably the chloroplast. The heterologous polynucleotide may further comprise, for example, transcriptional enhancers. Furthermore, the region encoding the HST enzyme can be "codon-optimised" depending on plant host in which expression of the HST enzyme is desired. The skilled person is well aware of plant operable promoters, transcriptional terminators, chloroplast transit peptides, enhancers etc that have utility with the context of the present invention.

The HST may be a "wild type" enzyme or it may be one which has been modified in order to afford preferential kinetic properties with regard to provision of herbicide tolerant plants. In a preferred embodiment the HST is characterised in that it comprises one or more of the following polypeptide motifs:-
W-(R/K)-F-L-R-P-H-T-I-R-G-T; and/or
N-G-(Y/F)-I-V-G-I-N-Q-I-(Y/F)-D; and/or
I-A-I-T-K-D-L-P; and/or
Y-(R/Q)-(F/W)-(I/V)-W-N-L-F-Y.

Suitable HSTs are derived from *Arabidopsis thaliana, Glycine max*, *Oryza sativa* or *Chlamydomonas reinhardtii.* In an even more preferred embodiment the HST is selected from the group consisting of SEQ ID NO:1 to SEQ ID NO. 10. It should be noted that amino acid sequences provided in SEQ ID NOS:1 to 10 are examples of HST amino acid sequences that include a region encoding a chloroplast transit peptide.

SEQ ID NOS 11-20 correspond to DNA sequences encoding the HSTs depicted as SEQ ID NO. 1-10 while SEQ ID NOS 21-24 are examples of DNA sequences encoding truncated mature HST sequences without the transit peptide region.

Amino acid sequences provided in SEQ ID NOS 25-28 are examples of HPPD amino acid sequences and SEQ ID NOS 29-32 are examples of DNA sequences encoding them. HPPDs suitable for providing tolerance to HPPD-inhibiting herbicides are well known to the skilled person - e.g WO 02/46387. SEQ ID No 33 provides the DNA sequence of the TMV translational enhancer and SEQ ID No 34 provides the DNA sequence of the TMV translational enhancer fused 5' to the DNA sequence encoding *Arabidopsis* HST.

It should be further understood that the crop plant used in said method may further comprise a further heterologous polynucleotide encoding a further herbicide tolerance enzyme. Examples of further herbicide tolerance enzymes include, for example, herbicide tolerance enzymes selected from the group consisting of, 5-enolpyruvylshikimate-3-phosphate synthase (EPSPS), Glyphosate acetyl transferase (GAT), Cytochrome P450, phosphinothricin acetyltransferase (PAT), Acetolactate synthase (ALS), Protoporphyrinogen oxidase (PPGO), Phytoene desaturase (PD), dicamba degrading enzymes (e.g WO 02/068607), and aryloxy herbicide degrading enzymes as taught in WO2007/053482 & WO2005/107437.

The pesticide composition used in the aforementioned methods may further comprise one or more additional pesticides - in particular herbicides - to which the crop plant is naturally tolerant, or to which it is resistant *via* expression of one or more additional transgenes as mentioned herein. In a preferred embodiment the one or more additional herbicides are selected from the group consisting of glyphosate (including agrochemically acceptable salts thereof); glufosinate (including agrochemically acceptable salts thereof); chloroacetanilides e.g alachlor, acetochlor, metolachlor, S-metholachlor; photo system II inhibitors e.g triazines such as ametryn, atrazine, cyanazine and terbuthylazine, triazinones such as hexazinone and metribuzin, ureas such as chlorotoluron, diuron, isoproturon, linuron and terbuthiuron; ALS-inhibitors e.g sulfonyl ureas such as amidosulfuron, chlorsulfuron, flupyrsulfuron, halosulfuron, nicosulfuron, primisulfuron, prosulfuron, rimsulfuron, triasulfuron, trifloxysulfuron and tritosulfuron; diphenyl ethers e.g aciflurofen and fomesafen.

Further disclosed is a recombinant polynucleotide which comprises a region which encodes an HST-enzyme operably linked to a plant operable promoter, wherein the region which encodes the HST-enzyme does not include the polynucleotide sequence depicted in SEQ ID NO. 11, SEQ ID NO. 12, SEQ ID NO. 14 or SEQ ID NO. 15. In preferred embodiment the HST-enzyme is selected from the group consisting of SEQ ID NO. 3, SEQ ID NO. 6, SEQ ID NO. 7, SEQ ID NO. 8, SEQ ID NO. 9 and SEQ ID NO. 10.

Further disclosed is a recombinant polynucleotide comprising (i) a region which encodes a HST enzyme operably linked to a plant operable promoter and (ii) at least one additional heterologous polynucleotide, which comprises a region which encodes an additional herbicide tolerance enzyme, operably linked to a plant operable promoter. The additional herbicide tolerance enzyme is, for example, selected from the group consisting of hydroxyphenyl pyruvate dioxygenase (HPPD), 5-enolpyruvylshikimate-3-phosphate synthase (EPSPS), Glyphosate acetyl transferase (GAT), Cytochrome P450, phosphinothricin acetyltransferase (PAT), Acetolactate synthase (ALS), Protoporphyrinogen oxidase (PPGO), Phytoene desaturase (PD) and dicamba degrading enzymes as taught in WO 02/068607.

Preferably the recombinant polynucleotide comprises (i) a region which encodes a HST operably linked to a plant operable promoter and (ii) a region which encodes an HPPD operably linked to a plant operable promoter. It is also possible for the recombinant polynucleotide to comprise at least two, three, or more additional regions each encoding a herbicide tolerance enzyme for example as defined previously. Thus, in another preferred embodiment the recombinant polynucleotide comprises (i) a region which encodes a HST enzyme, (ii) a region which encodes a HPPD enzyme and (iii) a region which encodes a glyphosate tolerance enzyme.

Further disclosed is a vector comprising a recombinant polynucleotide as described herein.

Further disclosed are transformed plants over expressing an HST enzyme which exhibit substantial resistance or substantial tolerance to HST-inhibiting herbicides and/or HPPD-inhibiting herbicides when compared with non transgenic like plants. It should also be appreciated that the transformed plants typically exhibit enhanced stress tolerance including heat and drought tolerance.

Thus, further disclosed is a plant cell which exhibits substantial resistance or substantial tolerance to HST-inhibiting herbicides and/or HPPD-inhibiting herbicides when compared with non transgenic like plant cell - said plant cell comprising the recombinant polynucleotide as herein described. It should be appreciated that the region encoding the HST and any region encoding one or more additional herbicide tolerance enzymes may be provided on the same ("linked") or indeed separate transforming recombinant polynucleotide molecules.

The plant cell may further comprise further transgenic traits, for example heterologous polynucleotides providing resistance to insects, fungi and/or nematodes.

Further disclosed are morphologically normal fertile HST-inhibitor tolerant plants, plant cells, tissues and seeds which comprise a plant cell as described herein.

Plants or plant cells transformed include but are not limited to, field crops, fruits and vegetables such as canola, sunflower, tobacco, sugar beet, cotton, maize, wheat, barley, rice, sorghum, tomato, mango, peach, apple, pear, strawberry, banana, melon, mangelworzel, potato, carrot, lettuce, cabbage, onion, etc. Particularly preferred genetically modified plants are soya spp, sugar cane, pea, field beans, poplar, grape, citrus, alfalfa, rye, oats, turf and forage gasses, flax and oilseed rape, and nut producing plants insofar as they are not already specifically mentioned. In a particularly preferred embodiment of the method the said plant is a dicot, preferably selected from the group consisting of canola, sunflower, tobacco, sugar beet, soybean, cotton, sorghum, tomato, mango, peach, apple, pear, strawberry, banana, melon, potato, carrot, lettuce, cabbage, onion, and is particularly preferably soybean. In further preferred embodiments the said plant is maize or rice. Preferably the plant is soybean, rice or maize. The disclosure also includes the progeny of the plant of the preceding sentence, and the seeds or other propagating material of such plants and progeny.

In a particularly preferred aspect, the recombinant polynucleotide is used to protect soybean crops from the herbicidal injury OF HPPD inhibitor herbicides of the classes of HPPD chemistry selected from the group consisting of the compounds of formula Ia or Ig. In a further embodiment the HPPD inhibitor herbicide is selected from sulcotrione, Mesotrione, tembotrione and compounds of formula Ia where X is nitrogen and R₄ is CF3, CF₂H or CFH₂ and/or where R₁ and R₂ together form an ethylene bridge.

The present invention still further provides a method of providing a transgenic plant which is tolerant to HST-inhibiting and/or HPPD-inhibiting herbicides which comprises transformation of plant material with a recombinant polynucleotide(s) which comprises a region which encodes an HST enzyme, selection of the transformed plant material using an HST-inhibiting herbicide and/or HPPD-inhibiting herbicide, and regeneration of that material into a morphological normal fertile plant. In a preferred embodiment the transformed plant material is selected using a HST-inhibiting herbicide alone or in combination with a HPPD-inhibiting herbicide.

The present invention further relates to the use of polynucleotide which comprises a region which encodes an HST enzyme as a selectable marker in plant transformation and to the use of a polynucleotide comprising a region which encodes an HST enzyme in the production of plants which are tolerant to herbicides which act wholly or in part by inhibiting HST.

Further disclosed is the use of HST inhibitors as selection agents in plant transformation and to the use of a recombinant HST enzyme in *in vitro* screening of potential herbicides.

Further disclosed is a herbicidal composition, preferably a synergistic herbicide composition, comprising an HPPD-inhibiting herbicide (as defined herein) and a HST-inhibiting herbicide (as defined herein). The ratio of the HPPD-inhibiting herbicide to the HST-inhibiting herbicide in the composition is any suitable ratio - typically from 100:1 to 1:100, preferably from 1:10 to 1:100, even more preferably from 1:1 to 1:20. The skilled person will recognize that the optimal ratio will depend on the relative potencies and spectrum of the two herbicides which can be derived as a matter of routine experimental optimisation.

The herbicidal composition may further comprise one or more additional pesticidal ingredient(s). The additional pesticides may include, for example, herbicides, fungicides or insecticides (such as thiomethoxam)-however herbicides are preferred. Thus, the additional herbicide is preferably selected from the group consisting of glyphosate (including agrochemically acceptable salts thereof); glufosinate (including agrochemically acceptable salts thereof); chloroacetanilides e.g alachlor, acetochlor, metolachlor, S-metholachlor; photo system II (PS-II) inhibitors e.g triazines such as ametryn, atrazine, cyanazine and terbuthylazine, triazinones such as hexazinone and metribuzin, and ureas such as chlorotoluron, diuron, isoproturon, linuron and terbuthiuron; ALS-inhibitors e.g sulfonyl ureas such as amidosulfuron, chlorsulfuron, flupyrsulfuron, halosulfuron, nicosulfuron, primisulfuron, prosulfuron, rimsulfuron, triasulfuron, trifloxysulfuron and tritosulfuron; diphenyl ethers e.g aciflurofen and fomesafen. PS-II herbicides are a particularly preferred as such mixtures exhibit particularly good efficacy.

Thus, further disclosed is a method of selectively controlling weeds at a locus comprising crop plants and weeds comprising applying to the locus a weed controlling amount of a synergistic herbicidal composition as previously defined. In a further embodiment of the current invention HPPD and HST inhibiting herbicides are sprayed sequentially rather than at the same time as a mixture. Thus for example, in a programme of weed control of the current invention, the HST herbicide can be advantageously applied over a crop locus to which an HPPD herbicide has already been previously applied. Normally this would be in the same season but, especially in the case of more persistent HPPD herbicides, there would also be an advantage in using the HST herbicide in the following season. In addition HST inhibiting herbicides are advantageously used as part of a programme of weed control wherein an HPPD inhibiting herbicide is applied earlier in the season or even in the preceding season.

The HPPD-inhibiting herbicide may be applied to the locus at any suitable rate - for example from 1 to 1000 g/ha, more preferably from 2 to 200 g/ha. Likewise, the HST-inhibiting herbicide may be applied at any suitable rate - for example from 10 to 2000 g/ha, more preferably from 50 to 400 g/ha.

In another embodiment, the HPPD-inhibiting herbicide is applied to the locus at a rate which is sub-lethal to the weeds were the HPPD-inhibiting herbicide to be applied in the absence of other herbicides. The actual sub-lethal rate will depend on weed species present and the actual HPPD inhibitor - but will typically be less than 50 g/ha - more preferably less than 10 g/ha. Thus, the present invention further provides the use of a sub-lethal application of an HPPD-inhibiting herbicide to increase the weed controlling efficacy of an HST-inhibiting herbicide.

The invention will be further apparent from the following non-limiting examples and sequence listings.

### SEQUENCE LISTING

SEQ ID NO. 1 ARABIDOPSIS HST AMINO ACID SEQUENCE 
melsisqsprvrfsslaprflaashhhrpsvhlagkfislprdvrftslstsrmrsk
fvstnyrkisiracsqvgaaesddpvldriarfqnacwrflrphtirgtalgstalv
tralienthlikwslvlkalsgllalicgngyivginqiydigidkvnkpylpiaag
dlsvqsawllviffaiagllvvgfnfgpfitslyslglflgtiysvpplrmkrfpva
afliiatvrgfllnfgvyhatraalglpfqwsapvafitsfvtlfalviaitkdlpd
vegdrkfqistlatklgvrniaflgsglllvnyvsaislafympqvfrgslmipahv
ilasglifqtwvlekanytkeaisgyyrfiwnlfyaeyllfpfl
SEQ ID NO. 2 RICE HST AMINO ACID SEQUENCE 
maslasppl pcraaatasrsgrpaprllgpppppaspllssasarfprapcnaarws
rrdavrvcsqagaagpaplsktlsdlkdscwrflrphtirgtalgsmslvaralien
pqlinwwlvfkafyglvalicgngyivginqiydiridkvnkpylpiaagdlsvqta
wllvvlfaaagfsivvtnfgpfitslyclglflgtiysvppfrlkrypvaafliiat
vrgfllnfgvyyatraalgltfqwsspvafitcfvtlfalviaitkdlpdvegdrky
qistlatklgvrniaflgsgllianyvaaiavaflmpqafrrtvmvpvhaalavgii
fqtwvleqakytkdaisqyyrfiwnlfyaeyiffpli
SEQ ID NO. 3 RICE HST VARIANT AMINO ACID SEQUENCE 
maslaspplpcraaatasrsgrpaprllgppppaspllssasarfprapcnaarws
rrdavrvcsqagaagpaplsktlsdlkdscwrflrphtirgtalgsialvaralien
pqlinwwlvfkafyglvalicgngyivginqiydiridkvnkpylpiaagdlsvqta
wllvvlfaaagfsivvtnfgpfitslyclglflgtiysvppfrlkrypvaafliiat
vrgfllnfgvyyatraalgltfqwsspvafitcfvtlfalviaitkdlpdvegdrky
qistlatklgvrniaflgsgllianyvaaiavaflmpqafrrtvmvpvhaalavgii
fqtwvleqakytkdaisqyyrfiwnlfyaeyiffpli
SEQ ID NO. 4 SOYA HST AMINO ACID SEQUENCE 
melslsptshrvpstiptlnsaklsstkatksqqplflgfskhfnsiglhhhsyrcc
snavperpqrpssiractgvgasgsdrplaerlldlkdacwrflrphtirgtalgsf
alvaralientnlikwslffkafcglfalicgngyivginqiydisidkvnkpylpi
aagdlsvqsawflviffaaaglsiaglnfgpfifslytlglflgtiysvpplrmkrf
pvaafliiatvrgfllnfgvyyatraslglafewsspvvfittfvtffalviaitkd
lpdvegdrkyqistfatklgvrniaflgsgillvnyivsvlaaiympqafrrwllip
ahtifaisliyqarileqanytkdaisgfyrfiwnlfyaeyaifpfi
SEQ ID NO. 5 CHLAMYDOMONAS HST AMINO ACID SEQUENCE 
mdlcsstgrgaclspastsrpcpapvhlrgrrlafspaqpagrrhlpvlssaavpap
lpnggndesfaqklanfpnafwkflrphtirgtilgttavtakvlmenpgcidwall
pkallglvallcgngyivginqiydvdidvvnkpflpvasgelspalawglclslaa
agagivaanfgnlitslytfglflgtvysvpplrlkqyavpafmiiatvrgfllnfg
vysatraalglpfewspavsfitvfvtlfatviaitkdlpdvegdqannistfatrm
gvrnvallaigllmanylgaialaltystafnvplmagahailaatlalrtlklhaa
sysreavasfyrwiwnlfyaeyallpfl
SEQ ID NO. 6 NICOTINIA HST AMINO ACID SEQUENCE 
melacsscsslrfssvlthqdtaasryrklpptspsckaanfvlkssknlsssaglh
igytnfsktvsyrkyrhisiracsqvgtagsepvldklsqfkdafwrflrphtirgt
algslslvtralienpnlirwslamkafsglialicgngyivginqiydigidkvnk
pylpiaagdlsvqsawflvllfamagllivginfgpfitslyclglflgtiysvppf
rmkrfavvafliiatvrgfllnygvyyattaalglsfqwsspvafittfvtlfalvi
aitkdlpdvegdrkfqistlatklgvrniaflgsglllanyigavvaaiympqafrs
slmipvhailalclvfqawllekanytkeaisayyqfiwnffyaeylifpfi
SEQ ID NO. 7 AQUILEGIA HST AMINO ACID SEQUENCE 
lcfsspsisipphcsttthyrkipinstfkstnflskasnnlttfgfsrnkkysrsi
lsrksrhfsiwassqvgaagsddpllkkipdfkdavwrflrphtirgtalgsialvs
ralienthlikwsllfkaicgvfalmcgngyivginqiydigidkvnkpylpiaagd
lsvqsawslvtffavagvcivafnfgpfitslyclglflgtiysvpplrmkrypvaa
fliiatvrgfllnfgvyhatraalgltfewsypvafittfvtmfalviaitkdlpdv
egdrkfqistlatklgvrniallgtglllanyigaivaaiylpqafrrnlmipahti
lalglvfqawaleqakyskeaildfyrfvwnlfyseyflfpfi
SEQ ID NO. 8 BRASSICA NAPUS HST AMINO ACID SEQUENCE 
melsishspclrfssssprflaasshhyrpsvhlagkllsrskdadltslssscmrs
kfvstnyrkisirassqvgaagsdpvldrlarfqnacwrflrphtirgtalgstalv
tralienthlikwslvlkalsgllalicgngyivginqiydigidkvnkpylpiaag
dlsvqsawllviffaiagltvvgfnfgpfitclyslglflgtiysvppfrmkrfpva
afliiatvrgfllnfgvyhatraalglsfqwsapvafitsfvtlfalviaitkdlpd
vegdrkfqistlatklgvrniaflgsglllvnyisaislafympqvfrgslmipahm
ilasclvfqtwvlekanytkeaiagyyrfiwnlfyaeyllfpff
SEQ ID NO. 9 VITIS VINIFERA HST AMINO ACID SEQUENCE 
mkvdavqastqvgaagsdpplnkfsvfkdacwrflrphtirgtalgstalvaralie
npnlikwsllfkafsgllalicgngyivginqiydisidkvnkpylpiaagdlsvqs
awflvlffavagvlivgsnfgsfitslyclglvlgtiysvppfrmkrfpvaafliia
tvrgfllnfgvyyatraalglpfmwsapvvfittfvtlfalviaitkdlpdvegdrk
 
yqistlatklgvrniaflgsglllvnyigsilaaiympqafrlslmipahailaagl
ifqarvleqanytkeaisdfyrfiwnlfyveyiifpfi
SEQ ID NO. 10 PHYSCOMITRELLA PATENS HST SEQUENCE 
mgltaivvdvaqassssvalsqgrgatrrlpgglalgdafkglrkreyaqglqcrvr
reggcasearvwkvrcssdsagslggdlpasqpqqsevsgirdpaaasaasfaplpq
rialfydafwrflrphtirgtflgtsalvtrallenptlinwallpkalrgllallc
gngfivginqifdsgidkvnkpflpiaagdlsvpaawalvgglaalgvglvatnfgp
littlytfglflgtiysvpplrlkqypvpafmiiatvrgfllnfgvyyatraalgls
yewspsvmfitifvtlfatviaitkdlpdiegdkkfnistfatnlgvrkisflgagl
llvnyigaivaafylpqafktkimvtghavlglsliyqtwlldtakyskeaisnfyr
fiwnlfyseyalfpfi
SEQ ID NO. 11 ARABIDOPSIS HST (DNA) 
atggagctctcgatctcacaatcaccgcgtgttcggttctcgtctctggcgcctcgt
ttcttagcagcttctcatcatcatcgtccttctgtgcatttagctggaagtttata
agcctccctcgagatgttcgcttcacgagcttatcaacttcaagaatgcggtccaaa
tttgtttcaaccaattatagaaaaatctcaatccgggcatgttctcaggttggtgct
gctgagtctgatgatccagtgctggatagaattgcccggttccaaaatgcttgctgg
agatttcttagaccccatacaatccgcggaacagctttaggatccactgccttggtg
acaagagctttgatagagaacactcatttgatcaaatggagtcttgtactaaaggca
ctttcaggtcttcttgctcttatttgtggaatggttatatagtcggcatcaatcag
atctacgacattggaatcgacaaagtgaacaaaccatacttgccaatagcagcagga
gatctatcagtgcagtctgcttggttgttagtgatatttttgcgatagcagggctt
ttagttgtcggatttaactttggtccattcattacaagcctatactctcttggcctt
tttctgggaaccatctattctgttccacccctcagaatgaaaagattcccagttgca
gcatttcttattattgccacggtacgaggtttccttcttaactttggtgtgtaccat
gctacaagagctgctcttggacttccatttcagtggagtgcacctgtggcgttcatc
acatcttttgtgacactgtttgcactggtcattgctattacaaaggaccttcctgat
gttgaaggagatcgaaagttccaaatatcaaccctggcaacaaaacttggagtgaga
aacattgcattcctcggttctggacttctgctagtaaattatgtttcagccatatca
ctagctttctacatgcctcaggtttttagaggtagcttgatgattcctgcacatgtg
atcttggcttcaggcttaattttccagacatgggtactagaaaaagcaaactacacc
aaggaagctatctcaggatattatcggtttatatggaatctcttctacgcagagtat
ctgttattccccttcctctag
SEQ ID NO. 12 RICE HST (DNA) 
atggcttccctcgcctcccctcctctcccctgccgcgccgccgccaccgccagccgc
agcgggcgtcctgctccgcgcctcctcggccctccgccgccgcccgcttcccctctc
ctctcctccgcttcggcgcgcttcccgcgtgcccccctgcaacgccgcacgctggagc
cggcgcgacgccgtgcgggtttgctctcaagctggtgcagctggaccagccccatta
tcgaagacattgtcagacctcaaggattcctgctggagatttttacggccacataca
 
attcgaggaactgccttgggatccatgtcattagttgctagagctttgatagagaac
ccccaactgataaattggtggttggtattcaaagcgttctatgggctcgtggcgtta
atctgtggcaatggttacatcgttgggatcaatcagatctatgacattagaatcgat
aaggtaaacaagccatatttaccaattgctgccggtgatctctcagttcagacagca
tggttattggtggtattatttgcagctgcgggattttcaattgttgtgacaaacttt
ggacctttcattacctctctatattgccttggtctatttcttggcaccatatactct
gttcctccattcagacttaagagatatcctgttgctgcttttcttatcattgcaacg
gtccgtggttttcttctcaactttggtgtgtactatgctactagagcagcactgggt
cttacattccaatggagctcgcctgttgctttcattacatgcttcgtgactttattt
gctttggtcattgctataaccaaagatctcccagatgttgaaggggatcggaagtat
caaatatcaactttggcgacaaagctcggtgtcagaaacattgcatttcttggctct
ggtttattgatagcaaattatgttgctgctattgctgtagcttttctcatgcctcag
gctttcaggcgcactgtaatggtgcctgtgcatgctgcccttgccgttggtataatt
ttccagacatgggttctggagcaagcaaaatatactaaggatgctatttcacagtac
taccggttcatttggaatctcttctatgctgaatacatcttcttcccgttgata
SEQ ID NO. 13 RICE VARIANT HST (DNA) 
atggcttccctcgcctcccctcctctcccctgccgcgccgccaccgccagccgc
agcgggcgtcctgctccgcgcctcctcggccctccgccgcccgcttcccctctc
ctctcctccgcttcggcgcgcttcccgcgtgcccctgcaacgccgcacgctggagc
cggcgcgacgccgtgcgggtttgctctcaagctggtgcagctggaccagccccatta
tcgaagacattgtcagacctcaaggattcctgctggagatttttacggccacataca
attcgaggaactgccttgggatccatagcattagttgctagagctttgatagagaac
cccaactgataaattggtggttggtattcaaagcgttctatgggctcgtggcgtta
atctgtggcaatggttacatcgttggatcaatcagatctatgacattagaatcgat
aaggtaaacaagccatatttaccaattgctgccggtgatctctcagttcagacagca
tggttattggtggtattatttgcagctgcgggatttcaattgttgtgacaaacttt
ggacctttcattacctctctatattgccttggtctatttcttggcaccatatactct
gttcctccattcagacttaagagatatcctgttgctgcttttcttatcattgcaacg
gtccgtggttttcttctcaactttggtgtgtactatgctactagagcagcactgggt
cttacattccaatggagctcgcctgttgctttcattacatgcttcgtgactttattt
gctttggtcattgctataaccaaagatctcccagatgttgaaggggatcggaagtat
caaatatcaactttggcgacaaagctcggtgtcagaaacattgcatttcttggctct
ggtttattgatagcaaattatgttgctgctattgctgtagcttttctcatgcctcag
gctttcaggcgcactgtaatggtgcctgtgcatgctgcccttgccgttggtataatt
ttccagacatgggttctggagcaagcaaaatatactaaggatgctatttcacagtac
taccggttcatttggaatctcttctatgctgaatacatcttcttcccgttgatatag
SEQ ID NO. 14 SOYA HST (DNA) 
tctgctaaattatcttctactaaagctactaaatctcaacaacctttatttttagga
ttttctaaacattttaattctattggattacatcatcattcttatagatgttgttct
aatgctgtacctgaaagacctcaaagaccttcttctattagagcttgtactggagta
ggagcttctggatctgatagacctttagctgaaagattattagatttaaaagatgct
tgttggagattttttaagacctcatactattagaggaactgctttaggatcttttgct
ttagtagctagagctttaattgaaaatactaatttaattaaatggtctttattttttt
 
aaagctttttgtggattatttgctttaatttgtggaaatggatatattgtaggaatt
aatcaaatttatgatatttctattgataaagtaaataaaccttatttacctattgct
gctggagatttatctgtacaatctgcttggttttagtaatttttttgctgctgct
ggattatctattgctggattaaatttggaccttttatttttctttatatacttta
ggattattttaggaactatttattctgtacctcctttaagaatgaaaagatttcct
gtagctgctttttttaattattgctactgtaagaggattttttattaaattttggagta
tattatgctactagagcttctttaggattagcttttgaatggtcttctcctgtagta
tttattactactttttgtaacttttttttgctttagtaattgctattactaaagattta
cctgatgtagaaggagatagaaaatatcaaatttctacttttgctactaaattagga
gtaagaaatattgctttttttaggatctggaattttattagtaaattatattgtatct
gtattagctgctatttatatgcctcaagcttttagaagatggttattaattcctgct
catactatttttgctatttctttaatttatcaagctagaattttagaacaagctaat
tatactaaagatgctatttctggattttatagatttatttggaatttattttatgct
gaatatgctatttttcctttttatt
SEQ ID NO. 15 CHLAMYDOMONAS HST (DNA) 
atggacctttgcagctcaactggaagaggagcatgcctttcgccggcatccacgtcg
cggccgtgcccagcaccagtgcatttgcgcggccgacgcctggctttctctccggct
cagcctgctggacggcgccacttgccggtgctctcatctgcagcggtccccgctccc
ctcccaaatggtggaaacgacgagagcttcgcacaaaactggctaactttccaaac
gccttctggaagttcctgcggccacacaccatccgggggactatcctgggcaccaca
gctgtgaccgccaaggtccttatggagaaccccggctgcatagactgggcactgctg
ccgaaggcgctgctcggcctggtggcgctgctgtgcggcaacggctacattgtgggc
atcaaccaaatctacgacgtcgacattgacgtggtcaacaagccattcctccccgtg
gcgtcgggcgagctgtcgccggcgctggcgtggggcctgtgtctgtcgctggcggct
gcgggcgcgggcatcgtagccgccaacttcggcaacctcatcaccagcctctacacc
tttggcctcttcctgggcaccgtgtacagtgtgcctcccctgcgcctgaagcagtac
gcggtgccggccttcatgatcatcgccacggtgcgcggcttcctgctcaacttcggc
gtgtacagcgccacgcgggcggcactgggactgcccttcgagtggagcccggccgtc
agcttcatcacggtgtttgtgacgctgtttgccactgtgatcgccatcaccaaggac
ctgccggacgtggagggcgaccaggccaacaacatctccaccttcgccacgcgcatg
ggcgtgcgcaacgtggcactgctggccatcggccttctcatggccaactacctgggt
gccatcgcgctggcactcacctactccaccgccttcaacgtgccgctcatggcgggc
gcgcacgccatcctggccgccacgctggcgctgcgcacgctcaagctgcacgccgcc
agctacagccgggaggcggtggcgtccttctaccgctggatctggaacctgttctac
gccgagtacgcgctgctgccgttcctgtag
SEQ ID NO. 16 NICOTINIA HST DNA 
atggaattagcttgttcttcttgttcttctttaagatttcttctgtattaactcat
caagatactgctgcttctagatatagaaaattacctcctacttctccttcttgtaaa
gctgctaattttgtattaaaatcttctaaaaatttatcttcttctgctggattacat
attggatatactaattttctaaaactgtatcttatagaaaatatagacatatttct
attagagcttgttctcaagtaggaactgctggatctgaacctgtattagataaatta
tctcaatttaaagatgcttttggagattttaagacctcatactattagaggaact
 
gctttaggatctttatctttagtaactagagctttaattgaaaatcctaatttaatt
agatggtctttagctatgaaagcttttctggattaattgctttaatttgtggaaat
ggatatattgtaggaattaatcaaatttatgatattggaattgataaagtaaataaa
ccttatttacctattgctgctggagatttatctgtacaatctgcttggttttagta
ttattatttgctatggctggattattaattgtaggaattaattttggaccttttatt
acttctttatattgtttaggattatttttaggaactatttattctgtacctcctttt
agaatgaaaagatttgctgtagtagctttttttaattattgctactgtaagaggattt
ttattaaattatggagtatattatgctactactgctgctttaggattatcttttcaa
tggtcttctcctgtagcttttattactactttttgtaactttatttgctttagtaatt
gctattactaaagatttacctgatgtagaaggagatagaaaatttcaaatttctact
ttagctactaaattaggagtaagaaatattgctttttttaggatctggattattatta
gctaattatattggagctgtagtagctgctatttatatgcctcaagcttttagatct
tctttaatgattcctgtacatgctattttagctttatgtttagtatttcaagcttgg
ttattagaaaaagctaattatactaaagaagctatttctgcttattatcaatttatt
tggaatttttttatgctgaatatttaatttttcctttttatt
SEQ ID NO. 17 AQUILEGIA HST DNA 
atgttatgtttttcttctccttctatttctattcctcctcattgttctactactact
cattatagaaaattcctattaattctactttaaatctactaatttttttatctaaa
gcttctaataatttaactactttggatttctagaaataaaaaatattctagatct
attttatctagaaatctagacatttttctatttgggcttcttctcaagtaggagct
gctggatctgatgatcctttattaaaaaaattcctgattttaaagatgctgtatgg
agattttaagacctcatactattagaggaactgctttaggatctattgctttagta
tctagagctttaattgaaaatactcatttaattaaatggtctttattatttaaagct
atttgtggagtatttgctttaatgtgtggaaatggatatattgtaggaattaatcaa
atttatgatattggaattgataaagtaaataaaccttatttacctattgctgctgga
gatttatctgtacaatctgcttggtctttagtaactttttttgctgtagctggagta
tgtattgtagcttttaattttggacctttattacttctttatattgtttaggatta
tttttaggaactatttattctgtacctcctttaagaatgaaaagatatcctgtagct
gctttttttaattattgctactgtaagaggattttttattaaattttggagtatatcat
gctactagagctgctttaggattaacttttgaatggtcttatcctgtagcttttatt
actacttttgtaactatgtttgctttagtaattgctattactaaagatttacctgat
gtagaaggagatagaaaatttcaaatttctactttagctactaaattaggagtaaga
aatattgctttattaggaactggattattattagctaattatattggagctattgta
gctgctatttatttacctcaagcttttagaagaatttaatgattcctgctcatact
attttagctttaggattagtatttcaagcttgggctttagaacaagctaaatattct
aaagaagctattttagattttatagatttgtatggaatttattttattctgaatat
tttttatttcctttttatt
SEQ ID NO. 18 BRASSICA NAPUS HST DNA 
ttagctgcttcttctcatcattatagaccttctgtacatttagctggaaattatta
tctagatctaaagatgctgatttaacttctttatcttcttcttgtatgagatctaaa
tttgtatctactaattatagaaaatttctattagagcttcttctcaagtaggagct
gctggatctgatcctgtattagatagattagctagatttcaaaatgcttgttggaga
tttttaagacctcatactattagaggaactgctttaggatctactgctttagtaact
 
agagctttaattgaaaatactcatttaattaaatggtctttagtattaaaagcttta
tctggattattagctttaatttgtggaaatggatatattgtaggaattaatcaaatt
tatgatattggaattgataaagtaaataaaccttatttacctattgctgctggagat
ttatctgtacaatctgcttggttattagtaatttttttgctattgctggattaact
gtagtaggatttaatttttggacctttattacttgtttatattctttaggattattt
ttaggaactatttattctgtacctccttttagaatgaaaagatttcctgtagctgct
tttttaattattgctactgtaagaggattttttattaaattttggagtatatcatgct
actagagctgctttaggattatcttttcaatggtctgctcctgtagcttttattact
tcttttgtaactttatttgctttagtaattgctattactaaagatttacctgatgta
gaaggagatagaaaatttcaatttctactttagctactaaattaggagtaagaaat
attgctttttaggatctggattattattagtaaattatatttctgctatttcttta
gctttttatatgcctcaagtatttagaggatctttaatgattcctgctcatatgatt
ttagcttcttgtttagtatttcaaacttgggtattagaaaaagctaattatactaaa
gaagctattgctggatattatagatttatttggaatttattttatgctgaatattta
ttatttcctttttttt
SEQ ID NO. 19 VITIS VINIFERA HST DNA 
atgaaagtagatgctgtacaagcttctactcaagtaggagctgctggatctgatcct
cctttaaataaattttctgtatttaaagatgcttgttggagattttaagacctcat
actattagaggaactgctttaggatctactgctttagtagctagagctttaattgaa
aatcctaatttaattaaatggtctttattatttaaagctttttctggattattagct
ttaatttgtggaaatggatatattgtaggaattaatcaaatttatgatatttctatt
gataaagtaaataaaccttatttacctattgctgctggagatttatctgtacaatct
gcttggtttttagtattatttttgctgtagctggagtattaattgtaggatctaat
tttggatcttttattacttctttatattgtttaggattagtattaggaactatttat
tctgtacctcctttagaatgaaaagatttcctgtagctgctttttaattattgct
actgtaagaggattttattaaattttggagtatattatgctactagagctgcttta
ggattacctttatgtggtctgctcctgtagtatttattactactttgtaacttta
tttgctttagtaattgctattactaaagatttacctgatgtagaaggagatagaaaa
tatcaaatttctactttagctactaaattaggagtaagaaatattgctttttagga
tctggattattattagtaaattatattggatctattttagctgctatttatatgcct
caagcttttagattatctttaatgattcctgctcatgctattttagctgctggatta
atttttcaagctagagtattagaacaagctaattatactaaagaagctatttctgat
ttttatagatttatttggaatttattttatgtagaatatattatttttcctttatt
SEQ ID NO. 20 PHYSCOMITRELLA PATENS HST DNA 
atgggattaactgctattgtagtagatgtagctcaagcttcttcttcttctgtagct
ttatctcaaggaagaggagctactagaagattacctggaggattagctttaggagat
gcttttaaaggattaagaaaaagagaatatgctcaaggattacaatgtagagtaaga
agagaaggaggatgtgcttctgaagctagagtatggaaagtaagatgttcttctgat
tctgctggatctttaggaggagatttacctgcttctcaacctcaacaatctgaagta
tctggaattagagatcctgctgctgcttctgctgcttcttttgctcctttacctcaa
agaattgctttattttatgatgctttttggagatttttaagacctcatactattaga
ggaactttttaggaacttctgctttagtaactagagctttattagaaatcctact
ttaattaattgggctttattacctaaagctttaagaggattattagctttattatgt
 
ggaaatggatttattgtaggaattaatcaaatttttgattctggaattgataaagta
aataaaccttttttacctattgctgctggagatttatctgtacctgctgcttgggct
ttagtaggaggattagctgctttaggagtaggattagtagctactaattttggacct
ttaattactactttatatacttttggattatttttaggaactatttattctgtacct
cctttaagattaaaacaatatcctgtacctgcttttatgattattgctactgtaaga
ggattttttattaaattttggagtatattatgctactagagctgctttaggattatct
tatgaatggtctccttctgtaatgtttattactattttttgtaactttatttgctact
gtaattgctattactaaagatttacctgatattgaaggagataaaaaatttaatatt
tctacttttgctactaatttaggagtaagaaaaatttctttttttaggagctggatta
ttattagtaaattatattggagctattgtagctgctttttatttacctcaagctttt
aaaactaaaattatggtaactggacatgctgtattaggattatctttaatttatcaa
acttggttattagatactgctaaatattctaaagaagctatttctaattttttataga
tttatttggaatttattttattctgaatatgctttatttccttttatt
SEQ ID NO. 21 DNA ENCODING MATURE ARABIDOPIS HST 
agaaaaatctcaatccgggcatgttctcaggttggtgctgctgagtctgatgatcca
gtgctggatagaattgcccggttccaaaatgcttgctggagatttcttagaccccat
acaatccgcggaacagctttaggatccactgccttggtgacaagagctttgatagag
aacactcatttgatcaaatggagtcttgtactaaaggcactttcaggtcttcttgct
cttatttgtgggaatggttatatagtcggcatcaatcagatctacgacattggaatc
gacaaagtgaacaaaccatacttgccaatagcagcaggagatctatcagtgcagtct
gcttggttgttagtgatatttttttgcgatagcagggcttttagttgtcggatttaac
tttggtccattcattacaagcctatactctcttggcctttttctgggaaccatctat
tctgttccacccctcagaatgaaaagattcccagttgcagcatttcttattattgcc
acggtacgaggtttccttcttaactttggtgtgtaccatgctacaagagctgctctt
ggacttccatttcagtggagtgcacctgtggcgttcatcacatcttttgtgacactg
tttgcactggtcattgctattacaaaggaccttcctgatgttgaaggagatcgaaag
ttccaaatatcaaccctggcaacaaaacttggagtgagaaacattgcattcctcggt
tctggacttctgctagtaaattatgtttcagccatatcactagctttctacatgcct
caggtttttagaggtagcttgatgattcctgcacatgtgatcttggcttcaggctta
attttccagacatgggtactagaaaaagcaaactacaccaaggaagctatctcagga
tattatcggtttatatggaatctcttctacgcagagtatctgttattccccttcctc
ta
SEQ ID NO. 22 DNA ENCODING MATURE RICE HST 
cggcgcgacgccgtgcgggtttgctctcaagctggtgcagctggaccagccccatta
tcgaagacattgtcagacctcaaggattcctgctggagattttacggccacataca
attcgaggaactgccttgggatccatgtcattagttgctagagctttgatagagaac
cccaactgataaattggtggttggtattcaaagcgttctatgggctcgtggcgtta
atctgtggcaatggttacatcgttgggatcaatcagatctatgacattagaatcgat
aaggtaaacaagccatatttaccaattgctgccggtgatctctcagttcagacagca
tggttattggtggtattatttgcagctgcgggattttcaattgttgtgacaaacttt
ggacctttcattacctctctatattgccttggtctatttcttggcaccatatactct
gttcctccattcagacttaagagatatcctgttgctgctttcttatcattgcaacg
 
gtccgtggttttcttctcaactttggtgtgtactatgctactagagcagcactgggt
cttacattccaatggagctcgcctgttgctttcattacatgcttcgtgactttattt
gctttggtcattgctataaccaaagatctcccagatgttgaaggggatcggaagtat
caaatatcaactttggcgacaaagctcggtgtcagaaacattgcatttcttggctct
ggtttattgatagcaaattatgttgctgctattgctgtagcttttctcatgcctcag
gctttcaggcgcactgtaatggtgcctgtgcatgctgcccttgccgttggtataatt
ttccagacatgggttctggagcaagcaaaatatactaaggatgctatttcacagtac
taccggttcatttggaatctcttctatgctgaatacatcttcttcccgttgatatag
SEQ ID NO. 23 DNA ENCODING MATURE RICE VARIANT HST 
cggcgcgacgccgtgcgggtttgctctcaagctggtgcagctggaccagccccatta
tcgaagacattgtcagacctcaaggattcctgctggagattttacggccacataca
attcgaggaactgccttgggatccatagcattagttgctagagctttgatagagaac
ccccaactgataaattggtggttggtattcaaagcgttctatgggctcgtggcgtta
atctgtggcaatggttacatcgttgggatcaatcagatctatgacattagaatcgat
aaggtaaacaagccatatttaccaattgctgccggtgatctctcagttcagacagca
tggttattggtggtattatttgcagctgcgggattttcaattgttgtgacaaacttt
ggacctttcattacctctctatattgccttggtctatttcttggcaccatatactct
gttcctccattcagacttaagagatatcctgttgctgcttttcttatcattgcaacg
gtccgtggttttcttctcaactttggtgtgtactatgctactagagcagcactgggt
cttacattccaatggagctcgcctgttgctttcattacatgcttcgtgactttattt
gctttggtcattgctataaccaaagatctcccagatgttgaaggggatcggaagtat
caaatatcaactttggcgacaaagctcggtgtcagaaacattgcatttcttggctct
ggtttattgatagcaaattatgttgctgctattgctgtagcttttctcatgcctcag
gctttcaggcgcactgtaatggtgcctgtgcatgctgcccttgccgttggtataatt
ttccagacatgggttctggagcaagcaaaatatactaaggatgctatttcacagtac
taccggttcatttggaatctcttctatgctgaatacatcttcttcccgttgatatag
SEQ ID NO. 24 DNA Encoding Insect Cell Codon Optimized Mature Arabidopsis HST 
gggatccctcgtgcttgctcccaggtcggcgctgctgagtccgacgaccccgtgctg
gaccgtatcgctcgtttccagaacgcttgctggcgtttcctgcgtccccacaccatc
cgtggcaccgctctgggttccaccgccctggtgacccgtgctctgatcgagaacacc
cacctgatcaagtggtccctggtgctgaaggctctgtccggtctgctggctctgatc
tgcggtaacggttacatcgtgggtatcaaccagatctacgacatcggtatcgacaag
gtgaacaagccctacctgcccatcgctgctggtgacctgtccgtgcagtccgcttgg
ctgctggtcatcttcttcgctatcgctggtctgctggtcgtgggtttcaacttcggt
cccttcatcacttccctgtactccctgggcctgttcctgggcaccatctactccgtg
ccccccctgcgtatgaagcgtttccccgtggctgctttcctgatcatcgctaccgtg
cgtggtttcctgctgaacttcggtgtctaccacgctacccgtgctgctctgggtctg
cccttccagtggtccgctcccgtggctttcatcaccagcttcgtgaccctgttcgct
ctggtgatcgctatcaccaaggacctgcccgacgtggaggtgaccgtaagttccag
 
atctccaccctggctaccaagctgggtgtgcgtaacatcgctttcctcggttccggc
ctgctgctcgtgaactacgtgtccgctatctccctggctttctacatgccccaggtg
ttccgtggttccctgatgatccccgctcacgtgatcctggcttccggtctgatcttc
cagacctgggtgctcgagaaggctaactacaccaaggaagctatctccggttactac
cgcttcatctggaacctgttctacgctgagtacctgctgttccccttcctgtaa
SEQ ID No. 25 HPPD a/a sequence from *Pseudomonas fluorescens* strain 87-79 
madqyenpmglmgfefiefasptpgtlepifeimgftkvathrsknvhlyrqgeinl
ilnnqpdslasyfaaehgpsvcgmafrvkdsqqaynralelgaqpihietgpmelnl
paikgiggaplylidrfgegssiydidfvylegvdrnpvgaglkvidhlthnvyrgr
maywanfyeklfnfrearyfdikgeytgltskamsapdgmiriplneesskgagqie
eflmqfngegiqhvafltedlvktwdalkkigmrfmtappdtyyemlegrlpnhgep
vdqlqargilldgssiegdkrlllqifsetlmgpvffefiqrkgddgfegnfkalf
esierdqvrrgvlttd
SEQ ID NO. 26 HPPD a/a sequence from *Avena sativa* 
mpptpatatgaaaaavtpehaarsfprvvrvnprsdrfpvlsfhhvelwcadaasaa
grfsfalgaplaarsdlstgnsahaslllrsgalaflftapyapppqeaataaatas
ipsfsadaartfaaahglavrsvgvrvadaaeafrvsvaggarpafapadlghgfgl
aevelygdvvlrfvsypdetdlpflpgfervsspgavdygltrfdhvvgnvpemapv
idymkgflgfhefaeftaedvgttesglnsvvlannseavllplnepvhgtkrrsqi
qtyleyhggpgvqhialasndvlrtlremrartpmggfefmappqakyyegvrriag
dvlseeqikecqelgvlvdrddqgvllqiftkpvgdrptfflemiqrigcmekdevg
qeyqkggcggfgkgnfselfksiedyekslevkqsvvaqks
SEQ ID NO. 27 HPPD a/a sequence from wheat 
mpptpttpaatgaaavtpeharprrmvrfnprsdrfhtlafhhvefwcadaasaagr
fafalgaplaarsdlstgnsvhasqllrsgnlaflftapyangcdaataslpsfsad
aarqfsadhglavrsialrvadaaeafrasvdggarpafspvdlgrgfgfaevelyg
dvvlrfvshpdgrdvpflpgfegvsnpdavdygltrfdhvvgnvpelapaaayvagf
tgfhefaefttedvgtaesglnsmvlannsegvllplnepvhgtkrrsqiqtflehh
ggsgvqhiavassdvlrtlremrarsamggfdflppplpkyyegvrriagdvlseaq
ikecqelgvlvdrddqgvllqiftkpvgdrptlflemiqrigcmekdergeeyqkgg
cggfgkgnfselfksiedyeksleakqsaavqgs
SEQ ID NO. 28 HPPD a/a sequence from *Shewanella collwelliana* 
Masegnplgllgieftefatpdldfmhkvfidfgfsklkkhkqkdivyykqndinfl
lnnekqgfsaqfakthgpaissmgwrvedanfafegavargakpaadevkdlpypai
ygigdsliyfidtfgddnniytsdfealdepiitqekgfievdhltnnvhkgtmeyw
snfykdifgftevryfdikgsqtalisyalrspdgsfcipinegkgddrnqideylk
 
eydgpgvqhlafrsrdivasldamegssiqtldiipeyydtifeklpqvtedrdrik
hhqilvdgdedgyllqiftknlfgpifieiiqrknnlgfgegnfkalfesierdqvr
rgvl
SEQ ID NO. 29 HPPD DNA sequence from *Pseudomonas fluorescens* strain 87-79 
atggccgaccaatacgaaaacccaatgggcctgatgggctttgaatttattgaattc
gcatcgccgactccgggcaccctggagccgatcttcgagatcatgggcttcaccaaa
gtcgcgacccaccgctccaagaatgtgcacctgtaccgccagggcgagatcaacctg
atcctcaacaaccagcccgacagcctggcctcgtacttcgccgccgaacacggccct
tcggtgtgcggcatggcgttccgggtcaaagactcgcagcaggcttacaaccgcgcg
ttggaactgggcgcccagccgattcatatcgaaaccggcccgatggaactcaacctg
ccggccatcaagggcatcggcggtgcgccgctgtacctgatcgaccgcttcggtgaa
ggcagctcgatatatgacatcgacttcgtgtacctcgaaggtgtcgaccgcaacccg
gtaggcgcgggcctcaaggtcatcgaccacctgacccacaacgtgtatcgcggccgc
atggcctactgggccaacttctacgagaaactgttcaacttccgtgaagcacgctac
ttcgatatcaagggcgaatacaccggccttacgtccaaggccatgagtgccccggac
ggcatgatccgcatcccgctgaacgaggaatcgtccaagggcgccggccagatcgaa
gagttcctgatgcagttcaacggcgagggcatccagcacgtggcgttcctcaccgaa
gacctggtcaagacctgggatgcgttgaagaagatcggcatgcgcttcatgaccgcg
ccgccggacacctactacgaaatgctcgaaggccgcctgccaaaccacggcgagccg
gtggaccaactgcaggcgcgcggtattttgctggacggctcctcgatcgagggcgac
aagcgcctgctgctgcagatcttctcggaaaccctgatgggcccggtgttcttcgaa
ttcatccagcgcaaaggcgacgatgggtttggcgagggcaacttcaaggcgctgttc
gagtcgatcgagcgcgaccaggtacgtcgcggtgtactgaccaccgac
SEQ ID NO. 30 HPPD DNA sequence from *Arena sativa* 
atgccgcccacccccgccaccgccaccggcgccgccgcggccgccgtgactccagag
cacgcggcccggagctttccccgagtggtccgcgtcaacccgcagcgaccgcttc
cccgtgctctccttccaccacgtcgagctctggtgcgccgacgccgcctcagcggcc
ggacgcttctccttcgcgctcggcgcgccgctcgccgcccggtccgacctctccacg
gggaactccgcgcacgcctccctcctgctccgctcgggcgccctcgccttcctcttc
acggcgccctacgcgccgccgccgcaggaggccgccacggccgcagccaccgcctcc
atcccctccttctccgccgacgccgcgcggacgttcgccgccgcccacggcctcgcg
gtgcgctccgtcggggtccgcgtcgctgacgccgccgaggccttccgcgtcagcgta
gccggcggcgctcgcccggccttcgccccagccgacctcggccatggcttcggcctc
gccgaggtcgagctctacggcgacgtcgtgctacgcttcgtcagctacccggacgag
acagacctgccattcctgccagggttcgagcgcgtgagcagccccggcgccgtggac
tacggcctcacgcggttcgaccacgtcgtgggcaacgtcccggagatggccccggtc
atagactacatgaaaggcttcttggggttccacgagttcgccgagttcaccgccgag
gacgtgggcacgaccgagagcgggctcaactcggtggtgctcgccaacaactccgag
gccgtgctgctgccgctcaacgagcccgtgcacggcacaaagcgacggagccagata
cagacgtacctggagtatcacggcgggcccggcgtgcagcacatcgcgctcgccagc
aacgacgtgctcaggacgctcagggagatgcgggcgcgcacgcccatgggcggcttc
 
gagttcatggcgccaccgcaggcgaaatactatgaaggcgtgcggcgcatcgcaggt
gacgtgctctcggaagagcagatcaaggaatgccaggagctggggtgctagtcgac
agggatgatcaaggggtgttgctccaaatcttcaccaagccagtaggggacaggcca
acgtttttcctggagatgatccaaagaatcgggtgcatggagaaggacgaggtcggg
caagagtaccagaagggtggctgcggcgggtttggcaagggcaatttctccgagctg
ttcaagtccattgaggactatgagaaatcccttgaggtcaagcaatctgttgtagct
cagaaatcctag
SEQ ID No. 31 HPPD cDNA sequence from Wheat 
atgccgcccacccccaccacccccgcagccaccggcgccgccgcggtgacgccggag
cacgcgcggccgcgccgaatggtccgcttcaacccgcgcagcgaccgcttccacacg
ctcgccttccaccacgtcgagttctggtgcggacgccgcctccgccgccggccgc
ttcgccttcgcgctcggcgcgccgctcgccgccaggtccgacctctccacggggaac
tccgtgcacgcctcccagctgctccgctcggcaacctcgccttcctcttcacggcc
ccctacgccaacggctgcgacgccgccaccgcctccctgccctccttctccgccgac
gccgcgcgccagttctccgcggaccacggcctcgcggtgcgctccatagcgctgcgc
gtcgcggacgctgccgaggccttccgcgccagcgtcgacgggggcgcgcgcccggcc
ttcagccctgtggacctcggccgcggcttcggcttcgcggaggtcgagctctacggc
gacgtcgtgctccgcttcgtcagccacccggacggcagggacgtgcccttcttgccg
gggttcgagggcgtgagcaacccagacgccgtggactacggcctgacgcggttcgac
cacgtcgtcggcaacgtcccggagcttgcccccgccgcggcctacgtcgccgggttc
acggggttccacgagttcgccgagttcacgacggaggacgtgggcacggccgagagc
gggctcaactcgatggtgctcgccaacaactcggagggcgtgctgctgccgctcaac
gagccggtgcacggcaccaagcgccggagccagatacagacgttcctggaacaccac
ggcggctcgggcgtgcagcacatcgcggtggccagcagcgacgtgctcaggacgctc
agggagatgcgtgcgcgctccgccatgggcggcttcgacttcctgccacccccgctg
ccgaagtactacgaaggcgtgcggcgcatcgccggggatgtgctctcggaggcgcag
atcaaggaatgccaggagctgggggtgctcgtcgacagggacgaccaaggggtgttg
ctacaaatcttcaccaagccagtaggggacaggccgacgttgttcctggagatgatc
cagaggatcgggtgcatggagaaggacgagagaggggaagagtaccagaagggtggc
tgcggcgggttcggcaaaggcaacttctccgagctgttcaagtccattgaagattac
gagaagtcccttgaagccaagcaatctgctgcagttcaggatcatag
SEQ ID NO. 32 HPPD DNA sequence from *Shewanella collwelliana* 
gactttatgagtcgcacaggtatcgaagcgggctacatgaccttacatcaaaaggc
gtgccgcatggaccacaacctggtcgtactgaagcctcagtgggcaaaactgaaacc
tatgagtatgcagtaatggtggacacctttgcaccactgcaactgacccagcatgtc
aatgcgtgcatgagcaaagattacaaccgttcctggctagaagagtaaaagcgttca
gccagtgctgaacatctaataaatataacaccagaggtgacaccgaagagtgccctt
ggttgcaataagttgaaagaggataattacatggcaagcgaacaaaacccactgggt
ctacttggtatcgaattcactgaatttgctacaccagatctagattttatgcataaa
gtttttatcgactttggtttctcaaacttaaaaaacacaagcagaaagatattgtt
tactataaacaaaatgatattaactttttactcaacaatgaaaaacaggcttttca
gcccagtttgccaaaacgcatggcccagccattagttctatgggctggcgtgtagaa
 
gatgccaactttgcctttgaaggtgctgtagcccgtggggctaaacccgcagcagat
gaggtgaaagatcttccctatcccgctatctatggcattggtgacagccttatctac
tttatcgatacgtttggcgatgacaacaatatctacacttctgattttgaagcgtta
gatgagcctatcatcacccaagagaaaggcttcattgaggtcgaccatctcaccaat
aatgtccataagggcaccatggaatattggtcaaacttctacaaagacatttttggc
tttacagaagtgcgttacttcgacattaagggctcacaaacagctcttatctcttac
gccctgcgctcgccagatggtagtttctgcattccaattaacgaaggcaaaggcgat
gatcgtaaccaaattgatgagtacttaaaagagtacgatggcccaggtgtccaacac
ttagcgttccgtagccgcgacatagttgcctcactggatgccatggaaggaagctcc
attcaaaccttggacataattccagagtattacgacactatctttgaaaagctgcct
caagtcactgaagacagagatcgcatcaagcatcatcaaatcctggtagatggcgat
gaagatggctacttactgcaaattttcaccaaaaatctatttggtccaatttttatc
gaaatcatccagcgtaaaaacaatctcggttttggcgaaggtaattttaaagcccta
tttgaatcgattgagcgtgatcaggtgcgtcgcggcgtactctaacaatcacccagt
gatccaacctcaaaaaccagcatcgcgctggtttttttattgcagcacaacaataa
acctctacactagca
SEQ ID NO. 33 TMV translational enhancer nucleotide sequence 
tatttttacaacaattaccaacaacaacaaacaacaaacaacattacaattactatt
tacaattacac
SEQ ID NO. 34 Fusion of TMV and Arabidopsis HST coding sequence 
tatttttacaacaattaccaacaacaacaaacaacaaacaacattacaattactatt
tacaattacacatatggagctctcgatctcacaatcaccgcgtgttcggttctcgtc
tctggcgcctcgtttcttagcagcttctcatcatcatcgtccttctgtgcatttagc
tgggaagtttataagcctcccacgagatgttcgcttcacgagcttatcaacttcaag
aatgcggtccaaatttgtttcaaccaattatagaaaatctcaatccgggcatgttc
tcaggttggtgctgctgagtctgatgatccagtgctggatagaattgcccggttcca
aaatgcttgctggagatttcttagaccccatacaatccgcggaacagctttaggatc
cactgccttggtgacaagagctttgatagagaacactcatttgatcaaatggagtct
tgtactaaaggcactttcaggtcttcttgctcttatttgtgggaatggttatatagt
cggcatcaatcagatctacgacattggaatcgacaaagtgaacaaaccatacttgcc
aatagcagcaggagatctatcagtgcagtctgcttggttgttagtgatatttttgc
gatagcagggcttttagttgtcggatttaactttggtccattcattacaagcctata
ctctcttggcctttttctgggaaccatctattctgttccacccctcagaatgaaaag
attcccagttgcagcatttcttattattgccacggtacgaggtttccttcttaactt
tggtgtgtaccatgctacaagagctgctcttggacttccatttcagtggagtgcacc
tgtggcgttcatcacatcttttgtgacactgtttgcactggtcattgctattacaaa
ggaccttcctgatgttgaaggagatcgaaagttccaaatatcaaccctggcaacaaa
acttggagtgagaaacattgcattcctcggttctggacttctgctagtaaattatgt
ttcagccatatcactagctttctacatgcctcaggtttttagaggtagcttgatgat
tcctgcacatgtgatcttggcttcaggcttaattttccagacatgggtactagaaaa
agcaaactacaccaaggaagctatctcaggatattatcggtttatatggaatctctt
ctacgcagagtatctgttattccccttcctctag
SEQ ID NO. 35 HST Polypeptide Motif 1.
   W(R/K)FLRPHTIRGT
SEQ ID NO. 36 HST Polypeptide Motif 2.
   NG(Y/F)IVGINQI(Y/F)D
SEQ ID NO. 37 HST Polypeptide Motif 3.
   IAITKDLP
SEQ ID NO. 38 HST Polypeptide Motif 4.
   Y(R/Q)(F/W)(I/V)WNLFY

### EXAMPLES

The average and distribution of herbicide tolerance or resistance levels of a range of primary plant transformation events are evaluated in the normal manner based upon plant damage, meristematic bleaching symptoms *etc.* at a range of different concentrations of herbicides. These data can be expressed in terms of, for example, GR50 values derived from dose/response curves having "dose" plotted on the x-axis and "percentage kill", "herbicidal effect", "numbers of emerging green plants" *etc.* plotted on the y-axis where increased GR50 values may, for example, correspond to increased levels of inherent inhibitor-tolerance (e.g increased Ki x kcat./ Km_{HPP} value) and/or level of expression of the expressed HPPD and/or HST.

The following experiments are conducted using a variety of HST-inhibiting herbicides which are described in Tables A-F.

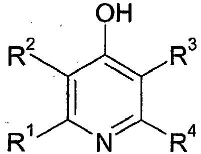

**Table A. Compounds 1.1 - 1.4.**

| Compound | R¹ | R² | R³ | R⁴ |
|---|---|---|---|---|
| 1.1 | F | Cl | Cl | F |
| 1.2 | Cl | Cl | Cl | Cl |
| 1.3 | F | F | Br | F |
| 1.4 | F | Br | Br | F |

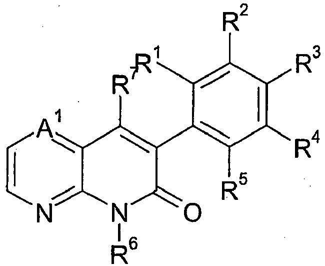

**Table B. Compounds 2.1 - 2.34.**

| Compound | A¹ | R¹ | R² | R³ | R⁴ | R⁵ | R⁶ | R⁷ |
|---|---|---|---|---|---|---|---|---|
| 2.1 | N | F | H | H | Br | Cl | H | OH |
| 2.2 | N | -CH₃ | H | -CH₃ | H | -CH₃ | -CH₃ | OH |
| 2.3 | N | F | H | H | F | Cl | -CH₂CF₂H | OH |
| 2.4 | N | F | H | H | F | Cl | CH₃ | OH |
| 2.5 | N | F | H | H | Br | Cl | CH₃ | OH |
| 2.6 | N | H | H | H | H | -OCF₃ | H | OH |
| 2.7 | N | F | H | H | F | Cl | H | OH |
| 2.8 | N | F | H | H | Br | Cl | -CH₂CF₂H | OH |
| 2.9 | N | H | CF₃ | H | H | Cl | -CH₂CF₂H | OH |
| 2.10 | N | -CH₂CH₃ | H | -CH₂CH₃ | H | -CH₂CH₃ | H | OH |
| 2.11 | N | -CH₃ | H | -CH₃ | H | -CH₃ | -CH₂CF₂H | OH |
| 2.12 | N | H | H | H | H | -OCF₃ | H | OH |
| 2.13 | CH | F | H | H | F | Cl | -CH₂CF₂H | OH |
| 2.14 | N | Cl | Cl | H | H | Cl | -CH₂CF₂H | OH |
| 2.15 | CH | F | H | H | Br | Cl | -CH₂CF₂H | OH |
| 2.16 | N | Cl | H | H | H | -CF₃ | -CH₃ | OH |
| 2.17 | N | F | H | H | F | Cl | -CH₂CF₃ | OH |
| 2.18 | N | F | H | H | F | Cl | -C≡CH | OH |
| 2.19 | N | H | Cl | H | H | CF₃ | -CH₂CF₂H | OH |
| 2.20 | N | Cl | H | H | H | CF₃ | -CH₂CF₂H | OH |
| 2.21 | N | F | H | H | Cl | Cl | -CH₂CF₂H | OH |
| 2.22 | CH | Cl | H | H | Br | Cl | -CH₂CF₂H | OH |
| 2.23 | N | F | H | H | Br | Cl | H | -O(C=O)-*Et* |
| 2.24 | N | -CH₃ | H | -CH₃ | H | -CH₃ | -CH₃ | -O(C=O)-*i*Pr |
| 2.25 | N | F | H | H | Cl | Cl | -CH₂CF₂H | -O(C=O)-*t*Bu |
| 2.26 | CH | F | H | H | F | Cl | CH₃ | -O(C=O)-*t*Bu |
| 2.27 | N | F | H | H | Br | Cl | CH₃ | -O(C=O)-*i*Pr |
| 2.28 | N | Cl | H | H | H | -CF₃ | -CH₂CF₂H | -O(C=O)-*i*Pr |
| 2.29 | N | F | H | H | F | Cl | H | -O(C=O)-*t*Bu |
| 2.30 | N | F | H | H | Br | Cl | -CH₂CF₂H | -O(C=O)-*i*Pr |
| 2.31 | N | Cl | H | H | H | CF₃ | -CH₂CF₂H | -O(C=O)-*i*Pr |
| 2.32 | N | -CH₂CH₃ | H | -CH₂CH₃ | H | -CH₂CH₃ | H | O(C=O)-*i*Pr |
| 2.33 | N | -CH₃ | H | -CH₃ | H | -CH₃ | -CH₂CF₂H | -O(C=O)-*Et* |
| 2.34 | N | H | H | H | H | -OCF₃ | H | -O(C=O)-*i*Pr |

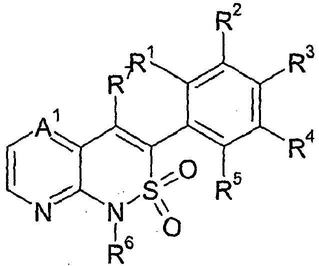

**Table C. Compounds 3.1 - 3.6.**

| Compound | A¹ | R¹ | R² | R³ | R⁴ | R⁵ | R⁶ | R⁷ |
|---|---|---|---|---|---|---|---|---|
| 3.1 | N | CF₃ | H | H | H | Cl | -CH₂CH₃ | OH |
| 3.2 | N | H | H | Cl^{.} | H | Cl | -CH₃ | OH |
| 3.3 | N | Cl | H | H | H | CF₃ | -CH₂CF₂H | OH |
| 3.4 | N | Cl | H | H | Cl | Cl | -CH₂CF₂H | OH |
| 3.5 | N | H | H | Br | H | CF₃ | -CH₂CF₂H | OH |
| 3.6 | N | CF₃ | H | H | Cl | H | -CH₂CF₂H | -O(C=O)-*i*Pr |

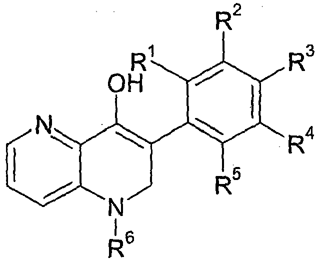

**Table D. Compound 4.1**

| Compound | R¹ | R² | R³ | R⁴ | R⁵ | R⁶ |
|---|---|---|---|---|---|---|
| 4.1 | F | H | H | F | Cl | CH₃ |

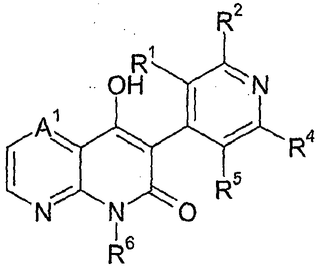

**Table E. Compound 5.1**

| Compound | A¹ | R¹ | R² | R⁴ | R⁵ | R⁶ |
|---|---|---|---|---|---|---|
| 5.1 | N | Cl | H | H | Cl | -CH₂CF₂H |

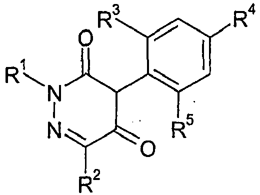

**Table F. Compounds 6.1**

| Compound | R¹ | R² | R³ | R⁴ | R⁵ |
|---|---|---|---|---|---|
| 6.1 | -CH₃ | -CH₃ | -CH₃ | -CH₃ | -CH₃ |

### EXAMPLE 1. Cloning and expressing plant HST enzymes in insect cells and in E. coli

The full length HST coding sequences (minus the ATG start codon) are amplified, with flanking EcoRI sites, from *Arabidopsis* (SEQ ID 11) and Rice (SEQ ID12 or SEQ ID 13) from cDNA libraries or made synthetically. Both these full length and also the truncated coding sequences (encoding the mature sequences starting from ARG 64) *Arabidopsis* SEQ ID 21, rice SEQ ID 22 and rice SEQ ID 23) were cloned into the EcoRI site of the pAcG3X vector (BD Biosciences Cat. No. 21415P) transformed into and then expressed in Sf9 *(Spodoptera fugiperda)* insect cells (as described below) as a N-terminal GST fusion proteins having a factor Xa cleavage site. Similarly, SEQ ID 24, the insect cell codon optimized DNA sequence encoding the alternative truncated mature (ARG 69) *Arabidopsis* HST is cloned into the EcoR1 site of pAcG3X and expressed in Sf9 cells as a N-terminal GST fusion protein. The *Arabidopsis* HST SWISSPROT accession number (protein) is Q1ACB3 and the *Arabidopsis* HST EMBL accession number (DNA): DQ231060.

Alternatively, *Arabidopsis* and *Chlamydomonas* mature HST coding sequences are cloned as GST N-terminal fusion enzymes and expressed in E*.coli.*

### EXAMPLE 2. Growth of Cells and Preparation of HST Enzyme Extracts.

*E.coli.* BL21A1 cells expressing mature *Arabidopsis* or *Chlamydomonas* HST as a GST N-terminal fusion proteins are grown, harvested, broken and membrane fractions expressing HST produced.

For example, 1ng of recombinant DNA is used to transform *BL21 DE3* cells to obtain a plateful of individual colonies. One of these colonies is picked and used to inoculate an overnight culture of 100ml of Luria Broth (LB) supplemented with 50ug/ml of kanamycin at final concentration, grown at 37° C with shaking at 220rpm. Next morning, 10mls of the overnight culture is used to inoculate 1l of fresh sterile LB supplemented with 50ug/ml of kanamycin at final concentration, grown at 37° C with shaking at 220rpm until the OD reached 0.6 at 600nm, induced with the addition of 0.1mM IPTG and left to induce at 15° C overnight. The cells are harvested by centrifugation at 4600 rpm for 10 min at 4° C and the pellet stored at -80°C. For example it is found that one litre of cells yields approximately 5g of wet cell pellet. *E.coli* cell pellet is then resuspended in 25 ml of 50mM Tris, pH 7.5 supplemented with Roche EDTA-free protease inhibitor tablet (one tablet in 200mls of buffer). 10 ml of cells are lysed by sonication on ice. The resultant lysed cells are centrifuged at 3000g for 10min to pellet the cell nuclei/debris etc. 10mls of supernatant is aspirated and centrifuged at 150,000g for 60 min at 4° C. The pellet containing the membranes is resuspended in 2 ml of the above buffer. These samples are stored as 100ul aliquots at -80° C, after being diluted with addition of glycerol to 50% v/v.

The HST expression pAcG3X -derived transfer vectors (described above) are independently co-transformed into Sf9 suspension cells with FlashBac (Oxford Expression Technologies) parental baculovirus vector. Baculovirus amplification and HST protein expression is performed in accordance with the manufacturer's instructions

Sf9 Suspension cell cultures are subcultured at a density of 1.0 EXP 6 cells / ml in 140 ml Sf900II medium (Invitrogen Cat No.10902) in 500 ml Erlenmeyer flasks. After 24 hours culture at 27°C shaking at 120rpm the cell density is measured and readjusted to 2.0 EXP 6 cells / ml in 140 ml. Volumes of amplified virus stock of known titre are added to prepared suspension flasks to give a multiplicity of infection of 10. Flasks are sealed and incubated at 27°C shaking at 125rpm for 72 hours to allow adequate protein expression without cell lysis. Cells are harvested by dividing flask contents evenly between three 50 ml Falcon tubes and centrifuging at 900rpm for 4 minutes. Medium is discarded leaving a 3 ml cell pellet which is snap frozen in liquid nitrogen and maintained at -80 °C

The pellet from 25mls of *Sf9* cells (after induction of expression for 4 days) is resuspended in 10mls of 50mM Tris, pH 7.5 supplemented with Roche-EDTA free protease inhibitor tablet (1tablet in 200mls of buffer) and homogenised using a hand held homogeniser. The resultant lysed cells are centrifuged at 3000g for 10min to pellet the cell nuclei/debris etc. 10mls of supernatant is aspirated and centrifuged at 150,000g for 60 min at 4° C. The pellet containing the membranes is resuspended in 1ml of above buffer and samples are stored as 100ul aliquots at -80° C, after first being diluted with addition of glycerol to 50% v/v.

Western blotting to monitor expression is with anti-GST HRP conjugated Ab (GE Healthcare, 1:5000 working dilution) incubation followed by ECL (GE Healthcare).

HST enzyme preparations for assay are also prepared directly from fresh plant material. For example HST enzyme preparations are from spinach. In the first step intact spinach chloroplasts are prepared from two lots of 500 g of fresh baby spinach leaves (e.g from the salad section of the local supermarket). Prepacked spinach is usually already washed, but if buying loose leaves these must be rinsed in water before proceeding. Stalks, large leaves and mid-ribs are removed. Each 500g lot of leaves is added to 1.5 l of 'Grinding medium' in a 2L plastic beaker. Grinding medium is cold (4 °C) 50 mM Tricine/ NaOH buffer at pH 7.1 containing 330 mM glucose, 2 mM sodium isoascorbate, 5 mM MgCl2 and 0.1% bovine serum albumen The beaker, kept at 4 ° C, is placed under a Polytron 6000 blender, fitted with a 1.5"cutting probe and the mixture blended in short bursts of 5-8sec up to 8-10K rpm until all the leaves are macerated. The homogenate is filtered into a 5L beaker (embedded in an ice bucket) through four layers of muslin, and two layers of 50µ mesh nylon cloth. The filtrate is transferred to 250ml buckets of a Beckman GS-6 centrifuge and spun at 200 x g (3020rpm) for 2 min at 4°C. The supernatant is drained away and discarded to leave a sediment of chloroplasts. Chloroplasts are resuspended in a few ml of cold resuspension medium by gentle swirling and gentle use of a quill brush soaked in resuspension medium. Resuspension medium is 50 mM Hepes/ KOH pH 7.8 containing 330 mM sorbitol, 2 mM EDTA, 5mM KH₂PO₄, 2 mM MgCl2 and 0.1% bovine serum albumen at 4°C. The chloroplasts are resuspended in 5-10 ml of resuspension buffer, recentrifuged down and resuspended again in order to wash them. The chloroplasts are then once again centrifuged down and then broken by resuspension in about 5 ml of 50 mM Tricine-NaOH pH 7.8 to a protein concentration of about 40 mg/ ml. The solution is stored frozen at -80°C in aliquots. This resuspension is defrosted and used directly in HST activity assays. Alternatively, chloroplasts are prepared resuspended in 50 mM Tris/ HCl buffer at pH 7.8 containing 330 mM sorbitol (alternative resuspension buffer) and layered on top of a percol gradient (comprising the same buffer containing 45% percol), spun down, the intact chloroplast fraction taken and washed 2 or 3 times in the alternative resuspension buffer and then spun down again, resuspended in breaking buffer (without sorbitol), flash frozen and stored in aliquots at - 80 °C.

### EXAMPLE 3. Assay of HST enzymes.

Prenyltransferase (HST) activities are measured by determining the prenylation rates of [U-¹⁴C]homogentisate using farnesyl diphosphate (FDP) as prenyl donor. ¹⁴C homogentisate is prepared from ¹⁴C tyrosine using L amino acid oxidase and HPPD.

For inhibitor testing, compounds are dissolved in dimethylsulfoxide (DMSO). DMSO added at up to 2% v/v has no effect on assays. Control assays contain DMSO at the same concentration as in inhibitor containing assays.

Assays using spinach chloroplast extracts (100 µl final volume) contain up to 2 mg of chloroplast protein, 50 mM Tricine-NaOH pH 8.5, 50 mM MgCl₂, 200 µM farnesyl diphosphate (FDP) and 26 µM ¹⁴C-homogentisate (167 dpm/pmol). Assays are run for about an hour at 28 °C. For inhibitor studies, haloxydine at a final concentration of 500 ppm is found to completely inhibit the reaction. Alternatively, stopping the reaction and carrying out solvent extraction at zero time also provides a 100% inhibition baseline reference. Lipophilic reactions products are extracted and analyzed essentially as described in the literature.

The recombinant *Chlamydomonas* HST expressed in *E.coli* membranes is assayed in standard reaction mixtures with 200 µM FDP and 100 µM ¹⁴C-homogentisate (40 dpm/pmol) in 50 mM Tricine-NaOH pH 8.5, 20 mM MgCl₂. Assays are started with the addition of enzyme and run for ∼ 20 min at 28 °C.

Recombinant *Arabidopsis* and Rice HSTs are expressed in insect cells. Assays are run as for *Chlamydomonas* HST except that assay temperature is 27°C. Assays are stopped with 300ul of solvent mix (1:2, Chloroform:Methanol) and 100ul of 0.5% NaCl, agitated / mixed and spun at 13,000rpm in a benchtop eppendorf centrifuge for 5 minutes. 80ul of of the lower phase extract is loaded onto a TLC plate (silica Gel 60, 20cm x 20cm) FLA3000 system and run for 35 minutes in dichloromethane. The radioactivity is quantified using a Fuji Phosphoimager and band intensity integrated as quantitative measure of product amount. The bands corresponding to oxidised and reduced 2-methyl-6-famesyl-1,4-benzoquinol (MFBQ) are identified and the total of the two (oxidised and reduced) band intensities is calculated in order to estimate the total amount of MFBQ product formation. Specific activities of 8 pmol MFBQ min⁻¹ mg⁻¹ protein (23 pmol) and 7 pmol MFBQ min⁻¹ mg⁻¹ protein (14 pmol) are , for example, estimated for the GST-fusion truncated *Arabidopsis* HST gene (SEQ ID # 3) expressed in membranes from insect cells 4 days and 5 days after transfection respectively. Similar results are noted from past literature on *E. coli* expressed *Arabidopsis* HST. Activity from the insect cell expressed GST-fusion truncated rice HST (SEQ ID# 4) is similar. Expression of the non-truncated HST coding sequences as GST fusions also gives HST activity. Expression of the insect cell optimised GST-fusion truncated Arabidopsis HST (SEQ ID#23) gives, for example, an approximately 3-10 fold increase in specific activity over the non-optimized genes.

Using the above assays percentage inhibition of the amount of MFBQ formed at a range of doses of inhibitors relative to the control amount obtained with no inhibitor present is reported (Table 1). The better inhibitors give greater percentage inhibition at lower doses.

It is found that for all of the HST enzyme preparations assayed under the prescribed reaction conditions formation of MFBQ is by no means the only reaction catalysed by HST from ¹⁴C homogentisate(HGA). In fact the major (∼90%) radiolabelled products from the ¹⁴C HGA are not MFBQ. These unknown other products, are also extracted into chloroform/ methanol but, in dichloromethane TLC, are found to stay on or chromatagraph near the base line. Four apparent ¹⁴C -labelled bands (presumably corresponding to two quinone/ quinol pairs) are partly resolved in a second dimension of TLC using 12:3:5:0.5, dichloromethane:hexane:acetonitrile:formic acid. No such bands are seen in the absence of FPP (or indeed when FPP is replaced with pyrophosphate) and, presumptively, the bands correspond to products formed due to farnesylation and decarboxylation not being tightly coupled; .i,e. farnesylation in the absence of decarboxylation (giving rise to carboxylated MFBQ) and decarboxylation in the absence of farnesylation (giving rise to the methyl quinol/ quinone). Whatever their identity it is quite clear that these products more polar than MFBQ are all *bone fide* enzyme reaction products since, in the absence of FDP or using like non-transgenic (non-HST expressing) membranes, they are not formed. In addition it is found that inhibitors such as haloxydine inhibit the formation of these other products in a way that, as dose is varied, is apparently co-linear with inhibition of the formation of MFBQ. Thus 500 ppm haloxydine about completely inhibits the HST enzyme reaction and neither MFBQ nor any of the other products are formed.

Thus in an improved, more sensitive and convenient version of the above assay, the TLC step is dispensed with, treatment with 500 ppm haloxydine (or other inhibitor at a suitable concentration) is used as the 100% inhibition 'control' and a portion of the chloroform/ methanol extract is taken directly into a scintillation vial and counted.

These assays are routinely run at 100µM ¹⁴C HGA, 25°C for <20 min (i.e over a period for which the control assay rate remains linear) and at a range of test inhibitor concentrations used so that IC50s can be derived by curve fitting to the Hill equation (allowing the value of the slope, n, to vary).

### Results.

**TABLE 1. Observed percentage inhibition of the HST reaction (relative to controls) at various concentrations of various compounds using HST from various sources (as labelled). Assays based on TLC and estimated amount of MFBQ formed.**

| **Compound** | **Chlamydomonas. HST %I at 10 ppm** | **Chlamydomonas.HST %I at 25 ppm** | **Chlamydomonas. HST %I at 100 ppm** | **Spinach chloroplast extract %I at 25 ppm** |
|---|---|---|---|---|
| Amitrole | 0 | - | 0 | - |
| Chlorsulfuron | - | 0 | - | 0 |
| 1.1 | 70 | 80 | 95 | 70 |
| 1.2 | - | 70 | - | 80 |
| 1.3 | - | 55 | - | 65 |
| 1.4 | - | 65 | - | 75 |
| 2.1 | 10 | - | 40 | - |
| 2.2 | 70 | - | 95 | - |
| 2.3 | 40 | - | 85 | - |
| 2.4 | 5 | - | 45 | - |
| 2.5 | 70 | - | 90 | - |
| 2.6 | 5 | - | 20 | - |
| 2.7 | 5 | - | 20 | - |
| 2.8 | - | 85 | - | 70 |
| 2.9 | - | 90 | - | 90 |
| 2.10 | - | 50 | - | 0 |
| 2.11 | - | 80 | - | 30 |
| 2.12 | - | 0 | - | 0 |
| 2.13 | - | 60 | - | 40 |
| 2.14 | - | 85 | - | 80 |
| 3.1 | - | 20 | - | 20 |
| 3.2 | - | 0 | - | 15 |
| 3.3 | - | 20 | - | 35 |
| 3.4 | - | 50 | - | 40 |
| 4.1 | 0 | - | 0 | - |
| 5.1 | - | 20 | - | 10 |

**TABLE 2. Estimated IC50 values for inhibition of Arabidopsis HST based on total extraction (non TLC) assay. *These estimates were from 3 point dose curves only conducted at 50 µM HPP.**

| Compound | Estimated IC50 (ppm) | 95% Confidence Limits |
|---|---|---|
| 2.3 | 19* | |
| 2.4 | 211* | |
| 2.5 | 48* | |
| 2.8 | 12 | 10.5 - 14 |
| 2.9 | 6 | 4.5 - 8 |
| 2.11 | 31* | |
| 2.13 | 54* | |
| 2.14 | 9 | 8 - 9.5 |
| 2.15 | 12 | 9.6 - 14 |
| 2.16 | 78 | 51 - 118 |
| 2.17 | 38 | 21 - 68 |
| 2.18 | 6 | 4.3 - 7.5 |
| 2.19 | 6 | 4.5 - 8 |
| 2.20 | 16 | 11 - 25 |
| 2.21 | 64 | 52 - 78 |
| 3.5 | 42 | 28 - 62 |
| 6.1 | 63 | 39 - 102 |

### EXAMPLE 4. Preparation of stable transgenic plants lines expressing a heterologous HST enzyme

For example the *Arabidopsis* HST SEQ ID # 11 is cloned behind a double 35s CMV promoter sequence and a TMV translational enhancer sequence and in front of the 3' terminator from the nos gene. This expression cassette is ligated into pMJB1 (described in WO98/20144) and then into pBIN19 and then transformed into *Agrobacterium tumefaciens* strains LBA4404 prior to plant transformation.

For example, the full length Arabidopsis HST seq ID # 11 is fused to the TMV translational enhancer sequence (SEQ ID #33) by overlapping PCR and, at the same time, 5' XhoI site and a 3'KpnI site are added by PCR. Site-directed mutagenesis is performed to remove an internal XhoI site. The TMV/HPPD fusion is removed from the pBIN19 by digestion with XhoI/KpnI and is replaced by the TMV/HST fusion (SEQ ID #34). The TMV/HST fusion is now cloned behind a double 35s promoter and in front of the 3' terminator from the nos gene. Again the modified pBIN19 vector ('pBinAT HST') is then transformed into Agrobacterium tumefaciens strain LBA4404.

Likewise, vectors for plant transformation are constructed to comprise DNA sequences any HST and, for example, SEQ Ids nos. 12-20.

Alternatively vectors comprise DNA encoding HSTs from photosynthetic protozoans, higher and lower plants the sequences of which are derived from cDNA libraries using methods known to the skilled man. For example total RNA is prepared from 5-20 -day-old plant seedlings using the method of Tri-Zol extraction (Life Technologies). mRNA is obtained, for example, from *Avena sativa* using the Oligotex mRNA purification system (Qiagen). The 5' end of, for example, the *A. sativa* HST gene is identified using 5' RACE, performed using the Gene Racer kit (Invitrogen) with internal HST gene specific primers (based on HST consensus regions e.g SEQ No. 35, 36, 37 and 38). The 3' end of the gene is identified by 3' RACE, performed using Themoscript RT (Life Technologies) with appropriate oligo dT primer and an appropriate internal HST gene primer, followed by PCR All methodologies are performed according to protocols provided by the various stated manufacturers. Products obtained from the 5' and 3' RACE reactions are cloned into pCR 2.1 TOPO (invitrogen) and the cloned products sequenced using universal M13 forward and reverse primers with an automated ABI377 DNA sequencer. Primers are then designed to the translation initiation and termination codons of the HST gene respectively. Both primers are used in conjunction with the One-step RTPCR kit (Qiagen or Invitrogen) to obtain full length coding sequences. Products obtained are cloned into pCR 2.1 TOPO, sequenced, and identified as HST by comparison with sequences known in the art (and for example the HST sequences wherein).

A master plate of *Agrobacterium tumefaciens* containing the binary vector pBinAT HST (described above) or analogous binary vector comprising a different HST is used to inoculate 10 ml LB containing 100 mg / l Rifampicin plus 50 mg / l Kanamycin using a single bacterial colony. This is incubated overnight at 28°C shaking at 200 rpm. This entire overnight culture is used to inoculate a 50 ml volume of LA (plus antibiotics). Again this is cultured overnight at 28°C shaking at 200 rpm.

The Agrobacterium cells are pelleted by centrifuging at 3000 rpm for 15 minutes then resuspended in MS medium with 30 g / l sucrose, pH 5.9 to an OD (600 nM) = 0.6. This suspension is dispensed in 25 ml aliquots into petri dishes.

Clonal micro-propagated tobacco shoot cultures are used to excise young (not yet fully expanded) leaves. The mid rib and outer leaf margins are removed and discarded, the remaining lamina is cut into 1 cm squares. These are transferred to the Agrobacterium suspension for 20 minutes. Explants are then removed, dabbed on sterile filter paper to remove excess suspension then transferred to NBM medium (MS medium, 30 g / 1 sucrose, 1 mg / l BAP, 0.1 mg / l NAA, pH 5.9, solidified with 8 g / l Plantagar), with the abaxial surface of each explant in contact with the medium. Approximately 7 explants are transferred per plate, which are then sealed then maintained in a lit incubator at 25°C, 16 hour photoperiod for 3 days.

Explants are then transferred to NBM medium containing 100 mg / l Kanamycin plus antibiotics to prevent further growth of Agrobacterium (200 mg / l timentin with 250 mg / l carbenicillin). Further subculture on to this same medium is then performed every 2 weeks.

As shoots start to regenerate from the callusing leaf explants these are removed to Shoot elongation medium (MS medium, 30 g / l sucrose, 8 g / l Plantagar, 100 mg / l Kanamycin, 200 mg / l timentin, 250 mg / l carbenicillin, pH 5.9). Stable transgenic plants readily root within 2 weeks. To provide multiple plants per event to ultimately allow more than one herbicide test per transgenic plant all rooting shoots are micropropagated to generate 3 or more rooted clones.

Putative transgenic plants that are rooting and show vigorous shoot growth on the medium incorporating Kanamycin are analysed by PCR using primers that amplified a 500bp fragment within the Arabidopsis HST transgene. Evaluation of this same primer set on untransformed tobacco showed conclusively that these primers would not amplify sequences from the native tobacco HST gene.

To roughly evaluate comparative levels of ectopic HST expression independent PCR positive tobacco shoots have young leaves removed, cut into 1 cm squares and plated onto NBM medium incorporating 1 mg / l haloxydine. These leaf explants produce callus and ultimately regenerated shoots. Those explants over expressing HST regenerated green callus and shoots. Untransformed explants or those from transformants with limited HST expression produced bleached callus and stunted bleached shoots that ultimately died.

It is found that PCR positive events correspond with callus which yields green shoot proliferation (scores > = 3) in the presence of Haloxydine and many more exhibit some level of tolerance that is greater than the untransformed control material.

Rooted transgenic T0 plantlets are transferred from agar and potted into 50% peat, 50% John Innes soil no 3 or, for example, MetroMix® 380 soil (Sun Gro Horticulture, Bellevue, WA) with slow-release fertilizer in 3 inch round or 4 inch square pots and left regularly watered to establish for 8-12d in the glass house. Glass house conditions are about 24-27°C day; 18-21°C night and approximately a 14h (or longer in UK summer) photoperiod. Humidity is ∼ 65% and light levels up to 2000 µmol/ m² at bench level. Once new tissue emerges and plants they have reached the 2-4 leaf stage some of the clones from each event are sprayed with test chemicals dissolved in water with 0.2-0.25% X-77 surfactant and sprayed from a boom on a suitable track sprayer moving at 2 mph in a DeVries spray chamber with the nozzle about 2 inches from the plant tops. Spray volume is suitably 25 gallons per acre or, for example, 200l/ ha.

Test chemicals are, for example, compound 2.3 at 500 g/ha. At the same time that transgenic plants are sprayed so too are w/t Samsun tobacco plants grown from seed as well as non-transgenic plants regenerated from tissue culture and non-transgenic tissue culture escapes. Damage is assessed versus unsprayed control plants of like size and development.

**Table 3. Arabidopsis HST transgenic tobacco plants assessed 11 DAT with compound 2.3 (designated compound 3 in the table). Compared to untreated controls all the plants are affected by treatment at 500g/ ha and are smaller and growth is set back. However, unlike controls that show white meristems and that are essentially dead many of the HST transgenics show green meristems and are recovering and some show essentially no bleaching. Plants are scored on a scale from 0 to 10 with 0 meaning plant substantially bleached / burnt and meristem dead/ white and 10 meaning that the entire plant looks green and undamaged.**

| **EVENT** | **Compound 3 at 500g/ ha** | **EVENT** | **Compound 3 at 500g/ ha** |
|---|---|---|---|
| A1 | 0 | E5 | 1 |
| A12 | 1.5 | E6 | 2.5 |
| A4 | 0 | E8 | 0 |
| A6 | 0 | F12 | 4 |
| A9 | 0.5 | F2 | 4 |
| B1 | 2 | F3 | 1 |
| B3 | 2.5 | F4 | 1 |
| B8 | 5 | F5 | 2 |
| C11 | 2.5 | F7 | 0 |
| C12 | 0 | G1 | 4 |
| C4 | 2 | G4 | 0 |
| C8 | 5 | G5 | 1 |
| C9 | 0 | G9 | 9 |
| D2 | 10 | H8 | 8 |
| D4 | 2 | W/T_SD | 0 |
| D5 | 0 | W/T_SD | 0 |
| D6 | 2.5 | W/T_SD | 0 |
| D8 | 3 | W/T_TC | 0 |
| E1 | 2 | W/T_TC | 0 |
| E10 | 0 | W/T_TC | 0 |

In addition to the above experiments two lines of *Arabidopsis* HST expressing transgenic plants, E9 and F6 were treated with haloxydine at 250 g/ha in a similar manner to the methods described above but at a slightly later growth stage (4-5 leaf). After assessment 8 DAT the w/t plants were 55 and 65% damaged, line F6 plants were 50 and 75% damaged whereas line E9 plants were only 20 and 40% damaged.

At 25 DAT the w/t and F6 plants remained stunted, bleached and small (50-70% damage) whereas the E9 plants now appeared as healthy and similar sized to untreated control plants. Thus expression of the *Arabidopsis* HST confers resistance to haloxydine.

The plants are assessed at various times after treatment up to 28 DAT. Those events (e.g C8, G9, E9) showing the least damage from HST herbicides are grown on to flowering, bagged and allowed to self. The seed from selected events are collected sown on again in pots and tested again for herbicide resistance in a spray test for herbicide resistance. Single copy events amongst the T1 plant lines are identified by their 3:1 segregation ratio (for example, dependent on the construct, by both kanamycin selection and wrt herbicide resistance phenotype) and by quantitative RT-PCR.

### EXAMPLE 5. Production and further testing of T1 and T2 transgenic plants transformed to express Arabidopsis HST , Avena HPPD or Pseudomonas HPPD

T0 transgenic tobacco plant lines B8 and G9 described above in the foregoing examples are selfed. About 50 of the resultant seed from each selfing each line are planted out into a soil/ peat mixture in 3 inch pots, grown in the glass house for 7-10 d and sprayed with 500g/ ha of compound 2.3 (all as described in the foregoing examples). For each line about three quarters of the plants display visible resistance to the herbicide and of these a few plants (possible homozygotes at a single insertion event) appear the most resistant. A few of these more highly tolerant T1 plants are selfed again to produce batches of T2 seed.

6 Seed from w/t Samsun tobacco and 8 T1 seed from events B8 and G9 are also planted out in 3 inch pots , grown on for 7 to 12 d and then, as described in the foregoing examples, the plantlets spray tested for resistance to various chemicals and assessed at 14 DAT. Chemicals are formulated in 0.2% X77 and sprayed at a spray volume of 200 l/ha. Results are depicted in Table 4 below. The results clearly demonstrate the heritability of the herbicide resistance phenotype. The results also show that, aside from obvious non-transgenic segregants, the transgenic Arabidopsis HST Tobacco T1 plants display resistance to HST herbicides as exemplified using compounds 2.15 and 2.30 but that the phenotype is specific and the plants are not significantly tolerant to the other two herbicides tested, norflurazon and atrazine.

**Table 4. Assessment of herbicide % damage to T1 progeny plants of Arabidopsis HST lines B8 and G9 at 14 DAT with various herbicides.**

| Line | Rep | Compound 2.15 1kg/ha | Compound 2.30 150 g/ha | Norflurazon 150g/ha | Atrazine 500g/ha |
|---|---|---|---|---|---|
| | | | | | |
| B8 | 1 | 5 | 60 | 35 | 100 |
| | 2 | 10 | 0 | 15 | 100 |
| | 3 | 15 | 0 | 15 | 100 |
| | 4 | 0 | 0 | 15 | 100 |
| | 5 | 0 | 0 | 15 | 100 |
| | 6 | 0 | 0 | 10 | 100 |
| | 7 | 0 | 0 | 15 | 100 |
| | 8 | 0 | 60 | 35 | 100 |
| G9-2 | 1 | 0 | 0 | 15 | 100 |
| | 2 | 0 | 65 | 20 | 100 |
| | 3 | 0 | 65 | 15 | 100 |
| | 4 | 0 | 0 | 15 | 100 |
| | 5 | 0 | 0 | 15 | 100 |
| | 6 | 0 | 0 | 20 | 100 |
| | 7 | 40 | 0 | 35 | 100 |
| | 8 | 0 | 0 | 40 | 100 |
| WT control | 1 | 30 | 60 | 20 | 100 |
| | 2 | 30 | 60 | 15 | 100 |
| | 3 | 20 | 70 | 10 | 100 |
| | 4 | 50 | 75 | 15 | 100 |
| | 5 | 50 | 75 | 15 | 100 |
| | 6 | 50 | 70 | 15 | 100 |

Tobacco plants expressing the wild-type HPPD gene of Pseudomonas fluorescens strain 87-79 under operable control of the double enhanced 35S CMV promoter region , Nos3' terminator and TMV translational enhancer were provided as detailed in Example 4 of WO0246387. A T0 event exhibiting tolerance to mesotrione was selfed to produce a single insertion T1 line (exhibiting 3:1 segregation of the herbicide tolerance and kanamycin selection phenotypes) which was again further selfed to provide the T2 line designated C2.

Seed of wild-type tobacco plants, C2 tobacco plants and of the T1 progeny of a further Arabidopsis-HST expressing tobacco line, D2 were planted out in 3 inch pots, grown on, sprayed with compounds 1.1, 2.30 and 3.6 at the rates shown in table 5. Percent damage scores were assessed at 7 DAT. Aside from the presumptive non-transgenic segregants the D2 line containing the *Arabidopsis* HST expression construct offered the highest level of tolerance to the two HST herbicides with the *Psuedomonas* HPPD , C2 line, offering only marginal tolerance under the conditions of this test.

**Table 5. Testing of wild-type (WT) Samsun, C2 and D2 tobacco lines versus various HST inhibitors. Results depict % damage at 7 DAT**

| **Compound** | **Rate gai/ha** | **WT** | **WT** | **WT** | **C2** | **C2** | **C2** | **C2** | **D2** | **D2** | **D2** | **D2** | **D2** | **D2** | **D2** | **D2** |
|---|---|---|---|---|---|---|---|---|---|---|---|---|---|---|---|---|
| 1.1 | 25 | 75 | 75 | 75 | 75 | 75 | 75 | 75 | 30 | 55 | 35 | 65 | 65 | 100 | 65 | 55 |
| 2.30 | 150 | 75 | 80 | 85 | 90 | 70 | 80 | 80 | 40 | 65 | 40 | 50 | 55 | 60 | 60 | 90 |
| | 300 | 75 | 70 | 80 | 85 | 85 | 85 | 85 | 90 | 60 | 80 | 60 | 95 | 98 | 70 | 90 |
| | 450 | 70 | 70 | 75 | 65 | 70 | 85 | 85 | 70 | 35 | 20 | 35 | 55 | 55 | 60 | 65 |
| 3.6 | 150 | 80 | 80 | 80 | 60 | 60 | 60 | 60 | 70 | 10 | 70 | 10 | 30 | 20 | 65 | 25 |
| | 300 | 80 | 80 | 75 | 65 | 65 | 65 | 70 | 40 | 65 | 35 | 7 | 85 | 80 | 85 | 75 |
| | 450 | 85 | 85 | 85 | 65 | 65 | 65 | 65 | 25 | 55 | 25 | 65 | 70 | 65 | 80 | 75 |

**Table 6 Testing of T0 lines of Arabidopsis HST tobacco treated with mesotrione. 5 lines of tobacco transformed with Arabidopsis HST were less damaged 10 DAT with 10 g/ha mesotrione than like-treated wild-type lines. Expression of Arabidopsis HST confers a degree of tolerance to the HPPD herbicide, mesotrione.**

| | Damage % | |
|---|---|---|
| Line | **5 DAT** | **10 DAT** |
| 367 | 25 | 10 |
| 332 | 30 | 10 |
| 404 | 45 | 20 |
| 426 | 45 | 20 |
| 351 | 50 | 25 |
| | | |
| wt | 55 | 25 |
| wt | 55 | 60 |
| wt | 55 | 60 |
| wt | 50 | 35 |
| wt | 60 | 60 |
| wt | 60 | 30 |

### EXAMPLE 6. Resistance of plants expressing Avena HPPD to HST herbicides.

Seed of segregating T1 lines of tobacco expressing the wild-type HPPD gene of Avena sativa under operable control of the double enhanced 35S CMV promoter region , Nos3' terminator and TMV translational enhancer were provided as described in WO0246387. About 30-40 T1 seed derived from selfing a mesotrione-tolerant T0 event were grown up to 7-10 d old plantlets sprayed and assessed as described above and the results (at 6 DAT) are depicted in the Table 7 below. Under the conditions of the experiment the Avena HPPD appearsto offer a degree tolerance to both HST inhibitors 2.30 and 3.6.

**Table 7. Testing of wild-type (WT) Samsun tobacco and an Avena HPPD expressing tobacco lines versus mesotrione, compound 2.30 and compound 3.6. Results depict percent damage at 6 DAT.**

| **Line** | **Rep.** | **Mesotrione 100g/ha** | **Compound 2.30 150 g/ha** | **Compound 3.6 300 g/ha** |
|---|---|---|---|---|
| **WT** | **1** | **70** | **70** | **65** |
| | **2** | **80** | **70** | **65** |
| | **3** | **80** | **70** | **65** |
| **Avena HPPD** | **1** | **5** | **55** | **10** |
| | **2** | **10** | **55** | **10** |
| | **3** | **20** | **55** | **5** |
| | **4** | **0** | **55** | **5** |
| | **5** | **0** | **55** | **3** |
| | **6** | **0** | **55** | **10** |
| | **7** | **0** | **40** | **10** |
| | **8** | **5** | **50** | **15** |
| | **9** | **10** | **45** | **5** |
| | **10** | **5** | **40** | **10** |
| | **11** | **2** | **45** | **10** |
| | **12** | **5** | **50** | **10** |
| | **13** | **0** | **50** | **5** |
| | **14** | **0** | **60** | **5** |
| | **15** | **8** | **30** | **15** |
| | **16** | **5** | **40** | **15** |
| | **17** | **3** | **60** | **5** |
| | **18** | **15** | **60** | **10** |
| | **19** | **3** | **60** | **5** |
| | **20** | **0** | **70** | **5** |
| | **21** | **15** | **65** | **5** |
| | **22** | **10** | **70** | **10** |
| | **23** | **5** | **55** | **8** |
| | **24** | **0** | **55** | **5** |
| | **25** | **5** | **55** | **5** |
| | **26** | **0** | **45** | **10** |
| | **27** | **80** | **55** | **15** |
| | **28** | **5** | **55** | **10** |
| | **29** | **3** | **45** | **5** |
| | **30** | **3** | **60** | **10** |

### EXAMPLE 7. Tobacco, transformation and selection of HST-expressing transformants on HST herbicides

A master plate of *Agrobacterium tumefaciens* containing the binary vector pBinAT HST (described in example 6) is used to inoculate 10 ml LB containing 100 mg / l Rifampicin plus 50 mg / l Kanamycin using a single bacterial colony. This is incubated overnight at 28°C shaking at 200 rpm.

This entire overnight culture is used to inoculate a 50 ml volume of LA (plus antibiotics). Again this is cultured overnight at 28°C shaking at 200 rpm. The *Agrobacterium* cells are pelleted by centrifuging at 3000 rpm for 15 minutes then resuspended in MS medium with 30 g / 1 sucrose, pH 5.9 to an OD (600 nM) = 0.6. This suspension is dispensed in 25 ml aliquots into petri dishes.

Clonal micro-propagated tobacco shoot cultures are used to excise young (not yet fully expanded) leaves. The mid rib and outer leaf margins are removed and discarded, the remaining lamina is cut into 1 cm squares. These are transferred to the *Agrobacterium* suspension for 20 minutes. Explants are then removed, dabbed on sterile filter paper to remove excess suspension then transferred to NBM medium (MS medium, 30 g / l sucrose, 1 mg / l BAP, 0.1 mg / l NAA, pH 5.9, solidified with 8 g / l Plantagar), with the abaxial surface of each explant in contact with the medium. Approximately 7 explants are transferred per plate, which are then sealed then maintained in a lit incubator at 25°C, 16 hour photoperiod for 3 days.

Explants are then transferred to NBM medium containing 0.5 mg / 1 Haloxydine plus antibiotics to prevent further growth of *Agrobacterium* (200 mg / l timentin with 250 mg / l carbenicillin). Further subculture on to this same medium is then performed every 2 weeks.

As shoots start to regenerate from the callusing leaf explants these are removed to Shoot elongation medium (MS medium, 30 g / l sucrose, 8 g / l Plantagar, Hung / l Haloxydine (or similar or concentration of any other HST herbicide at an appropriate discriminating concentration), 200 mg / l timentin, 250 mg / l carbenicillin, pH 5.9). Shoots that root and continue to proliferate are analysed for stable integration of the HST transgene by PCR. Ultimately these rooted shoots are transferred to soil and progressed under glasshouse conditions. T1 seed is produced from selected T0 lines,

Thus it is found that the use of a HST gene in combination with a HST-inhibitor herbicide provides a means for the selection of transgenic plant tissue

### EXAMPLE 8. Preparation and testing of stable transgenic plants lines expressing a heterologous HPPD enzyme

Transgenic lines of tobacco, soyabean and corn etc. can be engineered to express various heterologous HPPDs derived from, for example Avena (SEQ ID #26), Wheat (SEQ ID #27), *Pseudomonas fluorescens* (SEQ ID # 25) and *Shewanella collvelliana* (SEQ ID #28) as, for example, described in WO 02/46387.

The seed from selected events are collected sown on again in pots and tested again for herbicide resistance in a spray test for resistance to HPPD herbicide (for example mesotrione). Single copy events amongst the T1 plant lines are identified by their 3:1 segregation ratio (wrt kanamycin and or herbicide) and by quantitative RT-PCR. Seed from the thus selected T1 tobacco (var Samsun) lines are sown in 3 inch diameter pots containing 50% peat, 50% John Innes soil no 3. After growth to the 3 leaf stage, plants are sprayed, as described above, in order to test for herbicide tolerance relative to like- treated non-transgenic tobacco plants.

Control tobacco plants and transgenic T1 plants expressing either the *Pseudomonas* or the wheat HPPD gene are sprayed at 37, 111, 333 and 1000 g/ha rates of HST inhibitors and, for example, compound 2.3.

Plants are assessed and scored for % damage at 16 DAT.

**TABLE 8. Comparing % damage observed 16 DAT of w/t tobacco plants with transgenic plants expressing either Pseudomonas or Wheat HPPD**

| **Compound** | **RATE g/ha** | **Pseudomonas HPPD** | **Wheat HPPD** | **w/t tobacco** |
|---|---|---|---|---|
| Mesotrione | 37 | 3 | 0 | 66 |
| | 111 | 3 | 2 | 57 |
| | 333 | 43 | 0 | 57 |
| | 1000 | 67 | 13 | 54 |
| Compound 2.3 | 37 | 0 | 0 | 17 |
| | 111 | 3 | 0 | 40 |
| | 333 | 6 | 3 | 47 |
| | 1000 | 10 | 8 | 57 |

It is thus observed that expression of either HPPD gene provides tobacco with a high level of resistance to treatment with the HST inhibitor, 2.3 as well as to mesotrione. Treated w/t tobacco plants show white bleached meristems whereas transgenic HPPD expressing plants have green healthy meristems and new leaves and look almost undamaged. In this test plants were relatively large at the time of spraying and thus controls were not completely controlled.

### EXAMPLE 9. Preparation of transgenic plants lines expressing different heterologous HST and HPPD enzymes and stacked combinations thereof. Glasshouse testing for herbicide tolerance

The full length Arabidopsis (plus start codon) HST seq ID 11 is cloned behind a double 35s CMV promoter sequence and a TMV translational enhancer sequence and in front of the 3' terminator from the nos gene as described previously. As described above this expression construct is cloned into a binary vector (pBIN 35S Arabidopsis HST) that is transformed into tobacco to produce populations of 30-50 transgenic events which are subdivided at the callus stage to produce 2-4 clonal plants from each transgenic 'event' which are then regenerated and transferred into soil before transfer to the glass hosue and testing.

In just the same way the Chlamydomonas HST gene sequence (AM285678) is codon-optimised for tobacco and cloned behind the double Cauliflower mosaic virus 35S promoter and Tobacco mosaic virus enhancer sequences and in front of a nos gene terminator, cloned into a binary vector and transformed into tobacco to produce a population of T0 plants.

Exactly as described in example 5 of WO 02/46387 the wheat HPPD gene sequence (Embl DD064495) is cloned behind an Arabidopsis Rubisco small subunit (SSU) promoter and in front of a nos gene terminator to produce an 'SSU Wheat HPPD nos expression cassette' which is cloned into a binary vector and transformed into tobacco to produce a population of 30-50 transgenic events.

A "pBin Arabidopsis HST/Wheat HPPD" vector is also built in order to provide a population of plants that co-express the HST and HPPD enzymes. The SSU Wheat HPPD nos cassette (described above) is cloned into the EcoRI site of the pBin 35S Arabidopsis HST vector (described above) to generate the HST/HPPD expression construct and binary vector. Again this is transformed into tobacco to produce a population of primary transformants.

Alternatively, transgenic plants expressing both HPPD and HST are produced by first transforming to express either a heterologous HST or HPPD and then the progeny tissue are subsequently transformed with a construct designed to express the other enzyme. For example, as described in WO 02/46387 tobacco plants are transformed to express wheat, A vena or *Pseudomonas* HPPD under expression control of the Arabidopsis small subunit of rubisco promoter, TMV translational enhancer and nos gene 3' terminator. Examples of T0 events highly tolerant to mesotrione are selfed on to make T1 seed. Approximately 100 of these T1 seeds from a single event are surface sterilised using 1 % Virkon for 15 minutes then following washing in sterile water plated onto MS medium with 20 g / 1 sucrose, 100 mg / l Kanamycin, pH 5.8 solidified with 8 g / l plantagar. Individual plants are picked from the mixed population of hemizygous and homozygous plants that germinate, grown on *in vitro* and micropropagated to provide a clonal recombinant shoot culture. Leaves from these shoot cultures are subject to transformation using constructs and selection methods described previously. To initially evaluate whether co-expression of HPPD and HST results in elevated levels of plant resistance to mesotrione compared to expression of HPPD alone, shoot culture derived leaf explants from HPPD only and HPPD plus HST transfonnants are plated onto NBM medium containing a range of mesotrione concentrations between 0.1 to 5 mg / l. Explants from transgenics combining HPPD and HST may exhibit green callus and more limited bleaching of regenerating shoots at higher mesotrione concentrations than the HPPD only 'background' explants from the clonal single plant, HPPD event derived material.

'Control'plantlets are regenerated from the untransformed (with HST) HPPD-expressing clonal background material derived from a single plant of a single event. T0 HST transgenic plantlets that are additionally transformed with HST are selected against this background as described in the previous example on haloxydine, and are also regenerated. Plantlets are micropropagated into further clones, rooted and grown on in pots in the glass house as described in the previous examples.

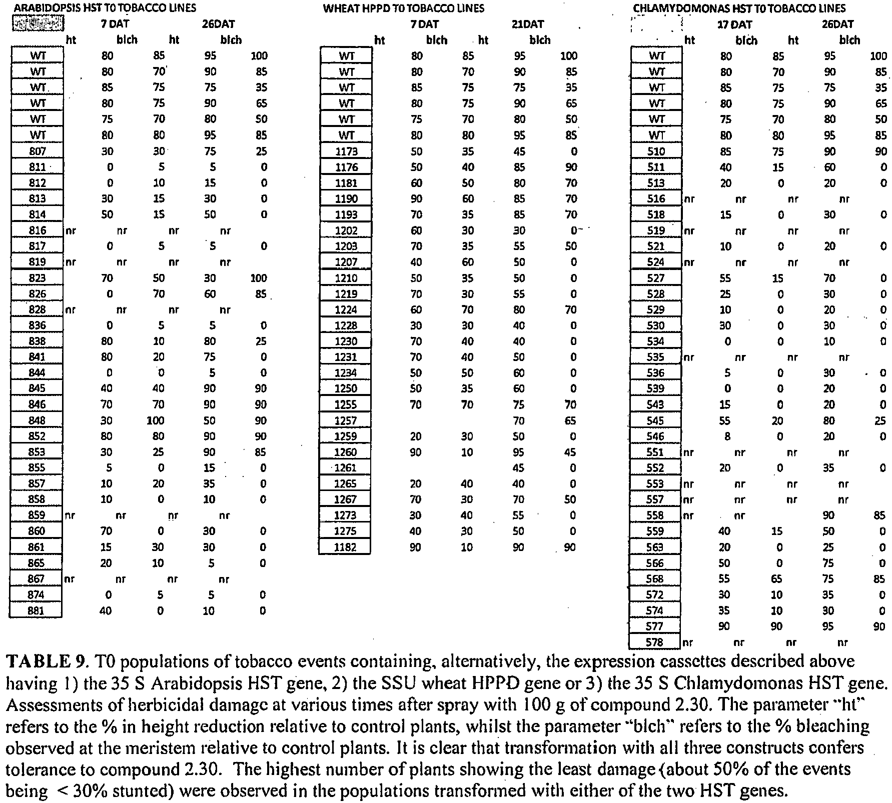

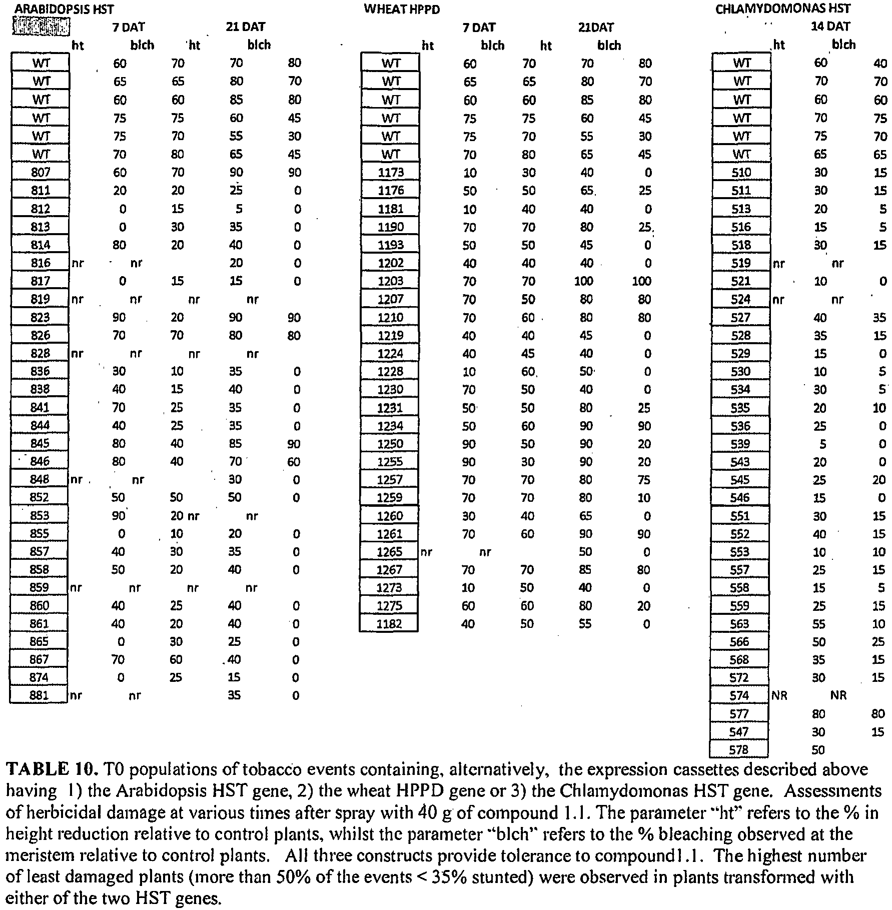

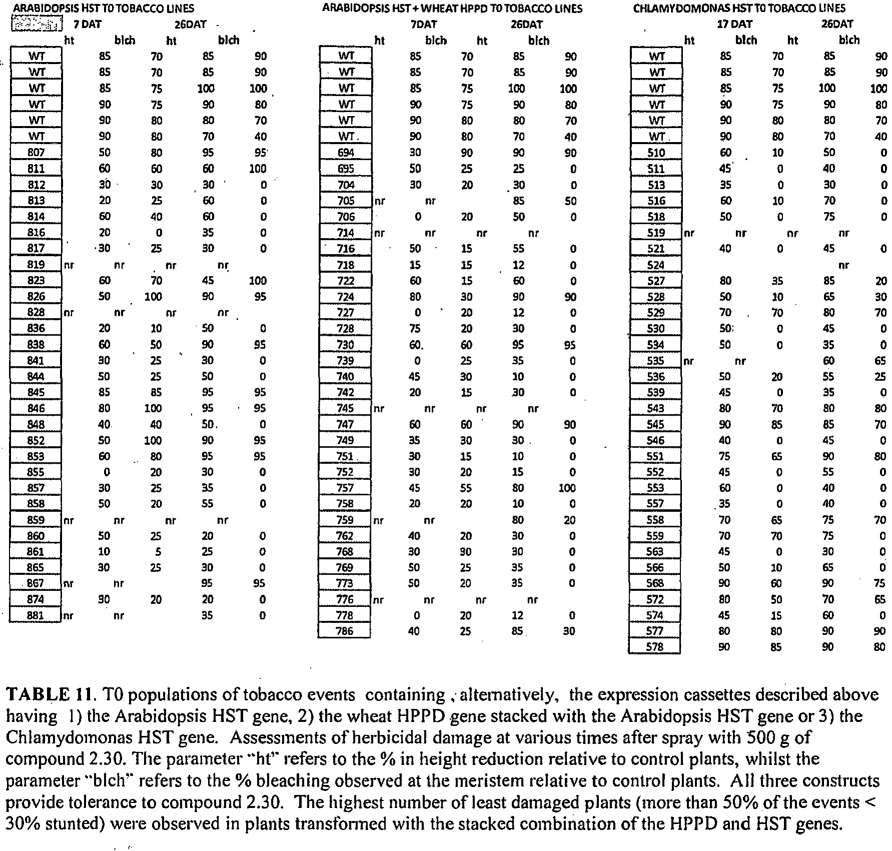

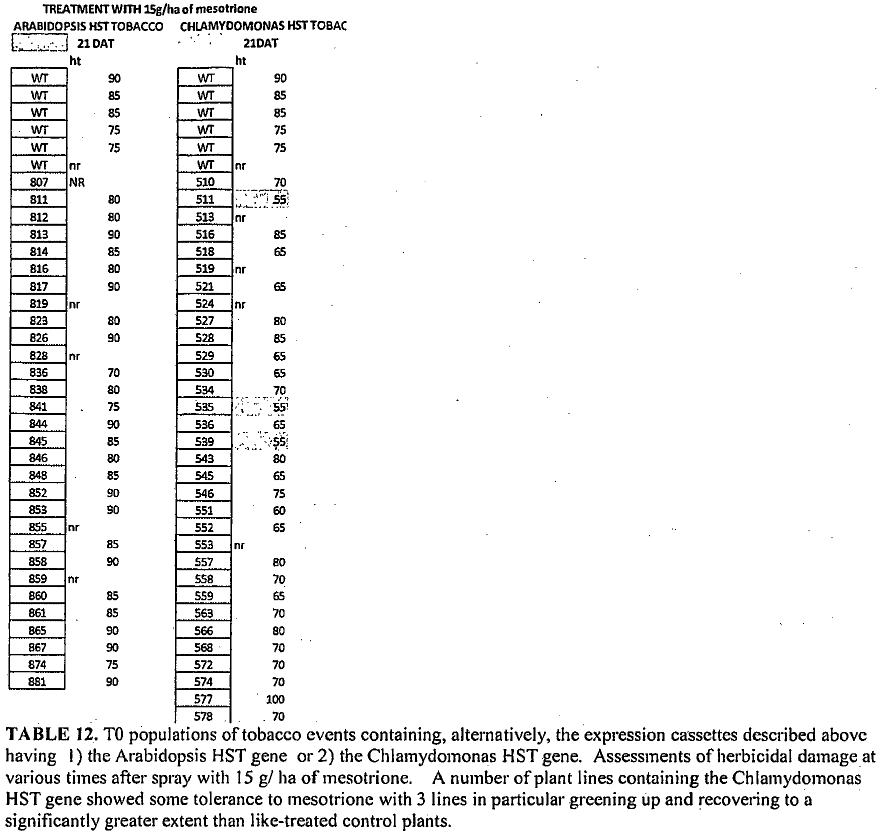

### EXAMPLE 10: Construction of soybean transformation vectors.

A binary vector (17107) for dicot (soybean) transformation is, for example, constructed, with the *Arabidopsis* UBQ3 promoter driving expression of the *Chlamydomonas* HST coding sequence (SEQ ID # 15), followed by Nos gene 3' terminator. The gene is codon optimized for soybean expression based upon the predicted amino acid sequence of the HST gene coding region. The amino acid sequence of the protein encoded by *Chlamydomonas* HST gene is provided in SEQ ID # 5. Optionally the transformation vector also contains two PAT gene cassettes (one with the 35S promoter and one with the CMP promoter, and both PAT genes are followed by the nos terminator) for glufosinate based selection during the transformation process.

A similar binary vector (17108) is similarly constructed but also comprising an expression cassette expressing the soyabean codon-optimized *Avena* HPPD gene. In this case there is no PAT gene and selection is carried out using a HPPD herbicide or, as described herein, a HST herbicide.

### Example 11: Soybean T0 Plant establishment and selection.

Soybean transformation is achieved using methods well known in the art. T0 plants were taken from tissue culture to the greenhouse where they are transplanted into saturated soil (Redi-Earth® Plug and Seedling Mix, Sun Gro Horticulture, Bellevue, WA) mixed with 1% granular Marathon® (Olympic Horticultural Products, Co., Mainland, PA) at 5-10 g/gal Redi-Earth® Mix in 2" square pots. The plants are covered with humidity domes and placed in a Conviron chamber (Pembina, ND) with the following environmental conditions: 24°C day; 18°C night; 23 hr photoperiod; 80% relative humidity.

After plants became established in the soil and new growth appeared (∼1-2 weeks), plants are sampled and tested for the presence of desired transgene by Taqman™ analysis using appropriate probes for the HST and/or HPPD genes, or promoters (for example prCMP and prUBq3). All positive plants and several negative plants are transplanted into 4" square pots containing MetroMix® 380 soil (Sun Gro Horticulture, Bellevue, WA). Sierra 17-6-12 slow release fertilizer is incorporated into the soil at the recommended rate. The negative plants serve as controls for the spray experiment. The plants are then relocated into a standard greenhouse to acclimatize (∼1 week). The environmental conditions are: 27°C day; 21°C night; 12 hr photoperiod (with ambient light); ambient humidity. After acclimatizing (∼1 week), the plants are ready to be sprayed with the desired herbicides.

### Example 12 HPPD/ HST herbicide mixtures. Effect of adding small amounts of HPPD inhibitor on the herbicidal activity of HST herbicides

Tobacco (var Samsun) plantlets germinated aseptically in agar made up in 1/3 strength Murashige and Skoog salts medium along with various doses of herbicide. Bleaching damage to emerging plantlets is assessed 7 DAT. The plantlets are kept covered under clear perspex and grown at 18 °C (night) and 24° C (day) under a 16h day (∼ 500-900 umol/ m²), 8 h darkness regime. Herbicide affected plantlets are bleached white and grow less. Synergistic / antagonistic responses are calculated using the Colby formula (Colby, S. R. (Calculating synergistic and antagonistic responses of herbicide Combinations", Weeds, 15, p. 20-22, 1967).

**TABLE 13. HST + HPPD herbicide effects on tobacco seedlings in agar. The % bleaching observed 7 DAT of germinating tobacco seeds in agar is assessed with various doses of haloxydine and 0.75% v/v DMSO in the presence or absence of 0.004 ppm mesotrione. At this dose the mesotrione by itself consistently gives 35% bleaching damage and the expected values for the damage in mixture with the various doses of haloxydine are therefore calculated accordingly as described by Colby (1967).**

| **% Injury** | | | | |
|---|---|---|---|---|
| **[Haloxydine] / ppm** | **Haloxydine only** | **Plus Mesotrione (0.004ppm) (OBSERVED)** | **plus Mesotrione (0.004ppm) (EXPECTED)** | **(O-E)** |
| 37.5 | 100 | 100 | 100 | 0 |
| 18.8 | 100 | 100 | 100 | 0 |
| 9.4 | 90 | 100 | 94 | 6 |
| 4.7 | 70 | 100 | 80 | 20 |
| 2.4 | 50 | 90 | 67.5 | 22.5 |
| 1.2 | 35 | 70 | 58 | 12 |
| 0.6 | 20 | 50 | 48 | 2 |
| 0.3 | 10 | 50 | 41.5 | 8.5 |
| 0.75% v/v DMSO | 0 | 35 | 35 | 0 |

In an alternative herbicide test procedure tobacco (var Samsun) plantlets germinated aseptically in agar made up in 1/3 strength Murashige and Skoog salts medium are transferred after 4d to float on top of 2.9 ml of sterile liquid culture medium (half strength Murashige and Skoog medium containing 30 mM sucrose) in wells of 12 well plates. Test compounds are added at various doses and bleaching damage is assessed after 14-20 DAT. The plantlets are kept covered under clear perspex and grown at 18 °C (night) and 24° C (day) under a 16h day (∼ 500-900 umol/ m²), 8 h darkness regime. Plantlets continue to grow and produce new tissue over the 14-20 DAT period but are bleached and grow less in the presence of controlling concentrations of herbicide.

**TABLE 14. HST + HPPD herbicide effects on tobacco seedlings growing on liquid. The % bleaching observed 20 DAT of tobacco seedlings on liquid culture medium is assessed versus the presence of various concentrations of the HST herbicide, compound 2.13 with 0.75% v/v DMSO in the presence or absence of 0.001 or 0.0005 ppm of mesotrione. At these doses the mesotrione by itself produced either minimal, 20% , or zero bleaching damage.**

| | **% Injury** | | |
|---|---|---|---|
| **Compound 2.13 / ppm** | **compound 2.13 only** | **Plus Mesotrione (0.001ppm)** | **plus Mesotrione (0.0005ppm)** |
| 23 | 100 | 100 | 100 |
| 7.67 | 50 | 100 | 100 |
| 2.56 | 0 | 90 | 90 |
| 0.85 | 0 | 90 | 40 |
| 0.28 | 0 | 80 | 0 |
| 0.09 | 0 | 80 | 0 |
| 0.03 | 0 | 60 | 0 |
| 0.01 | 0 | 40 | 0 |
| 0.75% v/v DMSO | 0 | 20 | 0 |

From the data provided it is apparent that addition of a low dose of mesotrione synergises the herbicidal effect of the HST inhibitor, haloxydine on agar grown tobacco plantlets.

**Table 15a**

| Haloxydine ppm | Haloxydine alone | + mesotrione 0.001 ppm |
|---|---|---|
| 47 | 100 | 100 |
| 16 | 100 | 100 |
| 5.2 | 100 | 100 |
| 1.7 | 100 | 100 |
| 0.58 | 90 | 100 |
| 0.19 | 50 | 100 |
| 0.75% DMSO | 0 | 5 |

**Table 15b**

| Compound 2.15 ppm | Compound 2.15 alone | + mesotrione 0.001 ppm |
|---|---|---|
| 47 | 100 | 100 |
| 16 | 100 | 100 |
| 5.2 | 40 | 70 |
| 1.7 | 5 | 80 |
| 0.58 | 0 | 5 |
| 0.19 | 5 | 0 |
| 0.75% DMSO | 0 | 5 |

**TABLES 15a and 15b. HST + HPPD herbicide injury on tobacco seedlings growing on liquid culture medium.** The % bleaching observed 14 DAT of tobacco seedlings on liquid culture medium is assessed versus the presence of various concentrations of the HST herbicides, haloxydine (Table 15a), and compound 2.15 (Table 15b), with 0.75% v/v DMSO in the presence or absence of 0.001 ppm mesotrione. At this dose mesotrione produced minimal (0-5%v) damage.

In liquid culture the synergising effect of mesotrione on the activity of HST inhibitor, 2.13 is even more apparent than that on haloxydine. Even addition of a dose of mesotrione that itself produces no visible, damage at all results in levels of 40, 90 and 100% bleaching injury at doses of compound 2.13 where the expected level of control (according to Colby) is only 0, 0 and 50%. Similarly, at the higher dose of mesotrione, 80 or 90% bleaching is observed across a range of rates of compound 2.13 where only 20% is expected. Similarly, under similar conditions and in repeat experiments, there are clear synergistic effects of low amounts of mesotrione on the injury observed down haloxydine and compound 2.15 dose responses.

### EXAMPLE 13. GLASS HOUSE WEED CONTROL BY MIXTURES OF HST AND HPPD HERBICIDES

Weed seeds are planted out in trays containing suitable soil (for example 50% peat, 50% John Innes soil no 3) and grown in the glass house conditions under 24-27°C day; 18-21°C night and approximately a 14h (or longer in UK summer) photoperiod.

Humidity is ∼ 65% and light levels up to 2000 µmol/ m² at bench level. Trays are sprayed with test chemicals dissolved in water with 0.2-0.25% X-77 surfactant and sprayed from a boom on a suitable track sprayer moving at about 2 mph in a suitable track sprayer (for example a DeVries spray chamber with the nozzle about 2 inches from the plant tops). Spray volume is suitably 500 - 1000 l/ha. Sprays are carried out both pre-emergence and over small plants at about 7-12 d post-emergence

Plants are assessed 14 DAT and herbicidal damage is scored on a scale from 0 to 100%. The HST inhibitor compound 2.30 (designated compound A in table 16 and 17), haloxydine (compound 1.1) and compound 2.13 (designated compound AE in tables 16 and 17) are sprayed at rates between 0 and 500g/ ha. Mesotrione is applied at a very low rate of 1g/ha at which it causes essentially no (< 10% damage).

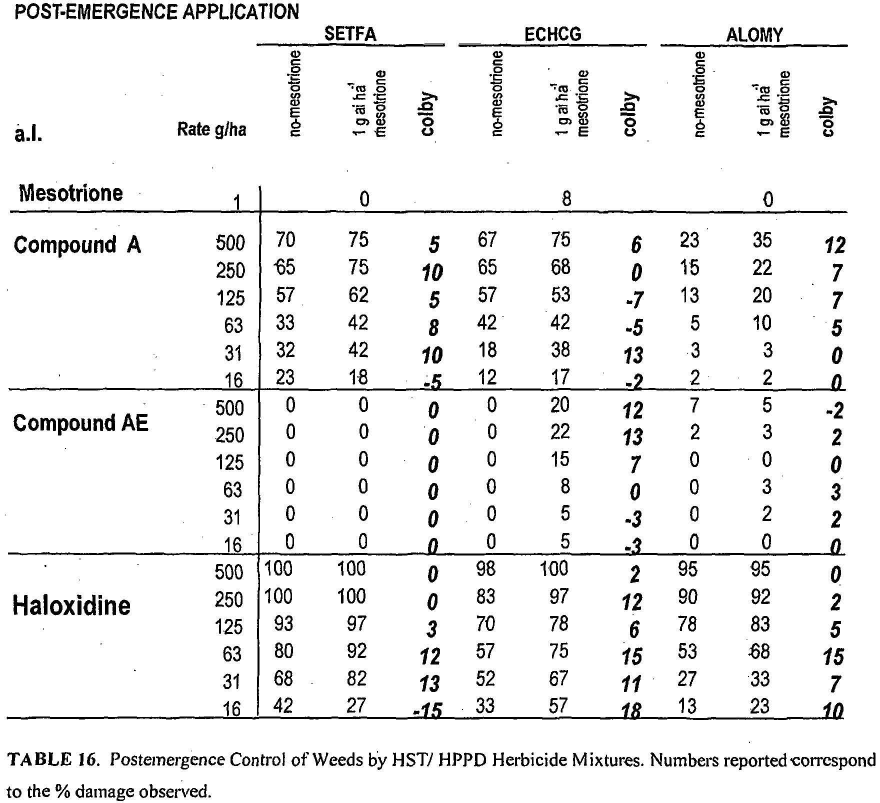

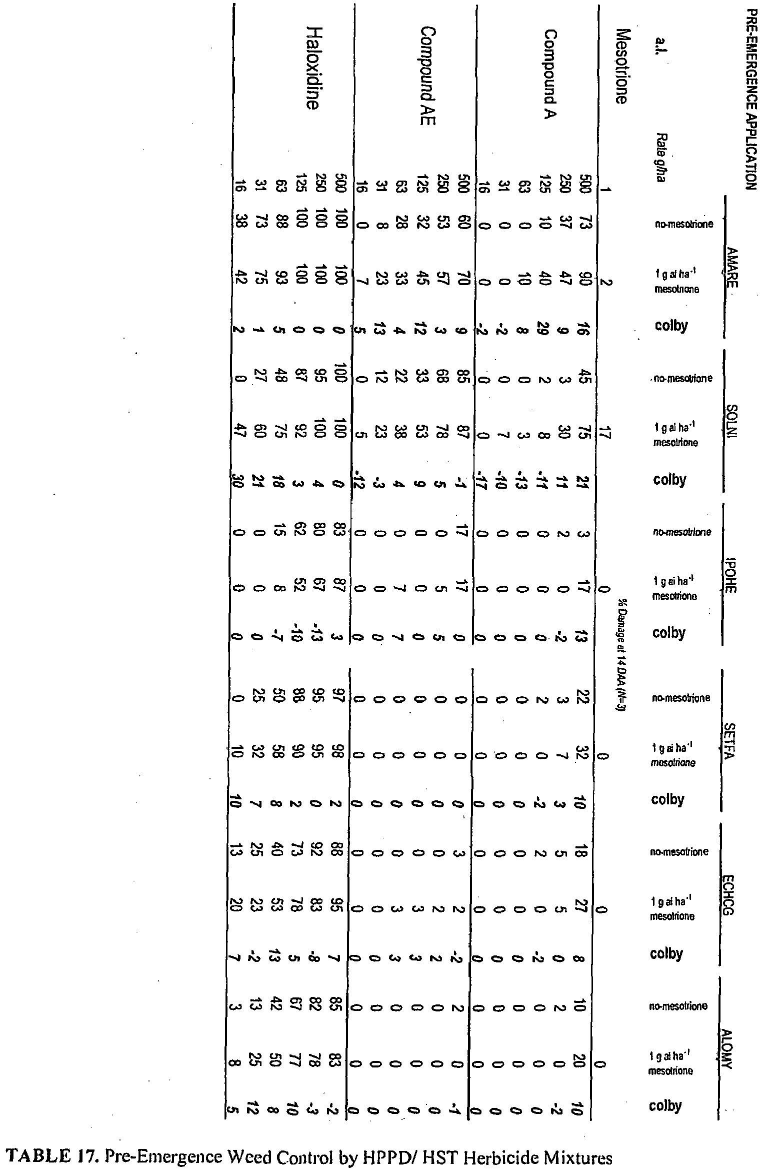

### EXAMPLE 14. Further studies showing Synergy between HPPD and HST herbicides

In a further test, the results of which are depicted in tables 18 and 19, weed seeds are planted out in trays containing 50% peat/ 50% John Innes no. 3 soil and grown in the glass house at 24- 27 C day; 18-21 C night and approximately a 15h photoperiod. Humidity is ∼ 65% and light levels at bench level are up to 2mmol/ m². Again all spray chemicals are dissolved in 0.2% X77 surfactant and sprayed from a boom on a track sprayer moving at 2 mph with the nozzle set about 2 inches above the plant tops. The spray volume is 500l/ ha. Sprays are carried out both pre-emergence and post-emergence over small plants at about 7-12d post-emergence. Plants are assessed at 14 DAT with herbicidal damage scored on a scale from 0 to 100%. The HPPD inhibiting herbicide is compound A22 (4-hydroxy-3-[[2-(2-methoxyethoxy)methyl]-6-(trifluoromethyl)-3-pyridinyl]carbonyl]-bicyclo[3.2.1]oct-3-en-2-one) which is sprayed at 2g/ ha both alone and in mixture with various HST herbicides. Results and spray rates are depicted in Tables 18 and 19. Again the Colby formula has been used to calculate synergy scores observed following treatment with the mixture based on the results obtained with the single components alone. Positive synergy is observed between the HPPD herbicide and a wide variety of HST inhibitor herbicides applied both pre and postemergence across a variety of weeds.

**TABLE 18 (continued). Postemergence weed control of a range of weeds. % control scores following sprays with a variety of HST inhibitors alone and in mixture with A22 (single replicate tests only)**

| Compound | Rate (g/ha) | SCORES 14DAT POST_EMERGENCE TREATMENT | | | | | | | | | | | |
|---|---|---|---|---|---|---|---|---|---|---|---|---|---|
| | | SIDSP | | DIGSA | | PANMI | | BRAPL | | SETFA | | ECHCG | |
| | | Obs | Colby (O-E) | Obs | Colby (O-E) | Obs | Colby (O-E) | Obs | Colby (O-E) | Obs | Colby (O-E) | Obs | Colby (O-E) |
| \|Compound A22 alone | 2 | 20 | | 5 | | 50 | | 2 | | 10 | | 30 | |
| | 2 | 15 | | 5 | | 55 | | 2 | | 2 | | 30 | |
| | | | | | | | | | | | | | |
| Compound 2.15 alone | 250 | 15 | | 0 | | 0 | | 5 | | 2 | | 2 | |
| | 62.5 | 10 | | 0 | | 0 | | 0 | | 0 | | 0 | |
| | 15.625 | 0 | | 0 | | 0 | | 0 | | 0 | | 0 | |
| | | | | | | | | | | | | | |
| Compound 2.15 + 2 g/ ha compound A22 | 250 | 60 | 30.125 | 10 | 5 | 50 | 2.5 | 30 | 23.1 | 10 | 4.08 | 60 | 28.6 |
| | 62.5 | 50 | 24.25 | 10 | 5 | 50 | -2.5 | 20 | 18 | 0 | -4 | 50 | 20 |
| | 15.625 | 30 | 12.5 | 0 | -5 | 40 | -12.5 | 10 | 8 | 0 | -4 | 40 | 10 |
| | | | | | | | | | | | | | |
| \|Compound 3.5 alone | 250 | 10 | | 5 | | 70 | | 65 | | 40 | | 30 | |
| | 62.5 | 10 | | 0 | | 15 | | 30 | | 10 | | | |
| | 15.625 | 2 | | 0 | | 5 | | 2 | | 2 | | 15 5 | |
| | | | | | | | | | | | | | |
| Compound 3.5 + 2 g/ ha compound A22 | 250 | 30 | 4.25 | 10 | 0.25 | 75 | -10.75 | 50 | -15.7 | 55 | 12.5 | 60 | 9 |
| | 62.5 | 25 | 0.75 | 2 | -3 | 75 | 15.375 | 40 | 86 | 30 | 16.4 | 50 | 9.5 |
| | 15.625 | 25 | 5.85 | 0 | -5 | 70 | 15.125 | 2 | -1.96 | 5 | -0.92 | 50 | 16.5 |
| | | | | | | | | | | | | | |
| \|Compound 2.21 alone | 250 | 40 | | 20 | | 70 | | 80 | | 60 | | 40 | |
| | 62.5 | 20 | | 10 | | 30 | | 30 | | 20 | | 10 | |
| | 15.625 | 5 | | 0 | | 10 | | 5 | | 2 | | 0 | |
| | | | | | | | | | | | | | |
| Compound 2.21 + 2 g/ ha compound A22 | 250 | 50 | -0.5 | 60 | 36 | 80 | -5.75 | 80 | -0.4 | 60 | -1.6 | 65 | 7 |
| | 62.5 | 50 | 16 | 50 | 35.5 | 70 | 3.25 | 75 | 43.6 | 50 | 26.8 | 65 | 28 |
| | 15.625 | 30 | 8.375 | 20 | 15 | 75 | 17.75 | 30 | 23.1 | 50 | 44.08 | 50 | 20 |
| | | | | | | | | | | | | | |
| \|Compound 2.14 alone | 250 | 50 | | 20 | | 90 | | 60 | | 70 | | 65 | |
| | 62.5 | 30 | | 20 | | 60 | | 60 | | 60 | | 50 | |
| | 15.625 | 15 | | 10 | | 40 | | 30 | | 25 | | 30 | |
| | | | | | | | | | | | | | |
| Compound 2.14 + 2 g/ ha compound A22 | 250 | 60 | 1.25 | 60 | 36 | 80 | -15.25 | 80 | 19.2 | 75 | 3.8 | 80 | 4.5 |
| | 62.5 | 50 | 7.75 | 40 | 16 | 80 | -1 | 60 | -0.8 | 70 | 8.4 | 70 | 5 |
| | 15.625 | 50 | 20.125 | 30 | 15.5 | 75 | 3.5 | 50 | 186 | 55 | 27 | 60 | 9 |
| | | | | | | | | | | | | | |
| \|Compound 2.19 alone | 250 | 50 | | 70 | | 85 | | 80 | | 80 | | 75 | |
| | 62.5 | 30 | | 50 | | 70 | | 70 | | 60 | | 45 | |
| | 15.625 | 15 | | 30 | | 40 | | 20 | | 30 | | 30 | |
| | | | | | | | | | | | | | |
| Compound 2.19 + 2 g/ ha compound A22 | 250 | 70 | 11.25 | 80 | 8.5 | 90 | -2.875 | 80 | -0.4 | 80 | -0.8 | 85 | 2.5 |
| | 62.5 | 65 | 22.75 | 75 | 22.5 | 80 | -5.75 | 70 | -0.6 | 80 | 18.4 | 80 | 18.5 |
| | 15.625 | 50 | 20.125 | 60 | 26.5 | 80 | 8.5 | 60 | 38.4 | 80 | 47.2 | 80 | 29 |
| | | | | | | | | | | | | | |
| \|Compound 2.18 alone | 250 | 70 | | 50 | | 70 | | 80 | | 85 | | 70 | |
| | 62.5 | 60 | | 30 | | 65 | | 60 | | 60 | | 40 | |
| | 15.625 | 15 | | 1 | | 10 | | 5 | | 10 | | 2 | |
| | | | | | | | | | | | | | |
| Compound 2.18 + 2 g/ ha compound A22 | 250 | 70 | -5.25 | 75 | 22.5 | 85 | -0.75 | 80 | -0.4 | 85 | -0.6 | 85 | 6 |
| | 62.5 | 50 | -17 | 60 | 26.5 | 80 | 3.375 | 70 | 9.2 | 80 | 18.4 | 80 | 22 |
| | 15.625 | 50 | 20.125 | 40 | 34.05 | 70 | 12.75 | 50 | 43.1 | 65 | 51.4 | 60 | 28.6 |
| | | | | 15 | | | | | | | | | |
| \|Compound 2.9 alone | 250 | 20 | | | | 70 | | 70 | | 65 | | 70 | |
| | 62.5 | 20 | | 10 | | 50 | | 55 | | 50 | | 40 | |
| | 15.625 | 10 | | 2 | | 30 | | 15 | | 20 | | 20 | |
| | | | | | | | | | | | | | |
| Compound 2.9 + 2 g/ ha compound A22 | 250 | 70 | 36 | 50 | 30.75 | 85 | -0.75 | 75 | 4.4 | 80 | 13.6 | 85 | 6 |
| | 62.5 | 70 | 36 | 40 | 25.5 | 80 | 3.75 | 70 | 14.1 | 80 | 28 | 75 | 17 |
| | 15.625 | 50 | 24.25 | 40 | 33.1 | 75 | 8.25 | 50 | 33.3 | 60 | 36.8 | 70 | 26 |
| \|Compound 2.3 alone | 250 | 55 | | 45 | | 65 | | 70 | | 60 | | 40 | |
| | 62.5 | 40 | | 35 | | 55 | | 50 | | 40 | | 30 | |
| | 15.625 | 10 | | 2 | | 2 | | 20 | | 5 | | 5 | |
| | | | | | | | | | | | | | |
| Compound 2.3 + 2 g/ha compound A22 | 250 | 50 | -12.875 | 60 | 12.25 | 85 | 1.625 | 70 | -0.6 | 60 | -1.6 | 65 | 7 |
| | 62.5 | 50 | -0.5 | 55 | 16.75 | 75 | -3.625 | 60 | 9 | 60 | 17.6 | 60 | 9 |
| | 15.625 | 40 | 14.25 | 50 | 43.1 | 75 | 21.55 | 50 | 28.4 | 60 | 51.2 | 60 | 26.5 |
| | | | | | | | | | | | | | |
| \|Compound 2.8 alone | 250 | 50 | | 60 | | 80 | | 85 | | 60 | | 65 | |
| | 62.5 | 45 | | 20 | | 50 | | 55 | | 65 | | 40 | |
| | 15.625 | 30 | | 5 | | 40 | | 20 | | 5 | | 10 | |
| | | | | | | | | | | | | | |
| Compound 2.8 + 2 g/ha compound A22 | 250 | 70 | 11.25 | 65 | 3 | 90 | -0.5 | 90 | 4.7 | 65 | 3.4 | 70 | -5.5 |
| | 62.5 | 60 | 5.375 | 60 | 36 | 75 | -1.25 | 80 | 24.1 | 60 | -6.4 | 70 | 12 |
| | 15.625 | 50 | 7.75 | 50 | 40.25 | 70 | -1.5 | 60 | 38.4 | 50 | 41.2 | 55 | 18 |
| | | | | | | | | | | | | | |
| \|Compound 2.17 alone | 250 | 50 | | 50 | | 55 | | 50 | | 40 | | 40 | |
| | 62.5 | 15 | | 5 | | 10 | | 15 | | 10 | | 5 | |
| | 15.625 | 5 | | 0 | | 0 | | 1 | | 0 | | 0 | |
| | | | | | | | | | | | | | |
| Compound 2.17 + 2 g/ ha compound A22 | 250 | 55 | -3.75 | 60 | 7.5 | 70 | -8.625 | 40 | -11 | 55 | 12.6 | 50 | -8 |
| | 62.5 | 50 | 20.125 | 50 | 40.25 | 70 | 12.75 | 40 | 23.3 | 50 | 36.4 | 50 | 16.5 |
| | 15.625 | 40 | 18.375 | 40 | 35 | 50 | 7.5 | 35 | 32.02 | 35 | 31 | 30 | 0 |
| | | | | | | | | | | | | | |
| \|Compound 2.16 alone | 250 | 50 | | 40 | | 70 | | 70 | | 65 | | 50 | |
| | 62.5 | 40 | | 2 | | 40 | | 30 | | 30 | | 15 | |
| | 15.625 | 5 | | 0 | | 5 | | 10 | | 1 | | 2 | |
| | | | | | | | | | | | | | |
| Compound 2.16 + 2 g/ha compound A22 | 250 | 75 | 16.25 | 70 | 27 | 75 | -10.75 | 70 | -0.6 | 75 | 9.3 | 70 | 5 |
| | 62.5 | 70 | 19.5 | 40 | 33.1 | 60 | -11.5 | 40 | 8.6 | 50 | 18.6 | 60 | 19.5 |
| | 15.625 | 40 | 18.375 | 40 | 35 | 60 | 5.125 | 20 | 8.2 | 40 | 37.02 | 50 | 18.6 |
| | | | | | | | | | | | | | |
| \|Compound 2.28 alone | 250 | 50 | | 70 | | 70 | | 75 | | 80 | | 70 | |
| | 62.5 | 40 | | 50 | | 35 | | 50 | | 50 | | 40 | |
| | 15.625 | 15 | | 10 | | 1 | | 5 | | 2 | | 2 | |
| | | | | | | | | | | | | | |
| Compound 2.28 + 2 g/ ha compound A22 | 250 | 75 | 16.25 | 80 | 8.5 | 85 | -0.75 | 80 | 4.5 | 75 | -5.4 | 80 | 1 |
| | 62.5 | 75 | 24.5 | 70 | 17.5 | 80 | 10.875 | 70 | 19 | 75 | 24 | 80 | 22 |
| | 15.625 | 70 | 40.125 | 60 | 45.5 | 75 | 22.025 | 50 | 43.1 | 65 | 61.04 | 60 | 28.6 |
| | | | | | | | | | | | | | |
| \|Compound 2.25 alone | 250 | 40 | | 10 | | 55 | | 60 | | 40 | | 30 | |
| | 62.5 | 30 | | 2 | | 10 | | 30 | | 5 | | 2 | |
| | 15.625 | 10 | | 0 | | 2 | | 15 | | 1 | | 1 | |
| | | | | | | | | | | | | | |
| Compound 2.25 + 2 g/ ha compound A22 | 250 | 60 | 9.5 | 60 | 45.5 | 75 | -3.625 | 80 | 19.2 | 60 | 18.8 | 70 | 19 |
| | 62.5 | 40 | -2.25 | 30 | 23.1 | 70 | 12.75 | 50 | 18.6 | 30 | 23.1 | 50 | 18.6 |
| | 15.625 | 30 | 4.25 | 15 | 10 | 60 | 6.55 | 30 | 13.3 | 25 | 22.02 | 40 | 9.3 |
| | | | | | | | | | | | | | |
| \|Compound 2.20 alone | 250 | 55 | | 80 | | 85 | | 95 | | 80 | | 90 | |
| | 62.5 | 50 | | 60 | | 45 | | 50 | | 65 | | 60 | |
| | 15.625 | 10 | | 20 | | 15 | | 30 | | 25 | | 20 | |
| | | | | | | | | | | | | | |
| Compound 2.20 + 2 g/ ha compound A22 | 250 | 80 | 17.125 | 85 | 4 | 90 | -2.875 | 90 | -5.1 | 85 | 4.6 | 85 | -8 |
| | 62.5 | 70 | 11.25 | 80 | 18 | 85 | 11.125 | 70 | 19 | 80 | 14.3 | 80 | 8 |
| | 15.625 | 65 | 39.25 | 60 | 36 | 80 | 20.375 | 70 | 38.6 | 80 | 53.5 | 80 | 36 |
| | | | | | | | | | | | | | |
| \|Compound 6.1 alone | 250 | 20 | | 30 | | 80 | | 75 | | 70 | | 50 | |
| | 62.5 | 15 | | 10 | | 50 | | 40 | | 20 | | 5 | |
| | 15.625 | 5 | | 0 | | 2 | | 10 | | 0 | | 0 | |
| | | | | | | | | | | | | | |
| Compound 6.1 + 2 g/ha compound A22 | 250 | 60 | 26 | 70 | 36.5 | 97 | 6.5 | 80 | 4.5 | 70 | -0.6 | 75 | 10 |
| | 62.5 | 50 | 20.125 | 50 | 35.5 | 75 | -1.25 | 30 | -11.2 | 60 | 38.4 | 75 | 41.5 |
| | 15.625 | 45 | 23.375 | 40 | 35 | 65 | 11.55 | 20 | 8.2 | 30 | 28 | 50 | 20 |

**TABLE 19. (continued) Pre-emergence weed control of a range of weeds . % control scores following sprays with a variety of HST inhibitors alone and in mixture with A22.**

| Compound | Rate (g/ha) | SCORES 14DAT PRE_EMERGENCE TREATMENT | | | | | | | | | | | |
|---|---|---|---|---|---|---|---|---|---|---|---|---|---|
| | | SIDSP | | DIGSA | | PANMI | | BRAPL | | SETFA | | ECHCG | |
| | | Obs | Colby (O-E) | Obs | Colby (O-E) | Obs | Colby (O-E) | Obs | Colby (O-E) | Obs | Colby (O-E) Obs | Obs | Colby (O-E) |
| Compound A22 alone | 2 | 2 | | 2 | | 0 | | 0 | | 5 | | 5 | |
| | 2 | 0 | | 0 | | 0 | | 0 | | 0 | | 0 | |
| | | | | | | | | | | | | | |
| Compound 2.15 alone | 250 | 0 | | 0 | | 0 | | 0 | | 0 | | 0 | |
| | 62.5 | 0 | | 0 | | 0 | | 0 | | 0 | | 0 | |
| | 15.625 | 0 | | 0 | | 0 | | 0 | | 0 | | 0 | |
| | | | | | | | | | | | | | |
| Compound 2.15 + 2 g/ ha compound A22 | | | | | | | | | | | | | |
| | 250 | 2 | 1 | 0 | -1 | 0 | 0 | 0 | 0 | 0 | -2.5 | 30 | 27.5 |
| | 62.5 | 2 | 1 | 0 | -1 | 0 | 0 | 0 | 0 | 0 | -2.5 | 20 | 17.5 |
| | 15.625 | 0 | -1 | 0 | -1 | 0 | 0 | 0 | 0 | 0 | -2.5 | 0 | -2.5 |
| | | | | | | | | | | | | | |
| Compound 3.5 alone | 250 | 2 | | 0 | | 0 | | 0 | | 0 | | 0 | |
| | 62.5 | 0 | | 0 | | 0 | | 0 | | 0 | | 0 | |
| | 15.625 | 0 | | 0 | | 0 | | 0 | | 0 | | 0 | |
| | | | | | | | | | | | | | |
| Compound 3.5 + 2 g/ ha compound A22 | 250 | 5 | 2.02 | 0 | -1 | 0 | 0 | 0 | 0 | 0 | -2.5 | 2 | 0.5 |
| | 62.5 | 1 | 0 | 0 | -1 | 0 | 0 | 0 | 0 | 0 | -2.5 | 2 | -0.5 |
| | 15.625 | 0 | -1 | 0 | -1 | 0 | 0 | 0 | 0 | 0 | -2.5 | 0 | 2.5 |
| | | | | | | | | | | | | | |
| Compound 2.21 alone | 250 | 1 | | 0 | | 0 | | 5 | | 2 | | 5 | |
| | 62.5 | 0 | | 0 | | 0 | | 2 | | 0 | | 0 | |
| | 15.625 | 0 | | 0 | | 0 | | 0 | | 0 | | 0 | |
| | | | | | | | | | | | | | |
| Compound 2.21 + 2 g/ ha compound A22 | | | | | | | | | | | | | |
| | 250 | 10 | 8.01 | 85 | 84 | 15 | 15 | 10 | 5 | 10 | 5.55 | 30 | 22.625 |
| | 62.5 | 0 | -1 | 0 | -1 | 0 | 0 | 0 | -2 | 1 | -1.5 | 0 | -2.5 |
| | 15.625 | 0 | -1 | 0 | -1 | 0 | 0 | 0 | 0 | 0 | -2.5 | 0 | 2.5 |
| | | | | | | | | | | | | | |
| \|Compound 2.14 alone | 250 | 5 | | 5 | | 2 | | 20 | | 20 | | 50 | |
| | 625 | 0 | | 0 | | 0 | | 2 | | 2 | | 5 | |
| | 15.625 | 0 | | 0 | | 0 | | 0 | | 0 | | 0 | |
| | | | | | | | | | | | | | |
| Compound 2.14 + 2 g/ ha compound A22 | | | | | | | | | | | | | |
| | 250 | 50 | 44.05 | 30 | 24.05 | 50 | 48 | 30 | 10 | 40 | 18 | 97 | 45.75 |
| | 62.5 | 0 | -1 | 0 | -1 | 0 | 0 | 10 | 8 | 20 | 15.55 | 50 | 52.625 |
| | 15.625 | 0 | -1 | 0 | -1 | 0 | 0 | 0 | 0 | 0 | -2.5 | 20 | 17.5 |
| | | | | | | | | | | | | | |
| \|Compound 2.19 alone | 250 | 10 | | 20 | | 60 | | 40 | | 60 | | 90 | |
| | 62.5 | 2 | | 0 | | 0 | | 10 | | 5 | | 50 | |
| | 15.625 | 0 | | 0 | | 0 | | 0 | | 0 | | 0 | |
| | | | | | | | | | | | | | |
| Compound 2.19 +2 g/ ha compound A22 | | | | | | | | | | | | | |
| | 250 | 65 | 54.1 | 80 | 59.2 | 65 | 5 | 70 | 30 | 70 | 9 | 80 | -10.25 |
| | 62.5 | 65 | 62.02 | 30 | 29 | NC | NC | 20 | 10 | 5 | -2.375 | 50 | 8.75 |
| | 15.625 | 30 | 29 | 2 | 1 | 2 | 2 | 0 | 0 | 2 | -0.5 | 10 | 7.5 |
| | | | | | | | | | | | | | |
| Compound 2.18 alone | 250 | 30 | | 0 | | 50 | | 40 | | 25 | | 20 | |
| | 62.5 | 30 | | 0 | | 0 | | 10 | | 20 | | 20 | |
| | 15.625 | 0 | | 0 | | 0 | | 0 | | 0 | | 0 | |
| | | | | | | | | | | | | | |
| Compound 2.18 + 2g/ ha compound A22 | | | | | | | | | | | | | |
| | 250 | 60 | 29.3 | 30 | 29 | 65 | 15 | 60 | 20 | 60 | 33.125 | 80 | 58 |
| | 62.5 | 20 | 10.7 | 10 | 9 | 0 | 0 | 5 | -5 | 2 | -20 | 2 | -20 |
| | 15.625 | 0 | -1 | 0 | -1 | 0 | 0 | 0 | 0 | 1 | -1.5 | 0 | -2.5 |
| | | | | | | | | | | | | | |
| Compound 2.9 alone | 250 | 40 | | 65 | | 60 | | 50 | | 30 | | 60 | |
| | 62.5 | 0 | | 0 | | 0 | | 2 | | 0 | | 0 | |
| | 15.625 | 0 | | 0 | | 0 | | 0 | | 0 | | 0 | |
| | | | | | | | | | | | | | |
| Compound 2.9 + 2 g/ ha compound A22 | 250 | 40 | -0.6 | 50 | -15.35 | 35 | -25 | 50 | 0 | 60 | 28.25 | 90 | 29 |
| | 62.5 | 2 | 1 | 1 | 0 | 0 | 0 | 2 | 0 | 20 | 17.5 | 75 | 72.5 |
| | 15.625 | 1 | 0 | 0 | -1 | 0 | 0 | 0 | 0 | 0 | -2.5 | 60 | 57.5 |
| \|Compound 2.3 alone | 250 | 20 | | 40 | | 50 | | 40 | | 30 | | 50 | |
| | 62.5 | 0 | | 0 | | 0 | | 2 | | 5 | | 10 | |
| | 15.625 | 0 | | 0 | | 0 | | 0 | | 0 | | 0 | |
| | | | | | | | | | | | | | |
| Compound 2.3 2 g/ ha compound A22 | 250 | 5 | -15.8 | 10 | -30.6 | 20 | -30 | 20 | -20 | 40 | 8.25 | 80 | 28.75 |
| | 62.5 | 0 | -1 | 5 | 4 | 0 | 0 | 10 | 8 | 30 | 22.625 | 70 | 57.75 |
| | 15.625 | NC | NC | 0 | -1 | 0 | 0 | 0 | 0 | 20 | 17.5 | 30 | 27.5 |
| | | | | | | | | | | | | | |
| \|Compound 2.8 alone | 250 | 2 | | 30 | | 60 | | 15 | | 10 | | 50 | |
| | 62.5 | 0 | | 0 | | 0 | | 1 | | 0 | | 2 | |
| | 15.625 | 0 | | 0 | | 0 | | 0 | | 0 | | 0 | |
| | | | | | | | | | | | | | |
| Compound 2.8 + 2 g/ha compound A22 | 250 | 2 | -0.98 | 2 | -28.7 | 5 | -55 | 20 | 5 | 30 | 17.75 | 60 | 8.75 |
| | 62.5 | 2 | 1 | 1 | 0 | 0 | 0 | 20 | 19 | 30 | 27.5 | 55 | 50.55 |
| | 15.625 | 0 | -1 | 0 | -1 | 0 | 0 | 0 | 0 | 10 | 7.5 | 40 | 37.5 |
| | | | | | | | | | | | | | |
| \|Compound 2.17 alone | 250 | 0 | | 0 | | 0 | | 2 | | 10 | | 10 | |
| | 62.5 | 0 | | 0 | | 0 | | 0 | | 0 | | 0 | |
| | 15.625 | 0 | | 0 | | 0 | | 0 | | 0 | | 0 | |
| | | | | | | | | | | | | | |
| Compound 2.17 + 2 g/ha compound A22 | | | | | | | | | | | | | |
| | 250 | 2 | 1 | 1 | 0 | 0 | 0 | 10 | 8 | 40 | 27.75 | 60 | 47.75 |
| | 62.5 | 0 | -1 | 0 | -1 | 0 | 0 | 0 | 0 | 2 | -0.5 | 2 | -0.5 |
| | 15.625 | 0 | -1 | 0 | -1 | 0 | 0 | 0 | 0 | 1 | -1.5 | 1 | -1.5 |
| | | | | | | | | | | | | | |
| \|Compound 2.16 alone | 250 | 0 | | 0 | | 40 | | 50 | | 45 | | 60 | |
| | 62.5 | 0 | | 0 | | 0 | | 5 | | 10 | | 15 | |
| | 15.625 | 0 | | 0 | | 0 | | 0 | | 0 | | 0 | |
| | | | | | | | | | | | | | |
| Compound 2.16 + 2 g/ ha compound A22 | | | | | | | | | | | | | |
| | 250 | 2 | 1 | 10 | 9 | 40 | 0 | 35 | -15 | 30 | -16.375 | 50 | -11 |
| | 62.5 | 5 | 4 | 15 | 14 | 0 | 0 | 2 | -3 | 10 | -2.25 | 20 | 2.875 |
| | 15.625 | 0 | -1 | 0 | -1 | 1 | 1 | 0 | 0 | 5 | 2.5 | 25 | 22.5 |
| | | | | | | | | | | | | | |
| \|Compound 2.28 alone | 250 | 10 | | 30 | | 60 | | 50 | | 60 | | 80 | |
| | 62.5 | 0 | | 0 | | 0 | | 2 | | 25 | | 50 | |
| | 15.625 | 0 | | 0 | | 0 | | 0 | | 0 | | 0 | |
| | | | | | | | | | | | | | |
| Compound 2.28 + 2 g/ha compound A22 | | | | | | | | | | | | | |
| | 250 | 20 | 9.1 | 30 | -0.7 | 55 | -5 | 60 | 10 | 70 | 9 | 97 | 16.5 |
| | 62.5 | 1 | 0 | 15 | 14 | 5 | 5 | 10 | 8 | 15 | -11.875 | 20 | -31.25 |
| | 15.625 | 0 | -1 | 0 | -1 | 0 | 0 | 2 | 2 | 10 | 7.5 | 20 | 17.5 |
| | | | | | | | | | | | | | |
| \|Compound 2.25 alone | 250 | 2 | | 5 | | 10 | | 20 | | 15 | | 30 | |
| | 62.5 | 1 | | 0 | | 0 | | 2 | | 1 | | 0 | |
| | 15.625 | 0 | | 0 | | 0 | | 0 | | 0 | | 0 | |
| | | | | | | | | | | | | | |
| Compound 2.25 + 2 g/ha compound A22 | | | | | | | | | | | | | |
| | 250 | 10 | 7.02 | 0 | -5.95 | 10 | 0 | 40 | 20 | 15 | -2.125 | 30 | -1.75 |
| | 62.5 | 0 | -1.99 | 0 | -1 | 0 | 0 | 5 | 3 | 15 | 11.525 | 40 | 37.5 |
| | 15.625 | 0 | -1 | 0 | -1 | 0 | 0 | 0 | 0 | 0 | -2.5 | 0 | -2.5 |
| | | | | | | | | | | | | | |
| \|Compound 2.20 alone | 250 | 5 | | 20 | | 30 | | 60 | | 65 | | 90 | |
| | 62.5 | 0 | | 0 | | 2 | | 10 | | 20 | | 15 | |
| | 15.625 | 0 | | 0 | | 0 | | 0 | | 0 | | 1 | |
| | | | | | | | | | | | | | |
| Compound 220 + 2 g/ ha compound A22 | | | | | | | | | | | | | |
| | 250 | 5 | -0.95 | 10 | -10.8 | 50 | 20 | 60 | 0 | 45 | -20.875 | 85 | -5.25 |
| | 62.5 | 5 | 4 | 2 | 1 | 0 | -2 | 20 | 10 | 50 | 28 | 85 | 67.875 |
| | 15.625 | 0 | -1 | 10 | 9 | 0 | 0 | 2 | 2 | 10 | 7.5 | 20 | 16.525 |
| | | | | | | | | | | | | | |
| \|Compound 6.1 alone | 250 | 0 | | 2 | | 40 | | 10 | | 20 | | 60 | |
| | 62.5 | 0 | | 0 | | 0 | | 0 | | 2 | | 1 | |
| | 15.625 | 0 | | 0 | | 0 | | 0 | | 0 | | 0 | |
| | | | | | | | | | | | | | |
| Compound 6.1 + 2 g/ha compound A22 | 250 | 80 | 79 | 80 | 77.02 | 80 | 40 | 97 | 87 | 98 | 76 | 85 | 24 |
| | 62.5 | 10 | 9 | 40 | 39 | 20 | 20 | 10 | 10 | 15 | 10.55 | 30 | 26.525 |
| | 15.625 | 0 | -1 | 0 | -1 | 0 | 0 | 20 | 20 | 30 | 27.5 | 50 | 47.5 |

The data provided in the above tables indicate that, in many cases, addition of even low (sub-lethal) levels of mesotrione improves weed control by HST inhibiting herbicides both pre and post emergence.

## Claims

1. A method of selectively controlling weeds at a locus comprising crop plants and weeds, wherein the method comprises application to the locus of a weed controlling amount of a pesticide composition comprising an homogentisate solanesyl transferase (HST) inhibiting herbicide selected from the group consisting of a compound of formula (IIa) 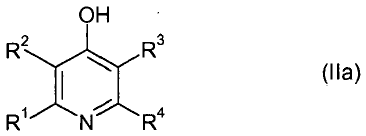 wherein
R¹, R², R³ and R⁴ are independently hydrogen or halogen; provided that at least three of R¹, R², R³ and R⁴ are halogen; or salts thereof;
a compound of formula (IIb) 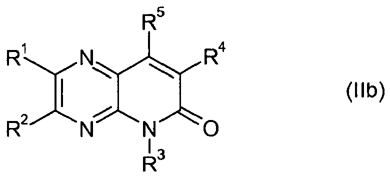 wherein
R¹ and R² are independently hydrogen, C₁-C₄alkyl, C₁-C₄haloalkyl, halo, cyano, hydroxy, C₁-C₄alkoxy, C₁-C₄alkylthio, aryl or aryl substituted by one to five R⁶, which may be the same or different, or heteroaryl or heteroaryl substituted by one to five R⁶, which may be the same or different;
R³ is hydrogen, C₁-C₁₀alkyl, C₂-C₁₀alkenyl, C₂-C₁₀alkynyl, C₃-C₁₀cycloalkyl, C₃-C₁₀ocyclo-alkyl-C₁-C₆alkyl-, C₁-C₁₀alkoxy-C₁-C₆alkyl-, C₁-C₁₀cyanoalkyl-, C₁-C₁₀alkoxycarbonyl-C₁-C₆alkyl-, *N*-C₁-C₃alkyl-aminocarbonyl-C₁-C₆alkyl-, *N,N*-di-(C₁-C₃alkyl)-aminocarbonyl-C₁-C₆alkyl-, aryl-C₁-C₆alkyl- or aryl-C₁-C₆alkyl- wherein the aryl moiety is substituted by one to three R⁷, which may be the same or different, or heterocyclyl-C₁-C₆alkyl- or heterocyclyl-C₁-C₆alkyl- wherein the heterocyclyl moiety is substituted by one to three R⁷, which may be the same or different;
R⁴ is aryl or aryl substituted by one to five R⁸, which may be the same or different, or heteroaryl or heteroaryl substituted by one to four R⁸, which may be the same or different;
R⁵ is hydroxy, R⁹-oxy-, R¹⁰-carbonyloxy-, tri-R¹¹-silyloxy- or R¹²-sulfonyloxy-, each R⁶, R⁷ and R⁸ is independently halo, cyano, nitro, C₁-C₁₀alkyl, C₁-C₄haloalkyl, C₂-C₁₀alkenyl, C₂-C₁₀alkynyl, hydroxy, C₁-C₁₀alkoxy, C₁-C₄haloalkoxy, C₁-C₁₀alkoxy-C₁-C₄alkyl-, C₃-C₇cycloalkyl, C₃-C₇cycloalkoxy, C₃-C₇cycloalkyl-C₁-C₄alkyl-, C₃-C₇cycloalkyl-C₁-C₄alkoxy-, C₁-C₆alkylcarbonyl-, formyl, C₁-C₄alkoxycarbonyl-, C₁-C₄alkylcarbonyloxy-, C₁-C₁₀alkylthio-, C₁-C₄haloalkylthio-, C₁-C₁₀alkylsulfinyl-, C₁-C₄haloalkylsulfinyl-, C₁-C₁₀alkylsulfonyl-, C₁-C₄haloalkylsulfonyl-, amino, C₁-C₁₀alkylamino-, di-C₁-C₁₀alkylamino-, C₁-C₁₀alkylcarbonylamino-, aryl or aryl substituted by one to three R¹³, which may be the same or different, heteroaryl or heteroaryl substituted by one to three R¹³, which may be the same or different, aryl-C₁-C₄alkyl- or aryl-C₁-C₄alkyl- wherein the aryl moiety is substituted by one to three R¹³, which may be the same or different, heteroaryl-C₁-C₄alkyl- or heteroaryl-C₁-C₄alkyl-wherein the heteroaryl moiety is substituted by one to three R¹³, which may be the same or different, aryloxy- or aryloxy- substituted by one to three R¹³, which may be the same or different, heteroaryloxy- or heteroaryloxy- substituted by one to three R¹³, which may be the same or different, arylthio- or arylthio- substituted by one to three R¹³, which may be the same or different, or heteroarylthio- or heteroarylthio- substituted by one to three R¹³, which may be the same or different;
R⁹ is C₁-C₁₀alkyl, C₂-C₁₀alkenyl, C₂-C₁₀alkynyl or aryl-C₁-C₄alkyl- or aryl-C₁-C₄alkyl- wherein the aryl moiety is substituted by one to five substituents independently selected from halo, cyano, nitro, C₁-C₆alkyl, C₁-C₆haloalkyl or C₁-C₆alkoxy;
R¹⁰ is C₁-C₁₀alkyl, C₃-C₁₀cycloalkyl, C₃-C₁₀cycloalkyl-C₁-C₁₀alkyl-, C₁-C₁₀haloalkyl, C₂-C₁₀alkenyl, C₂-C₁₀alkynyl, C₁-C₄alkoxy-C₁-C₁₀alkyl-, C₁-C₄alkylthio-C₁-C₄alkyl-, C₁-C₁₀alkoxy, C₂-C₁₀alkenyloxy, C₂-C₁₀alkynyloxy, C₁-C₁₀alkylthio-, *N-*C₁-C₄alkyl-amino-, *N,N-*di-(C₁-C₄alkyl)-amino-, aryl or aryl substituted by one to three R¹⁴, which may be the same or different, heteroaryl or heteroaryl substituted by one to three R¹⁴, which may be the same or different, aryl-C₁-C₄alkyl- or aryl-C₁-C₄alkyl- wherein the aryl moiety is substituted by one to three R¹⁴, which may be the same or different, heteroaryl-C₁-C₄alkyl- or heteroaryl-C₁-C₄alkyl-wherein the heteroaryl moiety is substituted by one to three R¹⁴, which may be the same or different, aryloxy- or aryloxy- substituted by one to three R¹⁴, which may be the same or different, heteroaryloxy- or heteroaryloxy- substituted by one to three R¹⁴, which may be the same or different, arylthio- or arylthio- substituted by one to three R¹⁴, which may be the same or different, or heteroarylthio- or heteroarylthio- substituted by one to three R¹⁴, which may be the same or different;
each R¹¹ is independently C₁-C₁₀alkyl or phenyl or phenyl substituted by one to five substituents independently selected from halo, cyano, nitro, C₁-C₆alkyl, C₁-C₆haloalkyl or C₁-C₆alkoxy;
R¹² is C₁-C₁₀alkyl or phenyl or phenyl substituted by one to five substituents independently selected from halo, cyano, nitro, C₁-C₆alkyl, C₁-C₆haloalkyl or C₁-C₆alkoxy;
each R¹³ is independently halo, cyano, nitro, C₁-C₆alkyl, C₁-C₆haloalkyl or C₁-C₆alkoxy; and each R¹⁴ is independently halo, cyano, nitro, C₁-C₁₀alkyl, C₁-C₄haloalkyl, C₁-C₁₀alkoxy, C₁-C₄alkoxycarbonyl-, C₁-C₄haloalkoxy, C₁-C₁₀alkylthio-, C₁-C₄haloalkylthio-, C₁-C₁₀alkylsulfinyl-, C₁-C₄haloalkylsulfinyl-, C₁-C₁₀alkylsulfonyl-, C₁-C₄haloalkylsulfonyl-, aryl or aryl substituted by one to five substituents independently selected from halo, cyano, nitro, C₁-C₆alkyl, C₁-C₆haloalkyl or C₁-C₆alkoxy, or heteroaryl or heteroaryl substituted by one to four substituents independently selected from halo, cyano, nitro, C₁-C₆alkyl, C₁-C₆haloalkyl or C₁-C₆alkoxy; or salts or *N-*oxides thereof;
a compound of formula (IIc) 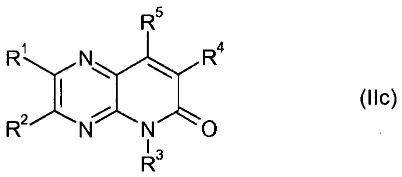 wherein
R¹ and R² are independently hydrogen, C₁-C₄alkyl, C₁-C₄haloalkyl, halo, cyano, hydroxy, C₁-C₄alkoxy, C₁-C₄alkylthio, aryl or aryl substituted by one to five R⁶, which may be the same or different, or heteroaryl or heteroaryl substituted by one to five R⁶, which may be the same or different;
R³ is C₁-C₄haloalkyl, C₂-C₄haloalkenyl or C₂-C₄haloalkynyl;
R⁴ is aryl or aryl substituted by one to five R⁸, which may be the same or different, or heteroaryl or heteroaryl substituted by one to four R⁸, which may be the same or different;
R⁵ is hydroxy or a group which can be metabolised to the hydroxy group;
each R⁶ and R⁸ is independently halo, cyano, nitro, C₁-C₁₀alkyl, C₁-C₄haloalkyl, C₂-C₁₀alkenyl, C₂-C₁₀alkynyl, hydroxy, C₁-C₁₀alkoxy, C₁-C₄haloalkoxy, C₁-C₁₀alkoxy-C₁-C₄alkyl-, C₃-C₇cycloalkyl, C₃-C₇cycloalkoxy, C₃-C₇cycloalkyl-C₁-C₄alkyl-, C₃-C₇cycloalkyl-C₁-C₄alkoxy-, C₁-C₆alkylcarbonyl-, formyl, C₁-C₄alkoxycarbonyl-, C₁-C₄alkylcarbonyloxy-, C₁-C₁₀alkylthio-, C₁-C₄haloalkylthio-, C₁-C₁₀alkylsulfinyl-, C₁-C₄haloalkylsulfinyl-, C₁-C₁₀alkylsulfonyl-, C₁-C₄haloalkylsulfonyl-, amino, C₁-C₁₀alkylamino-, di-C₁-C₁₀alkylamino-, C₁-C₁₀alkylcarbonylamino-, aryl or aryl substituted by one to three R¹³, which may be the same or different, heteroaryl or heteroaryl substituted by one to three R¹³, which may be the same or different, aryl-C₁-C₄alkyl- or aryl-C₁-C₄alkyl- wherein the aryl moiety is substituted by one to three R¹³, which may be the same or different, heteroaryl-C₁-C₄alkyl- or heteroaryl-C₁-C₄alkyl-wherein the heteroaryl moiety is substituted by one to three R¹³, which may be the same or different, aryloxy- or aryloxy- substituted by one to three R¹³, which may be the same or different, heteroaryloxy- or heteroaryloxy- substituted by one to three R¹³, which may be the same or different, arylthio- or arylthio- substituted by one to three R¹³, which may be the same or different, or heteroarylthio- or heteroarylthio- substituted by one to three R¹³, which may be the same or different; and
each R¹³ is independently halo, cyano, nitro, C₁-C₆alkyl, C₁-C₆haloalkyl or C₁-C₆alkoxy; or a salt or *N*-oxide thereof;
a compound of formula (IId) 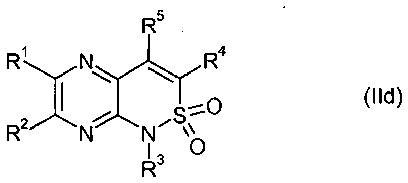 wherein
R¹ and R² are independently hydrogen, C₁-C₄alkyl, C₁-C₄haloalkyl, halo, cyano, hydroxy, C₁-C₄alkoxy, C₁-C₄alkylthio, aryl or aryl substituted by one to five R⁶, which may be the same or different, or heteroaryl or heteroaryl substituted by one to five R⁶, which may be the same or different;
R³ is hydrogen, C₁-C₁₀alkyl, C₁-C₄haloalkyl, C₂-C₁₀alkenyl, C₂-C₄haloalkenyl, C₂-C₁₀alkynyl, C₂-C₄haloalkynyl, C₃-C₁₀cycloalkyl, C₃-C₁₀cycloalkyl-C₁-C₆alkyl-, C₁-C₁₀alkoxy-C₁-C₆alkyl-, C₁-C₁₀cyanoalkyl-, C₁-C₁₀alkoxycarbonyl-C₁-C₆alkyl-, *N*-C₁-C₃alkyl-aminocarbonyl-C₁-C₆alkyl-, *N,N-*di-(C₁-C₃alkyl)-aminocarbonyl-C₁-C₆alkyl-, aryl-C₁-C₆alkyl- or aryl-C₁-C₆alkyl-wherein the aryl moiety is substituted by one to three R⁷, which may be the same or different, or heterocyclyl-C₁-C₆alkyl- or hetetocyclyl-C₁-C₆alkyl- wherein the heterocyclyl moiety is substituted by one to three R⁷, which may be the same or different;
R⁴ is aryl or aryl substituted by one to five R⁸, which may be the same or different, or heteroaryl or heteroaryl substituted by one to four R⁸, which may be the same or different;
R⁵ is hydroxy or a group which can be metabolised to the hydroxy group;
each R⁶, R⁷ and R⁸ is independently halo, cyano, nitro, C₁-C₁₀alkyl, C₁-C₄haloalkyl, C₂-C₁₀alkenyl, C₂-C₁₀alkynyl, hydroxy, C₁-C₁₀alkoxy, C₁-C₄haloalkoxy, C₁-C₁₀alkoxy-C₁-C₄alkyl-, C₃-C₇cycloalkyl, C₃-C₇cycloalkoxy, C₃-C₇cycloalkyl-C₁-C₄alkyl-, C₃-C₇cycloalkyl-C₁-C₄alkoxy-, C₁-C₆alkylcarbonyl-, formyl, C₁-C₄alkoxycarbonyl-, C₁-C₄alkylcarbonyloxy-, C₁-C₁₀alkylthio-, C₁-C₄haloalkylthio-, C₁-C₁₀alkylsulfinyl-, C₁-C₄haloalkylsulfinyl-, C₁-C₁₀alkylsulfonyl-, C₁-C₄haloalkylsulfonyl-, amino, C₁-C₁₀alkylamino-, di-C₁-C₁₀alkylamino-, C₁-C₁₀alkylcarbonylamino-, aryl or aryl substituted by one to three R¹³, which may be the same or different, heteroaryl or heteroaryl substituted by one to three R¹³, which may be the same or different, aryl-C₁-C₄alkyl- or aryl-C₁-C₄alkyl- wherein the aryl moiety is substituted by one to three R¹³, which may be the same or different, heteroaryl-C₁-C₄alkyl- or heteroaryl-C₁-C₄alkyl-wherein the heteroaryl moiety is substituted by one to three R¹³, which may be the same or different, aryloxy- or aryloxy- substituted by one to three R¹³, which may be the same or different, heteroaryloxy- or heteroaryloxy- substituted by one to three R¹³, which may be the same or different, arylthio- or arylthio- substituted by one to three R¹³, which may be the same or different, or heteroarylthio- or heteroarylthio- substituted by one to three R¹³, which may be the same or different; and
each R¹³ is independently halo, cyano, nitro, C₁-C₆alkyl, C₁-C₆haloalkyl or C₁-C₆alkoxy; or a salt or *N*-oxide thereof;
a compound of formula (IIe) 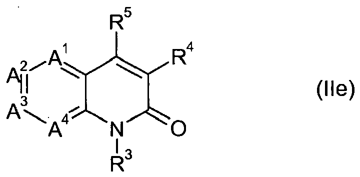 wherein
A¹, A², A³ and A⁴ are independently C-R¹ or N, provided at least one of A¹, A², A³ and A⁴ is N, and provided that if A¹ and A⁴ are both N, A² and A³ are not both C-R¹;
each R¹ is independently hydrogen, C₁-C₄alkyl, C₁-C₄haloalkyl, halo, cyano, hydroxy, C₁-C₄alkoxy, C₁-C₄alkylthio, aryl or aryl substituted by one to five R⁶, which may be the same or different, or heteroaryl or heteroaryl substituted by one to five R⁶, which may be the same or different;
R³ is hydrogen, C₁-C₁₀alkyl, C₁-C₄haloalkyl, C₂-C₁₀alkenyl, C₂-C₄haloalkenyl, C₂-C₁₀alkynyl, C₂-C₄haloalkynyl, C₃-C₁₀cycloalkyl, C₃-C₁₀cycloalkyl-C₁-C₆alkyl-, C₁-C₁₀alkoxy-C₁-C₆alkyl-, C₁-C₁₀cyanoalkyl-, C₁-C₁₀alkoxycarbonyl-C₁-C₆alkyl-, *N*-C₁-C₃alkyl-aminocarbonyl-C₁-C₆alkyl-, *N,N*-di-(C₁-C₃alkyl)-aminocarbonyl-C₁-C₆alkyl-, aryl-C₁-C₆alkyl- or aryl-C₁-C₆alkyl-wherein the aryl moiety is substituted by one to three R⁷, which may be the same or different, or heterocyclyl-C₁-C₆alkyl- or heterocyclyl-C₁-C₆alkyl- wherein the heterocyclyl moiety is substituted by one to three R⁷, which may be the same or different;
R⁴ is aryl or aryl substituted by one to five R⁸, which may be the same or different, or heteroaryl or heteroaryl substituted by one to four R⁸, which may be the same or different;
R⁵ is hydroxy or a group which can be metabolised to a hydroxy group;
each R⁶, R⁷ and R⁸ is independently halo, cyano, nitro, C₁-C₁₀alkyl, C₁-C₄haloalkyl, C₂-C₁₀alkenyl, C₂-C₁₀alkynyl, hydroxy, C₁-C₁₀alkoxy, C₁-C₄haloalkoxy, C₁-C₁₀alkoxy-C₁-C₄alkyl-, C₃-C₇cycloalkyl, C₃-C₇cycloalkoxy, C₃-C₇cycloalkyl-C₁-C₄alkyl-, C₃-C₇cycloalkyl-C₁-C₄alkoxy-, C₁-C₆alkylcarbonyl-, formyl, C₁-C₄alkoxycarbonyl-, C₁-C₄alkylcarbonyloxy-, C₁-C₁₀alkylthio-, C₁-C₄haloalkylthio-, C₁-C₁₀alkylsulfinyl-, C₁-C₄haloalkylsulfinyl-, C₁-C₁₀alkylsulfonyl-, C₁-C₄haloalkylsulfonyl-, amino, C₁-C₁₀alkylamino-, di-C₁-C₁₀alkylamino-, C₁-C₁₀alkylcarbonylamino-, aryl or aryl substituted by one to three R¹³, which may be the same or different, heteroaryl or heteroaryl substituted by one to three R¹³, which may be the same or different, aryl-C₁-C₄alkyl- or aryl-C₁-C₄alkyl- wherein the aryl moiety is substituted by one to three R¹³, which may be the same or different, heteroaryl-C₁-C₄alkyl- or heteroaryl-C₁-C₄alkyl-wherein the heteroaryl moiety is substituted by one to three R¹³, which may be the same or different, aryloxy- or aryloxy- substituted by one to three R¹³, which may be the same or different, heteroaryloxy- or heteroaryloxy- substituted by one to three R¹³, which may be the same or different, arylthio- or arylthio- substituted by one to three R¹³, which may be the same or different, or heteroarylthio- or heteroarylthio- substituted by one to three R¹³, which may be the same or different; and
each R¹³ is independently halo, cyano, nitro, C₁-C₆alkyl, C₁-C₆haloalkyl or C₁-C₆alkoxy; or a salt or *N*-oxide thereof; and
and a compound of formula (IIf) 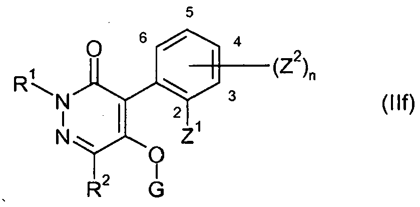 wherein
R¹ is C₁-C₆ alkyl or C₁-C₆alkyloxy-C₁-C₆alkyl;
R² is hydrogen or C₁-C₆alkyl;
G is a hydrogen, -(C=L)R³, -(SO₂)R⁴, or -(P=L)R⁵R⁶, wherein
L is oxygen or sulfur;
R³ is C₁-C₆alkyl, C₃-C₈cycloalkyl, C₂-C₆alkenyl, C₂-C₆alkynyl, C₆-C₁₀aryl, C₆-C₁₀aryl-C₁-C₆alkyl-, C₁-C₆alkyloxy, C₃-C₈cycloalkyloxy, C₂-C₆alkenyloxy, C₂-C₆alkynyloxy, C₆-C₁₀aryloxy, C₆-C₁₀aryl-C₁-C₆alkyloxy-, amino, C₁-C₆alkylamino, C₂-C₆alkenylamino, C₆-C₁₀arylamino, di(C₁-C₆alkyl)amino, di(C₂-C₆alkenyl)amino, (C₁-C₆alkyl)(C₆-C₁₀aryl)amino or a three- to eight-membered nitrogen containing heterocyclic ring,
R⁴ is C₁-C₆alkyl, C₆-C₁₀aryl, C₁-C₆alkylamino group or di(C₁-C₆ alkyl)amino; and
R⁵ and R⁶ may be same or different and are independently C₁-C₆alkyl, C₃-₈cycloalkyl, C₂-C₆alkenyl, C₆-C₁₀aryl, C₁-C₆alkyloxy, C₃-C₈cycloalkyloxy, C₆-C₁₀aryloxy, C₆-C₁₀aryl-C₁-C₆alkyloxy, C₁-C₆alkylthio, C₁-C₆alkylamino or di(C₁-C₆alkyl)amino,
whereby any R³, R⁴, R⁵ and R⁶ group may be substituted with halogen, C₃-Cscycloalkyl, C₆-C₁₀aryl, C₆-C₁₀aryl-C₁-C₆alkyl-, C₃-C₈cycloalkyloxy, C₆-C₁₀aryloxy, C₆-C₁₀aryl-C₁-C₆alkyloxy-, C₆-C₁₀arylamino, (C₁-C₆ alkyl)(C₆-C₁₀aryl)amino and a three- to eight-membered nitrogen containing heterocyclic ring which may be substituted with at least one C₁-C₆alkyl;
Z¹ is C₁-C₆alkyl;
Z² is C₁-C₆alkyl;
n is 0, 1, 2, 3 or 4;
and each of Z² may be same or different when n represents an integer of 2 or more, and a sum of the number of carbon atoms in the group represented by Z¹ and that in the group represented by Z² is equal to 2 or more,
and/or a hydroxyphenyl pyruvate dioxygenase (HPPD) inhibiting herbicide, wherein the crop plants comprise at least one heterologous polynucleotide which comprises a region which encodes an HST and which provides over expression of said HST in said crop plants.

2. A method according to 1, wherein the crop plants contain an additional heterologous polynucleotide which comprises a region which encodes a hydroxyphenyl pyruvate dioxygenase (HPPD).

3. A method according to any one of the previous claims, wherein the pesticide composition comprises an homogentisate solanesyl transferase-inhibiting herbicide and a hydroxyphenyl pyruvate dioxygenase (HPPD)-inhibiting herbicide.

4. A method according to any one of the preceding claims, wherein the hydroxyphenyl pyruvate dioxygenase-inhibiting herbicide is selected from the group consisting of mesotrione, sulcotrione, isoxaflutole, tembotrione, topramezone, benzofenap, pyrazolate, pyrazoxyfen, pyrasulfotole, ketospiradox or the free acid thereof, 4-hydroxy-3-[[2-(2-methoxyethoxy)methyl]-6-(trifluoromethyl)-3-pyridinyl]carbonyl]-bicyclo[3.2.1]oct-3-en-2-one, [2-chloro-3-(2-methoxyethoxy)-4-(methylsulfonyl)phenyl](1-ethyl-5-hydroxy-1*H-*pyrazol-4-yl)-methanone, α-(cyclopropylcarbonyl)-2-(methylsulfonyl)-β-oxo-4-(trifluoromethyl)-benzenepropanenitrile, and (2,3-dihydro-3,3,4-trimethyl-1,1-dioxidobenzo[b]thien-5-yl)(5-hydroxy-1-methyl-1*H*-pyrazol-4-yl)-methanone.

5. A method according to any one of the preceding claims, wherein the homogentisate solanesyltransferase enzyme is derived from *Arabidopsis thaliana, Glycine max, Oryza sativa* or *Chlamydomonas reinhardtii.*

6. A method according to any one of the preceding claims, wherein the homogentisate solanesyltransferase enzyme is selected from the group consisting of
(a) the HST enzymes depicted as SEQ ID NO. 1 to 10; and
(b) an HST which comprises one or more of the following polypeptide motifs:-
W-(R/K)-F-L-R-P-H-T-I-R-G-T; and/or
N-G-(Y/F)-I-V-G-I-N-Q-I-(Y/F)-D; and/or
I-A-I-T-K-D-L-P; and/or
Y-(R/Q)-(F/W)-(I/V)-W-N-L-F-Y.

7. A method according to any one of the previous claims, wherein the crop plant comprises a further recombinant polynucleotide encoding a further herbicide tolerance enzyme.

8. A method according to claim 7, wherein the further herbicide tolerance enzyme is selected from the group consisting of, 5-enolpyruvylshikimate-3-phosphate synthase (EPSPS), Glyphosate acetyl transferase (GAT), Cytochrome P450, phosphinothricin acetyltransferase (PAT), Acetolactate synthase (ALS), Protoporphyrinogen oxidase (PPGO), Phytoene desaturase (PD), dicamba degrading enzymes and aryloxy herbicide degrading enzymes.

9. A method according to any one of the previous claims, wherein the pesticide composition comprises one or more additional herbicides.

10. A method according to claim 9, wherein the one or more additional herbicides is selected from the group consisting of glyphosate (including agrochemically acceptable salts thereof); glufosinate (including agrochemically acceptable salts thereof); chloroacetanilides e.g alachlor, acetochlor, metolachlor, S-metholachlor; photo system II inhibitors e.g triazines such as ametryn, atrazine, cyanazine and terbuthylazine, triazinones such as hexazinone and metribuzin, and ureas such as chlorotoluron, diuron, isoproturon, linuron and terbuthiuron; ALS-inhibitors e.g sulfonyl ureas such as amidosulfuron, chlorsulfuron, flupyrsulfuron, halosulfuron, nicosulfuron, primisulfuron, prosulfuron, rimsulfuron, triasulfuron, trifloxysulfuron and tritosulfuron; diphenyl ethers e.g aciflurofen and fomesafen.

11. A method according to claim 10, wherein the one or more additional herbicides is glyphosate and/or a PS-II inhibiting herbicide.

12. A method according to any one of the preceding claims, further comprising application to the locus of an insecticide and/or a fungicide.

13. A method of providing a transgenic plant which is tolerant to homogentisate solanesyl transferase (HST)-inhibiting and/or hydroxyphenyl pyruvate dioxygenase (HPPD)-inhibiting herbicides which comprises transformation of plant material with a recombinant polynucleotide which comprises a region which encodes an HST and, optionally, a region encoding a HPPD, selection of the transformed plant material using an HST-inhibitor selected from the group consisting of Formula (IIa), (IIb), (IIc), (IId), (IIe) and (IIf) as defined in claim 1 and/or an HPPD inhibitor, and regeneration of that material into a morphological normal fertile plant.

14. A method according to 13, wherein the recombinant polynucleotide further comprises a region encoding the target for a non-homogentisate solanesyl transferase inhibitor herbicide and/or a region encoding a protein capable of conferring on plant material transformed with the region resistance to insects, fungi and/or nematodes.

## Patentansprüche

1. Verfahren zur selektiven Bekämpfung von Unkräutern an einem Standort, an dem sich Nutzpflanzen und Unkräuter befinden, bei dem man auf den Standort eine unkrautbekämpfende Menge einer Pestizidzusammensetzung, die ein Homogentisatsolanesyltransferase (HST) inhibierendes Herbizid aus der Gruppe bestehend aus einer Verbindung der Formel (IIa) 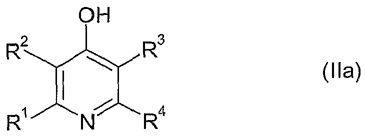 worin
R¹, R², R³ und R⁴ unabhängig voneinander für Wasserstoff oder Halogen stehen; mit der Maßgabe, dass mindestens drei der Variablen R¹, R², R³ und R⁴ für Halogen stehen; oder Salze davon;
einer Verbindung der Formel (IIb) 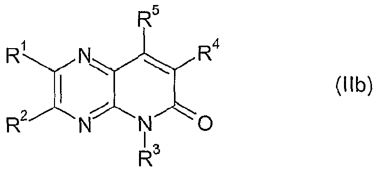 worin
R¹ und R² unabhängig voneinander für Wasserstoff, C₁-C₄-Alkyl, C₁-C₄-Halogenalkyl, Halogen, Cyano, Hydroxy, C₁-C₄-Alkoxy, C₁-C₄-Alkylthio, Aryl oder Aryl, das durch eins bis fünf R⁶, die gleich oder verschieden sein können, substituiert ist, oder Heteroaryl oder Heteroaryl, das durch eins bis fünf R⁶, die gleich oder verschieden sein können, substituiert ist, stehen;
R³ für Wasserstoff, C₁-C₁₀-Alkyl, C₂-C₁₀-Alkenyl, C₂-C₁₀-Alkinyl, C₃-C₁₀-Cycloalkyl, C₃-C₁₀-Cycloalkyl-C₁-C₆-alkyl-, C₁-C₁₀-Alkoxy-C₁-C₆-alkyl-, C₁-C₁₀-Cyano-alkyl-, C₁-C₁₀-Alkoxycarbonyl-C₁-C₆-alkyl-, *N*-C₁-C₃-Alkyl-aminocarbonyl-C₁-C₆-alkyl-, *N,N-*Di-(C₁-C₃-alkyl)-aminocarbonyl-C₁-C₆-alkyl-, Aryl-C₁-C₆-alkyl-oder Aryl-C₁-C₆-alkyl-, worin die Arylgruppierung durch eins bis drei R⁷, die gleich oder verschieden sein können, substituiert ist, oder Heterocyclyl-C₁-C₆-alkyl- oder Heterocyclyl-C₁-C₆-alkyl-, worin die Heterocyclylgruppierung durch eins bis drei R⁷, die gleich oder verschieden sein können, substituiert ist, steht;
R⁴ für Aryl oder Aryl, das durch eins bis fünf R⁸, die gleich oder verschieden sein können, substituiert ist, oder Heteroaryl oder Heteroaryl, das durch eins bis vier R⁸, die gleich oder verschieden sein können, substituiert ist, steht; R⁵ für Hydroxy, R⁹-Oxy-, R¹⁰-Carbonyloxy-, Tri-R¹¹-silyloxy- oder R¹²-Sulfonyloxy- steht, R⁶, R⁷ und R⁸ jeweils unabhängig voneinander für Halogen, Cyano, Nitro, C₁-C₁₀-Alkyl, C₁-C₄-Halogenalkyl, C₂-C₁₀-Alkenyl, C₂-C₁₀-Alkinyl, Hydroxy, C₁-C₁₀-Alkoxy, C₁-C₄-Halogenalkoxy, C₁-C₁₀-Alkoxy-C₁-C₄-alkyl-, C₃-C₇-Cycloalkyl, C₃-C₇-Cycloalkoxy, C₃-C₇-Cycloalkyl-C₁-C₄-alkyl-, C₃-C₇-Cycloalkyl-C₁-C₄-alkoxy-, C₁-C₆-Alkylcarbonyl-, Formyl, C₁-C₄-Alkoxycarbonyl-, C₁-C₄-Alkylcarbonyloxy-, C₁-C₁₀-Alkylthio-, C₁-C₄-Halogenalkylthio-, C₁-C₁₀-Alkylsulfinyl-, C₁-C₄-Halogenalkylsulfinyl-, C₁-C₁₀-Alkylsulfonyl-, C₁-C₄-Halogenalkylsulfonyl-, Amino, C₁-C₁₀-Alkylamino-, Di-C₁-C₁₀-alkylamino-, C₁-C₁₀-Alkylcarbonylamino-, Aryl oder Aryl, das durch eins bis drei R¹³, die gleich oder verschieden sein können, substituiert ist, Heteroaryl oder Heteroaryl, das durch eins bis drei R¹³, die gleich oder verschieden sein können, substituiert ist, Aryl-C₁-C₄-alkyl- oder Aryl-C₁-C₄-alkyl-, worin die Arylgruppierung durch eins bis drei R¹³, die gleich oder verschieden sein können, substituiert ist, Heteroaryl-C₁-C₄-alkyl-oder Heteroaryl-C₁-C₄-alkyl-, worin die Heteroarylgruppierung durch eins bis drei R¹³, die gleich oder verschieden sein können, substituiert ist, Aryloxy- oder Aryloxy-, das durch eins bis drei R¹³, die gleich oder verschieden sein können, substituiert ist, Heteroaryloxy- oder Heteroaryloxy-, das durch eins bis drei R¹³, die gleich oder verschieden sein können, substituiert ist, Arylthio- oder Arylthio-, das durch eins bis drei R¹³, die gleich oder verschieden sein können, substituiert ist, oder Heteroarylthio- oder Heteroarylthio-, das durch eins bis drei R¹³, die gleich oder verschieden sein können, substituiert ist, stehen;
R⁹ für C₁-C₁₀-Alkyl, C₂-C₁₀-Alkenyl, C₂-C₁₀-Alkinyl oder Aryl-C₁-C₄-alkyl- oder Aryl-C₁-C₄-alkyl-, worin die Arylgruppierung durch eins bis fünf Substituenten, die unabhängig voneinander aus Halogen, Cyano, Nitro, C₁-C₆-Alkyl, C₁-C₆-Halogenalkyl oder C₁-C₆-Alkoxy ausgewählt sind, substituiert ist, steht;
R¹⁰ für C₁-C₁₀-Alkyl, C₃-C₁₀-Cycloalkyl, C₃-C₁₀-Cycloalkyl-C₁-C₁₀-alkyl-, C₁-C₁₀-Halogenalkyl, C₂-C₁₀-Alkenyl, C₂-C₁₀-Alkinyl, C₁-C₄-Alkoxy-C₁-C₁₀-alkyl-, C₁-C₄-Alkylthio-C₁-C₄-alkyl-, C₁-C₁₀-Alkoxy, C₂-C₁₀-Alkenyloxy, C₂-C₁₀-Alkinyloxy, C₁-C₁₀-Alkyl-thio-, *N*-C₁-C₄-Alkyl-amino-, *N,N*-Di-(C₁-C₄-alkyl)-amino-, Aryl oder Aryl, das durch eins bis drei R¹⁴, die gleich oder verschieden sein können, substituiert ist, Heteroaryl oder Heteroaryl, das durch eins bis drei R¹⁴, die gleich oder verschieden sein können, substituiert ist, Aryl-C₁-C₄-alkyl- oder Aryl-C₁-C₄-alkyl-, worin die Arylgruppierung durch eins bis drei R¹⁴, die gleich oder verschieden sein können, substituiert ist, Heteroaryl-C₁-C₄-alkyl- oder Heteroaryl-C₁-C₄-alkyl-, worin die Heteroarylgruppierung durch eins bis drei R¹⁴, die gleich oder verschieden sein können, substituiert ist, Aryloxy- oder Aryloxy-, das durch eins bis drei R¹⁴, die gleich oder verschieden sein können, substituiert ist, Heteroaryloxy- oder Heteroaryloxy-, das durch eins bis drei R¹⁴, die gleich oder verschieden sein können, substituiert ist, Arylthio- oder Arylthio-, das durch eins bis drei R¹⁴, die gleich oder verschieden sein können, substituiert ist, oder Heteroarylthio- oder Heteroarylthio-, das durch eins bis drei R¹⁴, die gleich oder verschieden sein können, substituiert ist, steht;
R¹¹ jeweils unabhängig voneinander für C₁-C₁₀-Alkyl, Phenyl oder Phenyl, das durch eins bis fünf Substituenten, die unabhängig voneinander aus Halogen, Cyano, Nitro, C₁-C₆-Alkyl, C₁-C₆-Halogenalkyl oder C₁-C₆-Alkoxy ausgewählt sind, substituiert ist, steht;
R¹² für C₁-C₁₀-Alkyl, Phenyl oder Phenyl, das durch eins bis fünf Substituenten, die unabhängig voneinander aus Halogen, Cyano, Nitro, C₁-C₆-Alkyl, C₁-C₆-Halogenalkyl oder C₁-C₆-Alkoxy ausgewählt sind, substituiert ist, steht;
R¹³ jeweils unabhängig voneinander für Halogen, Cyano, Nitro, C₁-C₆-Alkyl, C₁-C₆-Halogenalkyl oder C₁-C₆-Alkoxy steht; und
R¹⁴ jeweils unabhängig voneinander für Halogen, Cyano, Nitro, C₁-C₁₀-Alkyl, C₁-C₄-Halogenalkyl, C₁-C₁₀-Alkoxy, C₁-C₄-Alkoxycarbonyl, C₁-C₄-Halogen-alkoxy, C₁-C₁₀-Alkylthio-, C₁-C₄-Halogenalkylthio-, C₁-C₁₀-Alkylsulfinyl-, C₁-C₄-Halogenalkylsulfinyl-, C₁-C₁₀-Alkylsulfonyl-, C₁-C₄-Halogenalkylsulfonyl-, Aryl oder Aryl, das durch eins bis fünf Substituenten, die unabhängig voneinander aus Halogen, Cyano, Nitro, C₁-C₆-Alkyl, C₁-C₆-Halogenalkyl oder C₁-C₆-Alkoxy ausgewählt sind, substituiert ist, Heteroaryl oder Heteroaryl, das durch eins bis vier Substituenten, die unabhängig voneinander aus Halogen, Cyano, Nitro, C₁-C₆-Alkyl, C₁-C₆-Halogenalkyl oder C₁-C₆-Alkoxy ausgewählt sind, substituiert ist, steht; oder Salzen oder N-Oxiden davon;
einer Verbindung der Formel (IIc) 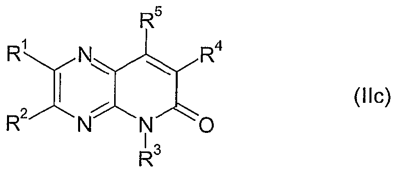 worin
R¹ und R² unabhängig voneinander für Wasserstoff, C₁-C₄-Alkyl, C₁-C₄-Halogenalkyl, Halogen, Cyano, Hydroxy, C₁-C₄-Alkoxy, C₁-C₄-Alkylthio, Aryl oder Aryl, das durch eins bis fünf R⁶, die gleich oder verschieden sein können, substituiert ist, oder Heteroaryl oder Heteroaryl, das durch eins bis fünf R⁶, die gleich oder verschieden sein können, substituiert ist, stehen;
R³ für C₁-C₄-Halogenalkyl, C₂-C₄-Halogenalkenyl oder C₂-C₄-Halogenalkinyl steht;
R⁴ für Aryl oder Aryl, das durch eins bis fünf R⁸, die gleich oder verschieden sein können, substituiert ist, oder Heteroaryl oder Heteroaryl, das durch eins bis vier R⁸, die gleich oder verschieden sein können, substituiert ist, steht; R⁵ für Hydroxy oder eine Gruppe, die zur Hydroxygruppe metabolisiert werden kann, steht,
R⁶ und R⁸ jeweils unabhängig voneinander für Halogen, Cyano, Nitro, C₁-C₁₀-Alkyl, C₁-C₄-Halogenalkyl, C₂-C₁₀-Alkenyl, C₂-C₁₀-Alkinyl, Hydroxy, C₁-C₁₀-Alkoxy, C₁-C₄-Halogenalkoxy, C₁-C₁₀-Alkoxy-C₁-C₄-alkyl-, C₃-C₇-Cycloalkyl, C₃-C₇-Cycloalkoxy, C₃-C₇-Cycloalkyl-C₁-C₄-alkyl-, C₃-C₇-Cycloalkyl-C₁-C₄-alkoxy-, C₁-C₆-Alkylcarbonyl-, Formyl, C₁-C₄-Alkoxycarbonyl-, C₁-C₄-Alkylcarbonyloxy-, C₁-C₁₀-Alkylthio-, C₁-C₄-Halogenalkylthio-, C₁-C₁₀-Alkylsulfinyl-, C₁-C₄-Halogenalkylsulfinyl-, C₁-C₁₀-Alkylsulfonyl-, C₁-C₄-Halogenalkylsulfonyl-, Amino, C₁-C₁₀-Alkylamino-, Di-C₁-C₁₀-alkylamino-, C₁-C₁₀-Alkylcarbonylamino-, Aryl oder Aryl, das durch eins bis drei R¹³, die gleich oder verschieden sein können, substituiert ist, Heteroaryl oder Heteroaryl, das durch eins bis drei R¹³, die gleich oder verschieden sein können, substituiert ist, Aryl-C₁-C₄-alkyl- oder Aryl-C₁-C₄-alkyl-, worin die Arylgruppierung durch eins bis drei R¹³, die gleich oder verschieden sein können, substituiert ist, Heteroaryl-C₁-C₄-alkyl-oder Heteroaryl-C₁-C₄-alkyl-, worin die Heteroarylgruppierung durch eins bis drei R¹³, die gleich oder verschieden sein können, substituiert ist, Aryloxy- oder Aryloxy-, das durch eins bis drei R¹³, die gleich oder verschieden sein können, substituiert ist, Heteroaryloxy- oder Heteroaryloxy-, das durch eins bis drei R¹³, die gleich oder verschieden sein können, substituiert ist, Arylthio- oder Arylthio-, das durch eins bis drei R¹³, die gleich oder verschieden sein können, substituiert ist, oder Heteroarylthio- oder Heteroarylthio-, das durch eins bis drei R¹³, die gleich oder verschieden sein können, substituiert ist, stehen; und
R¹³ jeweils unabhängig voneinander für Halogen, Cyano, Nitro, C₁-C₆-Alkyl, C₁-C₆-Halogenalkyl oder C₁-C₆-Alkoxy steht; oder einem Salz oder N-Oxid davon;
einer Verbindung der Formel (IId) 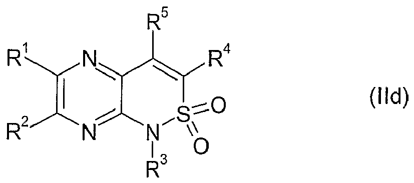 worin
R¹ und R² unabhängig voneinander für Wasserstoff, C₁-C₄-Alkyl, C₁-C₄-Halogenalkyl, Halogen, Cyano, Hydroxy, C₁-C₄-Alkoxy, C₁-C₄-Alkylthio, Aryl oder Aryl, das durch eins bis fünf R⁶, die gleich oder verschieden sein können, substituiert ist, oder Heteroaryl oder Heteroaryl, das durch eins bis fünf R⁶, die gleich oder verschieden sein können, substituiert ist, stehen;
R³ für Wasserstoff, C₁-C₁₀-Alkyl, C₁-C₄-Halogen-alkyl, C₂-C₁₀-Alkenyl, C₂-C₄-Halogenalkenyl, C₂-C₁₀-Alkinyl, C₂-C₄-Halogenalkinyl, C₃-C₁₀-Cycloalkyl, C₃-C₁₀-Cycloalkyl-C₁-C₆-alkyl-, C₁-C₁₀-Alkoxy-C₁-C₆-alkyl-, C₁-C₁₀-Cyanoalkyl-, C₁-C₁₀-Alkoxycarbonyl-C₁-C₆-alkyl-, *N*-C₁-C₃-Alkyl-aminocarbonyl-C₁-C₆-alkyl-, *N,N*-Di-(C₁-C₃-alkyl)-aminocarbonyl-C₁-C₆-alkyl-, Aryl-C₁-C₆-alkyl- oder Aryl-C₁-C₆-alkyl-, worin die Arylgruppierung durch eins bis drei R⁷, die gleich oder verschieden sein können, substituiert ist, oder Heterocyclyl-C₁-C₆-alkyl- oder Heterocyclyl-C₁-C₆-alkyl-, worin die Heterocyclylgruppierung durch eins bis drei R⁷, die gleich oder verschieden sein können, substituiert ist, steht;
R⁴ für Aryl oder Aryl, das durch eins bis fünf R⁸, die gleich oder verschieden sein können, substituiert ist, oder Heteroaryl oder Heteroaryl, das durch eins bis vier R⁸, die gleich oder verschieden sein können, substituiert ist, steht; R⁵ für Hydroxy oder eine Gruppe, die zur Hydroxygruppe metabolisiert werden kann, steht, R⁶, R⁷ und R⁸ jeweils unabhängig voneinander für Halogen, Cyano, Nitro, C₁-C₁₀-Alkyl, C₁-C₄-Halogenalkyl, C₂-C₁₀-Alkenyl, C₂-C₁₀-Alkinyl, Hydroxy, C₁-C₁₀-Alkoxy, C₁-C₄-Halogenalkoxy, C₁-C₁₀-Alkoxy-C₁-C₄-alkyl-, C₃-C₇-Cycloalkyl, C₃-C₇-Cycloalkoxy, C₃-C₇-Cycloalkyl-C₁-C₄-alkyl-, C₃-C₇-Cycloalkyl-C₁-C₄-alkoxy-, C₁-C₆-Alkylcarbonyl-, Formyl, C₁-C₄-Alkoxycarbonyl-, C₁-C₄-Alkylcarbonyloxy-, C₁-C₁₀-Alkylthio-, C₁-C₄-Halogenalkylthio-, C₁-C₁₀-Alkylsulfinyl-, C₁-C₄-Halogenalkylsulfinyl-, C₁-C₁₀-Alkylsulfonyl-, C₁-C₄-Halogenalkylsulfonyl-, Amino, C₁-C₁₀-Alkylamino-, Di-C₁-C₁₀-alkylamino-, C₁-C₁₀-Alkylcarbonylamino-, Aryl oder Aryl, das durch eins bis drei R¹³, die gleich oder verschieden sein können, substituiert ist, Heteroaryl oder Heteroaryl, das durch eins bis drei R¹³, die gleich oder verschieden sein können, substituiert ist, Aryl-C₁-C₄-alkyl- oder Aryl-C₁-C₄-alkyl-, worin die Arylgruppierung durch eins bis drei R¹³, die gleich oder verschieden sein können, substituiert ist, Heteroaryl-C₁-C₄-alkyl-oder Heteroaryl-C₁-C₄-alkyl-, worin die Heteroarylgruppierung durch eins bis drei R¹³, die gleich oder verschieden sein können, substituiert ist, Aryloxy- oder Aryloxy-, das durch eins bis drei R¹³, die gleich oder verschieden sein können, substituiert ist, Heteroaryloxy- oder Heteroaryloxy-, das durch eins bis drei R¹³, die gleich oder verschieden sein können, substituiert ist, Arylthio- oder Arylthio-, das durch eins bis drei R¹³, die gleich oder verschieden sein können, substituiert ist, oder Heteroarylthio- oder Heteroarylthio-, das durch eins bis drei R¹³, die gleich oder verschieden sein können, substituiert ist, stehen; und
R¹³ jeweils unabhängig voneinander für Halogen, Cyano, Nitro, C₁-C₆-Alkyl, C₁-C₆-Halogenalkyl oder C₁-C₆-Alkoxy steht; oder einem Salz oder N-Oxid davon;
einer Verbindung der Formel (IIe) 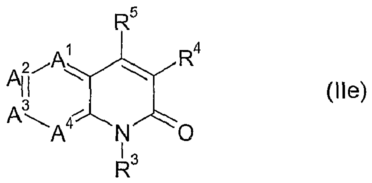 worin
A¹, A², A³ und A⁴ unabhängig für C-R¹ oder N stehen, mit der Maßgabe, dass mindestens eine der Variablen A¹, A², A³ und A⁴ für N steht, und mit der Maßgabe, dass dann, wenn A¹ und A⁴ beide für N stehen, A² und A³ nicht beide für C-R¹ stehen;
R¹ jeweils unabhängig voneinander für Wasserstoff, C₁-C₄-Alkyl, C₁-C₄-Halogenalkyl, Halogen, Cyano, Hydroxy, C₁-C₄-Alkoxy, C₁-C₄-Alkylthio, Aryl oder Aryl, das durch eins bis fünf R⁶, die gleich oder verschieden sein können, substituiert ist, oder Heteroaryl oder Heteroaryl, das durch eins bis fünf R⁶, die gleich oder verschieden sein können, substituiert ist, steht;
R³ für Wasserstoff, C₁-C₁₀-Alkyl, C₁-C₄-Halogen-alkyl, C₂-C₁₀-Alkenyl, C₂-C₄-Halogenalkenyl, C₂-C₁₀-Alkinyl, C₂-C₄-Halogenalkinyl, C₃-C₁₀-Cycloalkyl, C₃-C₁₀-Cycloalkyl-C₁-C₆-alkyl-, C₁-C₁₀-Alkoxy-C₁-C₆-alkyl-, C₁-C₁₀-Cyanoalkyl-, C₁-C₁₀-Alkoxycarbonyl-C₁-C₆-alkyl-, *N*-C₁-C₃-Alkyl-aminocarbonyl-C₁-C₆-alkyl-, *N,N*-Di-(C₁-C₃-alkyl)-aminocarbonyl-C₁-C₆-alkyl-, Aryl-C₁-C₆-alkyl- oder Aryl-C₁-C₆-alkyl-, worin die Arylgruppierung durch eins bis drei R⁷, die gleich oder verschieden sein können, substituiert ist, oder Heterocyclyl-C₁-C₆-alkyl- oder Heterocyclyl-C₁-C₆-alkyl-, worin die Heterocyclylgruppierung durch eins bis drei R⁷, die gleich oder verschieden sein können, substituiert ist, steht;
R⁴ für Aryl oder Aryl, das durch eins bis fünf R⁸, die gleich oder verschieden sein können, substituiert ist, oder Heteroaryl oder Heteroaryl, das durch eins bis vier R⁸, die gleich oder verschieden sein können, substituiert ist, steht; R⁵ für Hydroxy oder eine Gruppe, die zur Hydroxygruppe metabolisiert werden kann, steht;
R⁶, R⁷ und R⁸ jeweils unabhängig voneinander für Halogen, Cyano, Nitro, C₁-C₁₀-Alkyl, C₁-C₄-Halogenalkyl, C₂-C₁₀-Alkenyl, C₂-C₁₀-Alkinyl, Hydroxy, C₁-C₁₀-Alkoxy, C₁-C₄-Halogenalkoxy, C₁-C₁₀-Alkoxy-C₁-C₄-alkyl-, C₃-C₇-Cycloalkyl, C₃-C₇-Cycloalkoxy, C₃-C₇-Cycloalkyl-C₁-C₄-alkyl-, C₃-C₇-Cycloalkyl-C₁-C₄-alkoxy-, C₁-C₆-Alkylcarbonyl-, Formyl, C₁-C₄-Alkoxycarbonyl-, C₁-C₄-Alkylcarbonyloxy-, C₁-C₁₀-Alkylthio-, C₁-C₄-Halogenalkylthio-, C₁-C₁₀-Alkylsulfinyl-, C₁-C₄-Halogenalkylsulfinyl-, C₁-C₁₀-Alkylsulfonyl-, C₁-C₄-Halogenalkylsulfonyl-, Amino, C₁-C₁₀-Alkylamino-, Di-C₁-C₁₀-alkylamino-, C₁-C₁₀-Alkylcarbonylamino-, Aryl oder Aryl, das durch eins bis drei R¹³, die gleich oder verschieden sein können, substituiert ist, Heteroaryl oder Heteroaryl, das durch eins bis drei R¹³, die gleich oder verschieden sein können, substituiert ist, Aryl-C₁-C₄-alkyl- oder Aryl-C₁-C₄-alkyl-, worin die Arylgruppierung durch eins bis drei R¹³, die gleich oder verschieden sein können, substituiert ist, Heteroaryl-C₁-C₄-alkyl-oder Heteroaryl-C₁-C₄-alkyl-, worin die Heteroarylgruppierung durch eins bis drei R¹³, die gleich oder verschieden sein können, substituiert ist, Aryloxy- oder Aryloxy-, das durch eins bis drei R¹³, die gleich oder verschieden sein können, substituiert ist, Heteroaryloxy- oder Heteroaryloxy-, das durch eins bis drei R¹³, die gleich oder verschieden sein können, substituiert ist, Arylthio- oder Arylthio-, das durch eins bis drei R¹³, die gleich oder verschieden sein können, substituiert ist, oder Heteroarylthio- oder Heteroarylthio-, das durch eins bis drei R¹³, die gleich oder verschieden sein können, substituiert ist, stehen; und
R¹³ jeweils unabhängig voneinander für Halogen, Cyano, Nitro, C₁-C₆-Alkyl, C₁-C₆-Halogenalkyl oder C₁-C₆-Alkoxy steht; oder einem Salz oder N-Oxid davon; und
einer Verbindung der Formel (IIf) 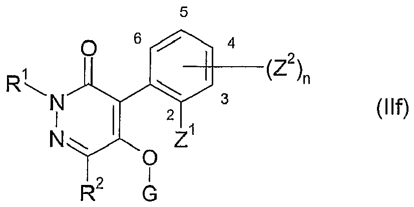 worin
R¹ für C₁-C₆-Alkyl oder C₁-C₆-Alkyloxy-C₁-C₆-alkyl steht;
R² für Wasserstoff oder C₁-C₆-Alkyl steht;
G für Wasserstoff, -(C=L)R³, -(SO₂)R⁴ oder -(P=L)R⁵R⁶ steht, worin
L für Sauerstoff oder Schwefel steht;
R³ für C₁-C₆-Alkyl, C₃-C₈-Cycloalkyl, C₂-C₆-Alkenyl, C₂-C₆-Alkinyl, C₆-C₁₀-Aryl, C₆-C₁₀-Aryl-C₁-C₆-alkyl-, C₁-C₆-Alkyloxy, C₃-C₈-Cycloalkyloxy, C₂-C₆-Alkenyloxy, C₂-C₆-Alkinyloxy, C₆-C₁₀-Aryloxy, C₆-C₁₀-Aryl-C₁-C₆-alkyloxy-, Amino, C₁-C₆-Alkylamino, C₂-C₆-Alkenylamino, C₆-C₁₀-Arylamino, Di(C₁-C₆-alkyl)amino, Di (C₂-C₆-alkenyl) amino, (C₁-C₆-Alkyl) (C₆-C₁₀-aryl)amino oder einen drei- bis achtgliedrigen stickstoffhaltigen heterocyclischen Ring steht,
R⁴ für C₁-C₆-Alkyl, C₆-C₁₀-Aryl, C₁-C₆-Alkylamino oder Di(C₁-C₆-alkyl)amino steht; und
R⁵ und R⁶ gleich oder verschieden sein können und unabhängig voneinander für C₁-C₆-Alkyl, C₃-C₈-Cycloalkyl, C₂-C₆-Alkenyl, C₆-C₁₀-Aryl, C₁-C₆-Alkyloxy, C₃-C₈-Cycloalkyloxy, C₆-C₁₀-Aryloxy, C₆-C₁₀-Aryl-C₁-C₆-alkyloxy-, C₁-C₆-Alkylthio, C₁-C₆-Alkylamino oder Di(C₁-C₆-alkyl)amino stehen,
wobei jede R³-, R⁴-, R⁵- oder R⁶-Gruppe durch Halogen, C₃-C₈-Cycloalkyl, C₆-C₁₀-Aryl, C₆-C₁₀-Aryl-C₁-C₆-alkyl-, C₃-C₈-Cycloalkyloxy, C₆-C₁₀-Aryloxy, C₆-C₁₀-Aryl-C₁-C₆-alkyloxy-, C₆-C₁₀-Arylamino, (C₁-C₆-Alkyl) (C₆-C₁₀-aryl)amino oder einen drei- bis achtgliedrigen stickstoffhaltigen heterocyclischen Ring, der durch mindestens ein C₁-C₆-Alkyl substituiert sein kann, substituiert sein kann;
Z¹ für C₁-C₆-Alkyl steht;
Z² für C₁-C₆-Alkyl steht;
n für 0, 1, 2, 3 oder 4 steht;
und Z² jeweils gleich oder verschieden sein kann, wenn n für eine ganze Zahl von 2 oder mehr steht, und eine Summe der Zahl der Kohlenstoffatome in der durch Z¹ wiedergegebenen Gruppe und derjenigen in der durch Z² wiedergegebenen Gruppe gleich 2 oder mehr ist,
und/oder ein Hydroxyphenylpyruvatdioxygenase (HPPD) inhibierendes Herbizid
umfasst, ausbringt, wobei die Nutzpflanzen mindestens ein heterologes Polynucleotid, das eine Region, die für eine HST codiert, umfasst und Überexpression der HST in den Nutzpflanzen bereitstellt, umfassen.

2. Verfahren nach Anspruch 1, bei dem die Nutzpflanzen ein zusätzliches heterologes Polynucleotid, das eine Region, die für eine Hydroxyphenylpyruvatdioxygenase (HPPD) codiert, umfasst, umfassen.

3. Verfahren nach einem der vorhergehenden Ansprüche, bei dem die Pestizidzusammensetzung ein Homogentisatsolanesyltransferase inhibierendes Herbizid und ein Hydroxyphenylpyruvatdioxygenase (HPPD) inhibierendes Herbizid umfasst.

4. Verfahren nach einem der vorhergehenden Ansprüche, bei dem das Hydroxyphenylpyruvatdioxygenase inhibierende Herbizid aus der Gruppe bestehend aus Mesotrion, Sulcotrion, Isoxaflutol, Tembotrion, Topramezon, Benzofenap, Pyrazolat, Pyrazoxyfen, Pyrasulfotol, Ketospiradox oder die freie Säure davon, 4-Hydroxy-3-[[2-(2-methoxyethoxy)methyl]-6-(trifluormethyl)-3-pyridinyl]carbonyl]bicyclo-[3.2.1]oct-3-en-2-on, [2-Chlor-3-(2-methoxy-ethoxy)-4-(methylsulfonyl)phenyl](1-ethyl-5-hydroxy-1*H*-pyrazol-4-yl)-methanon, α-(Cyclopropyl-carbonyl)-2-(methylsulfonyl)-β-oxo-4-(trifluor-methyl)benzolpropannitril und (2,3-Dihydro-3,3,4-trimethyl-1,1-dioxidobenzo[b]thien-5-yl)(5-hydroxy-1-methyl-1*H*-pyrazol-4-yl)-methanon ausgewählt wird.

5. Verfahren nach einem der vorhergehenden Ansprüche, bei dem das Homogentisatsolanesyltransferase-Enzym aus *Arabidopsis thaliana, Glycine max, Oryza sativa* oder *Chlamydomonas reinhardtii* stammt.

6. Verfahren nach einem der vorhergehenden Ansprüche, bei dem das Homogentisatsolanesyltransferase-Enzym aus der Gruppe bestehend aus
(a) den HST-Enzymen, die als SEQ ID No. 1 bis 10 dargestellt sind; und
(b) einer HST, die ein oder mehrere der folgenden Polypeptidmotive umfasst:
W-(R/K)-F-L-R-P-H-T-I-R-G-T; und/oder
N-G-(Y/F)-I-V-G-I-N-Q-I-(Y/F)-D; und/oder
I-A-I-T-K-D-L-P; und/oder
Y-(R/Q)-(F/W)-(I/V)-W-N-L-F-Y
ausgewählt wird.

7. Verfahren nach einem der vorhergehenden Ansprüche, bei dem die Nutzpflanze ein weiteres rekombinantes Polynucleotid, das für ein weiteres Herbizidtoleranzenzym codiert, umfasst.

8. Verfahren nach Anspruch 7, bei dem das weitere Herbizidtoleranzenzym aus der Gruppe bestehend aus 5-Enolpyruvylshikimat-3-phosphatsynthase (EPSPS), Glyphosatacetyltransferase (GAT), Cytochrom P450, Phosphinothricinacetyltransferase (PAT), Aceto-lactatsynthase (ALS), Protoporphyrinogenoxidase (PPGO), Phytoendesaturase (PD), Dicamba abbauenden Enzymen und Aryloxy-Herbizid abbauenden Enzymen ausgewählt wird.

9. Verfahren nach einem der vorhergehenden Ansprüche, wobei die Pestizidzusammensetzung ein oder mehrere zusätzliche Herbizide umfasst.

10. Verfahren nach Anspruch 9, bei dem man das eine oder die mehreren zusätzlichen Herbizide aus der Gruppe bestehend aus Glyphosat (einschließlich agrochemisch verträglicher Salze davon); Glufosinat (einschließlich agrochemisch verträglicher Salze davon); Chloracetaniliden, z.B. Alachlor, Acetochlor, Metolachlor, S-Metholachlor; Photosystem-II-Inhibitoren, z.B. Triazinen wie Ametryn, Atrazin, Cyanazin und Terbuthylazin, Triazinonen wie Hexazinon und Metribuzin, und Harnstoffen wie Chlorotoluron, Diuron, Isoproturon, Linuron und Terbuthiuron; ALS-Inhibitoren, z.B. Sulfonylharnstoffen wie Amidosulfuron, Chlorsulfuron, Flupyrsulfuron, Halosulfuron, Nicosulfuron, Primisulfuron, Prosulfuron, Rimsulfuron, Triasulfuron, Trifloxysulfuron und Tritosulfuron; Diphenylethern, z.B. Aciflurofen und Fomesafen, auswählt.

11. Verfahren nach Anspruch 10, bei dem es sich bei dem einen oder den mehreren zusätzlichen Herbiziden um Glyphosat und/oder ein PS-II inhibieren des Herbizid handelt.

12. Verfahren nach einem der vorhergehenden Ansprüche, bei dem man ferner ein Insektizid und/oder ein Fungizid auf den Standort ausbringt.

13. Verfahren zur Bereitstellung einer transgenen Pflanze, die gegenüber Homogentisatsolanesyltransferase (HST) inhibierenden Herbiziden und/oder Hydroxyphenylpyruvatdioxygenase (HPPD) inhibierenden Herbiziden tolerant ist, das die Transformation von Pflanzenmaterial mit einem rekombinanten Polynucleotid, das eine Region, die für eine HST codiert, und gegebenenfalls eine Region, die für ein HPPD codiert, umfasst, die Selektion des transformierten Pflanzenmaterials unter Verwendung eines HST-Inhibitors aus der Gruppe bestehend aus Formel (IIa), (IIb), (IIc), (IId), (IIe) und (IIf) gemäß Anspruch 1 und/oder eines HPPD-Inhibitors und die Regeneration des Materials zu einer morphologisch normalen fertilen Pflanze umfasst.

14. Verfahren nach Anspruch 13, bei dem das rekombinante Polynucleotid ferner eine Region, die für das Target eines Nicht-Homogentisatsolanesyltransferase-Inhibitor-Herbizids codiert, und/oder eine Region, die für ein Protein, das transformiertem Pflanzenmaterial Regionsresistenz gegenüber Insekten, Pilzen und/oder Nematoden verleihen kann, codiert, umfasst.

## Revendications

1. Méthode de contrôle de manière sélective d'adventices au niveau d'un lieu comprenant des plantes de culture et des adventices, **caractérisée en ce que** la méthode comprend l'application au lieu d'une quantité de contrôle d'adventices d'une composition pesticide comprenant un herbicide inhibiteur de l'homogentisate solanésyl transférase (HST) choisi dans le groupe constitué par un composé de formule (IIa) 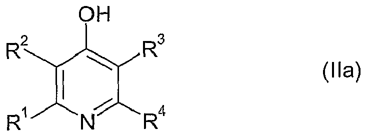 dans laquelle
R¹, R², R³ et R⁴ sont indépendamment hydrogène ou halogène ;
à condition qu'au moins trois parmi R¹, R², R³ et R⁴ soient halogène ;
ou des sels de celui-ci ;
un composé de formule (IIb), 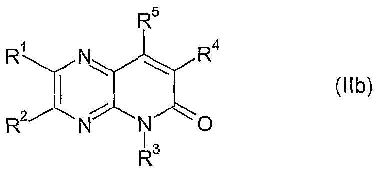 dans laquelle
R¹ et R² sont indépendamment hydrogène, C₁-C₄alkyle, C₁-C₄halogénoalkyle, halogéno, cyano, hydroxy, C₁-C₄alcoxy, C₁-C₄alkylthio, aryle ou aryle substitué par de un à cinq R⁶, pouvant être identiques ou différents, ou hétéroaryle ou hétéroaryle substitué par de un à cinq R⁶, pouvant être identiques ou différents ;
R³ est hydrogène, C₁-C₁₀alkyle, C₂-C₁₀alcényle, C₂-C₁₀-alcynyle, C₃-C₁₀cycloalkyle, C₃-C₁₀cycloalkyl-C₁-C₆alkyl-, C₁-C₁₀alcoxy-C₁-C₆alkyl-, C₁-C₁₀cyanoalkyl-, C₁-C₁₀-alcoxycarbonyl-C₁-C₆alkyl-, N-C₁-C₃alkyl-aminocarbonyl-C₁-C₆alkyl-, N,N-di(C₁-C₃alkyl)-aminocarbonyl-C₁-C₆alkyl-, aryl-C₁-C₆alkyl- ou aryl-C₁-C₆alkyl- où le motif aryle est substitué par de un à trois R⁷, pouvant être identiques ou différents, ou hétérocyclyl-C₁-C₆alkyl- ou hétérocyclyl-C₁-C₆alkyl- où le motif hétérocyclyle est substitué par de un à trois R⁷, pouvant être identiques ou différents ;
R⁴ est aryle ou aryle substitué par de un à cinq R⁸, pouvant être identiques ou différents, ou hétéroaryle ou hétéroaryle substitué par de un à quatre R⁸, pouvant être identiques ou différents ;
R⁵ est hydroxy, R⁹-oxy-, R¹⁰-carbonyloxy-, tri-R¹¹-silyloxy- ou R¹²-sulfonyloxy-,
chaque R⁶, R⁷ et R⁸ est indépendamment halogéno, cyano, nitro, C₁-C₁₀alkyle, C₁-C₄halogénoalkyle, C₂-C₁₀alcényle, C₂-C₁₀alcynyle, hydroxy, C₁-C₁₀alcoxy, C₁-C₄halogéno-alcoxy, C₁-C₁₀alcoxy-C₁-C₄alkyl-, C₃-C₇cycloalkyle, C₃-C₇cycloalcoxy, C₃-C₇cycloalkyl-C₁-C₄alkyl-, C₃-C₇-cycloalkyl-C₁-C₄alcoxy-, C₁-C₆alkylcarbonyl-, formyle, C₁-C₄alcoxycarbonyl-, C₁-C₄alkylcarbonyloxy-, C₁-C₁₀-alkylthio-, C₁-C₄halogénoalkylthio-, C₁-C₁₀alkyl-sulfinyl-, C₁-C₄halogénoalkylsulfinyl-, C₁-C₁₀-alkylsulfonyl-, C₁-C₄halogénoalkylsulfonyl-, amino, C₁-C₁₀alkylamino-, di-C₁-C₁₀alkylamino-, C₁-C₁₀-alkylcarbonylamino-, aryle ou aryle substitué par de un à trois R¹³, pouvant être identiques ou différents, hétéroaryle ou hétéroaryle substitué par de un à trois R¹³, pouvant être identiques ou différents, aryl-C₁-C₄alkyl- ou aryl-C₁-C₄alkyl- où le motif aryle est substitué par de un à trois R¹³, pouvant être identiques ou différents, hétéroaryl-C₁-C₄alkyl- ou hétéroaryl-C₁-C₄alkyl- où le motif hétéroaryle est substitué par de un à trois R¹³, pouvant être identiques ou différents, aryloxy- ou aryloxy- substitué par de un à trois R¹³, pouvant être identiques ou différents, hétéroaryloxy-ou hétéroaryloxy- substitué par de un à trois R¹³, pouvant être identiques ou différents, arylthio- ou arylthio- substitué par de un à trois R¹³, pouvant être identiques ou différents, ou hétéroarylthio- ou hétéroarylthio- substitué par de un à trois R¹³, pouvant être identiques ou différents ;
R⁹ est C₁-C₁₀alkyle, C₂-C₁₀alcényle, C₂-C₁₀alcynyle, ou aryl-C₁-C₄alkyl- ou aryl-C₁-C₄alkyl- où le motif aryle est substitué par de un à cinq substituants choisis indépendamment parmi halogéno, cyano, nitro, C₁-C₆-alkyle, C₁-C₆halogénoalkyle ou C₁-C₆alcoxy ;
R¹⁰ est C₁-C₁₀alkyle, C₃-C₁₀cycloalkyle, C₃-C₁₀cycloalkyl-C₁-C₁₀alkyl-, C₁-C₁₀halogénoalkyle, C₂-C₁₀alcényle, C₂-C₁₀-alcynyle, C₁-C₄alcoxy-C₁-C₁₀alkyl-, C₁-C₄alkylthio-C₁-C₄-alkyl-, C₁-C₁₀alcoxy, C₂-C₁₀alcényloxy, C₂-C₁₀alcynyloxy, C₁-C₁₀alkylthio-, N-C₁-C₄alkyl-amino-, N,N-di-(C₁-C₄alkyl)-amino-, aryle ou aryle substitué par de un à trois R¹⁴, pouvant être identiques ou différents, hétéroaryle ou hétéroaryle substitué par de un à trois R¹⁴, pouvant être identiques ou différents, aryl-C₁-C₄alkyle ou aryl-C₁-C₄alkyl- où le motif aryle est substitué par de un à trois R¹⁴, pouvant être identiques ou différents, hétéroaryl-C₁-C₄alkyl- ou hétéroaryl-C₁-C₄alkyl- où le motif hétéroaryle est substitué par de un à trois R¹⁴, pouvant être identiques ou différents, aryloxy- ou aryloxy- substitué par de un à trois R¹⁴, pouvant être identiques ou différents, hétéroaryloxy-ou hétéroaryloxy- substitué par de un à trois R¹⁴, pouvant être identiques ou différents, arylthio- ou arylthio- substitué par de un à trois R¹⁴, pouvant être identiques ou différents, ou hétéroarylthio- ou hétéroarylthio- substitué par de un à trois R¹⁴, pouvant être identiques ou différents ;
chaque R¹¹ est indépendamment C₁-C₁₀alkyle ou phényle ou phényle substitué par de un à cinq substituants choisis indépendamment parmi halogéno, cyano, nitro, C₁-C₆alkyle, C₁-C₆halogénoalkyle ou C₁-C₆alcoxy ;
R¹² est C₁-C₁₀alkyle ou phényle ou phényle substitué par de un à cinq substituants choisis indépendamment parmi halogéno, cyano, nitro, C₁-C₆alkyle, C₁-C₆halogénoalkyle ou C₁-C₆alcoxy ;
chaque R¹³ est indépendamment halogéno, cyano, nitro, C₁-C₆alkyle, C₁-C₆halogénoalkyle ou C₁-C₆alcoxy ; et chaque R¹⁴ est indépendamment halogéno, cyano, nitro, C₁-C₁₀alkyle, C₁-C₄halogénoalkyle, C₁-C₁₀alcoxy, C₁-C₄-alcoxycarbonyl-, C₁-C₄halogénoalcoxy, C₁-C₁₀alkylthio-, C₁-C₄halogénoalkylthio-, C₁-C₁₀alkylsulfinyl-, C₁-C₄-halogénoalkylsulfinyl-, C₁-C₁₀alkylsulfonyl-, C₁-C₄-halogénoalkylsulfonyl-, aryle ou aryle substitué par de un à cinq substituants choisis indépendamment parmi halogéno, cyano, nitro, C₁-C₆alkyle, C₁-C₆halogénoalkyle ou C₁-C₆alcoxy, ou hétéroaryle ou hétéroaryle substitué par de un à quatre substituants choisis indépendamment parmi halogéno, cyano, nitro, C₁-C₆alkyle, C₁-C₆-halogénoalkyle ou C₁-C₆alcoxy ;
ou des sels ou N-oxydes de celui-ci ;
un composé de formule (IIc), 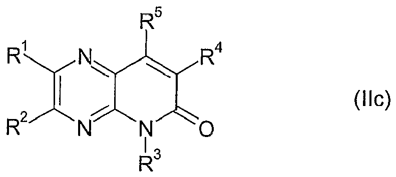 dans laquelle
R¹ et R² sont indépendamment hydrogène, C₁-C₄alkyle, C₁-C₄halogénoalkyle, halogéno, cyano, hydroxy, C₁-C₄alcoxy, C₁-C₄alkylthio, aryle ou aryle substitué par de un à cinq R⁶, pouvant être identiques ou différents, ou hétéroaryle ou hétéroaryle substitué par de un à cinq R⁶, pouvant être identiques ou différents ;
R³ est C₁-C₄halogénoalkyle, C₂-C₄halogénoalcényle ou C₂-C₄halogénoalcynyle ;
R⁴ est aryle ou aryle substitué par de un à cinq R⁸, pouvant être identiques ou différents, ou hétéroaryle ou hétéroaryle substitué par de un à quatre R⁸, pouvant être identiques ou différents ;
R⁵ est hydroxy ou un groupement pouvant être métabolisé en groupement hydroxy ;
chaque R⁶ et R⁸ est indépendamment halogéno, cyano, nitro, C₁-C₁₀alkyle, C₁-C₄halogénoalkyle, C₂-C₁₀alcényle, C₂-C₁₀alcynyle, hydroxy, C₁-C₁₀alcoxy, C₁-C₄halogéno-alcoxy, C₁-C₁₀alcoxy-C₁-C₄alkyl-, C₃-C₇cycloalkyle, C₃-C₇-cycloalcoxy, C₃-C₇cycloalkyl-C₁-C₄alkyl-, C₃-C₇-cycloalkyl-C₁-C₄alcoxy-, C₁-C₆alkylcarbonyl-, formyle, C₁-C₄alcoxycarbonyl-, C₁-C₄alkylcarbonyloxy-, C₁-C₁₀-alkylthio-, C₁-C₄halogénoalkylthio-, C₁-C₁₀-alkylsulfinyl-, C₁-C₄halogénoalkylsulfinyl-, C₁-C₁₀-alkylsulfonyl-, C₁-C₄halogénoalkylsulfonyl-, amino, C₁-C₁₀alkylamino-, di-C₁-C₁₀alkylamino-, C₁-C₁₀-alkylcarbonylamino-, aryle ou aryle substitué par de un à trois R¹³, pouvant être identiques ou différents, hétéroaryle ou hétéroaryle substitué par de un à trois R¹³, pouvant être identiques ou différents, aryl-C₁-C₄alkyl- ou aryl-C₁-C₄alkyl- où le motif aryle est substitué par de un à trois R¹³, pouvant être identiques ou différents, hétéroaryl-C₁-C₄alkyl- ou hétéroaryl-C₁-C₄alkyl- où le motif hétéroaryle est substitué par de un à trois R¹³, pouvant être identiques ou différents, aryloxy- ou aryloxy- substitué par de un à trois R¹³, pouvant être identiques ou différents, hétéroaryloxy-ou hétéroaryloxy- substitué par de un à trois R¹³, pouvant être identiques ou différents, arylthio- ou arylthio- substitué par de un à trois R¹³, pouvant être identiques ou différents, ou hétéroarylthio- ou hétéroarylthio- substitué par de un à trois R¹³, pouvant être identiques ou différents ; et
chaque R¹³ est indépendamment halogéno, cyano, nitro, C₁-C₆alkyle, C₁-C₆halogénoalkyle ou C₁-C₆alcoxy ;
ou un sel ou N-oxyde de celui-ci ;
un composé de formule (IId), 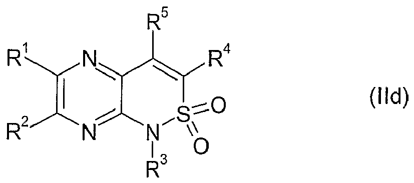 dans laquelle
R¹ et R² sont indépendamment hydrogène, C₁-C₄alkyle, C₁-C₄halogénoalkyle, halogéno, cyano, hydroxy, C₁-C₄alcoxy, C₁-C₄alkylthio, aryle ou aryle substitué par de un à cinq R⁶, pouvant être identiques ou différents, ou hétéroaryle ou hétéroaryle substitué par de un à cinq R⁶, pouvant être identiques ou différents ;
R³ est hydrogène, C₁-C₁₀alkyle, C₁-C₄halogénoalkyle, C₂-C₁₀alcényle, C₂-C₄halogénoalcényle, C₂-C₁₀alcynyle, C₂-C₄-halogénoalcynyle, C₃-C₁₀cycloalkyle, C₃-C₁₀cycloalkyl-C₁-C₆alkyl-, C₁-C₁₀alcoxy-C₁-C₆alkyl-, C₁-C₁₀cyanoalkyl-, C₁-C₁₀alcoxycarbonyl-C₁-C₆alkyl-, N-C₁-C₃alkyl-amino-carbonyl-C₁-C₆alkyl-, N,N-di(C₁-C₃alkyl)-aminocarbonyl-C₁-C₆alkyl-, aryl-C₁-C₆alkyl- ou aryl-C₁-C₆alkyl- où le motif aryle est substitué par de un à trois R⁷, pouvant être identiques ou différents, ou hétérocyclyl-C₁-C₆-alkyl- ou hétérocyclyl-C₁-C₆alkyl- où le motif hétérocyclyle est substitué par de un à trois R⁷, pouvant être identiques ou différents ;
R⁴ est aryle ou aryle substitué par de un à cinq R⁸, pouvant être identiques ou différents, ou hétéroaryle ou hétéroaryle substitué par de un à quatre R⁸, pouvant être identiques ou différents ;
R⁵ est hydroxy ou un groupement pouvant être métabolisé en groupement hydroxy ;
chaque R⁶, R⁷ et R⁸ est indépendamment halogéno, cyano, nitro, C₁-C₁₀alkyle, C₁-C₄halogénoalkyle, C₂-C₁₀alcényle, C₂-C₁₀alcynyle, hydroxy, C₁-C₁₀alcoxy, C₁-C₄-halogénoalcoxy, C₁-C₁₀alcoxy-C₁-C₄alkyl-, C₃-C₇-cycloalkyle, C₃-C₇cycloalcoxy, C₃-C₇cycloalkyl-C₁-C₄-alkyl-, C₃-C₇cycloalkyl-C₁-C₄alcoxy-, C₁-C₆alkyl-carbonyl-, formyle, C₁-C₄alcoxycarbonyl-, C₁-C₄alkyl-carbonyloxy-, C₁-C₁₀alkylthio-, C₁-C₄halogénoalkylthio-, C₁-C₁₀alkylsulfinyl-, C₁-C₄halogénoalkylsulfinyl-, C₁-C₁₀-alkylsulfonyl-, C₁-C₄halogénoalkylsulfonyl-, amino, C₁-C₁₀alkylamino-, di-C₁-C₁₀alkylamino-, C₁-C₁₀-alkylcarbonylamino-, aryle ou aryle substitué par de un à trois R¹³, pouvant être identiques ou différents, hétéroaryle ou hétéroaryle substitué par de un à trois R¹³, pouvant être identiques ou différents, aryl-C₁-C₄alkyl- ou aryl-C₁-C₄alkyl- où le motif aryle est substitué par de un à trois R¹³, pouvant être identiques ou différents, hétéroaryl-C₁-C₄alkyl- ou hétéroaryl-C₁-C₄alkyl- où le motif hétéroaryle est substitué par de un à trois R¹³, pouvant être identiques ou différents, aryloxy- ou aryloxy- substitué par de un à trois R¹³, pouvant être identiques ou différents, hétéroaryloxy-ou hétéroaryloxy- substitué par de un à trois R¹³, pouvant être identiques ou différents, arylthio- ou arylthio- substitué par de un à trois R¹³, pouvant être identiques ou différents, ou hétéroarylthio- ou hétéroarylthio- substitué par de un à trois R¹³, pouvant être identiques ou différents ; et
chaque R¹³ est indépendamment halogéno, cyano, nitro, C₁-C₆alkyle, C₁-C₆halogénoalkyle ou C₁-C₆alcoxy ;
ou un sel ou N-oxyde de celui-ci ;
un composé de formule (IIc), 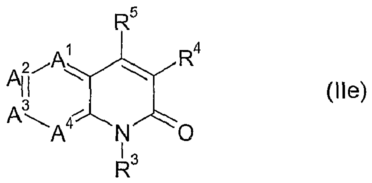 dans laquelle
A¹, A², A³ et A⁴ sont indépendamment C-R¹ ou N, à condition qu'au moins l'un parmi A¹, A², A³ et A⁴ soit N, et à condition que si A¹ et A⁴ sont tous deux N, A² et A³ ne soient pas tous deux C-R¹ ;
chaque R¹ est indépendamment hydrogène, C₁-C₄alkyle, C₁-C₄halogénoalkyle, halogéno, cyano, hydroxy, C₁-C₄alcoxy, C₁-C₄alkylthio, aryle ou aryle substitué par de un à cinq R⁶, pouvant être identiques ou différents, ou hétéroaryle ou hétéroaryle substitué par de un à cinq R⁶, pouvant être identiques ou différents ;
R³ est hydrogène, C₁-C₁₀alkyle, C₁-C₄halogénoalkyle, C₂-C₁₀alcényle, C₂-C₄halogénoalcényle, C₂-C₁₀alcynyle, C₂-C₄-halogénoalcynyle, C₃-C₁₀cycloalkyle, C₃-C₁₀cycloalkyl-C₁-C₆alkyl-, C₁-C₁₀alcoxy-C₁-C₆alkyl-, C₁-C₁₀cyanoalkyl-, C₁-C₁₀alcoxycarbonyl-C₁-C₆alkyl-, N-C₁-C₃alkyl-amino-carbonyl-C₁-C₆alkyl-, N,N-di(C₁-C₃alkyl)-aminocarbonyl-C₁-C₆alkyl-, aryl-C₁-C₆alkyl- ou aryl-C₁-C₆alkyl- où le motif aryle est substitué par de un à trois R⁷, pouvant être identiques ou différents, ou hétérocyclyl-C₁-C₆alkyl- ou hétérocyclyl-C₁-C₆alkyl- où le motif hétérocyclyle est substitué par de un à trois R⁷, pouvant être identiques ou différents ;
R⁴ est aryle ou aryle substitué par de un à cinq R⁸, pouvant être identiques ou différents, ou hétéroaryle ou hétéroaryle substitué par de un à quatre R⁸, pouvant être identiques ou différents ;
R⁵ est hydroxy ou un groupement pouvant être métabolisé en groupement hydroxy ;
chaque R⁶, R⁷ et R⁸ est indépendamment halogéno, cyano, nitro, C₁-C₁₀alkyle, C₁-C₄halogénoalkyle, C₂-C₁₀alcényle, C₂-C₁₀alcynyle, hydroxy, C₁-C₁₀alcoxy, C₁-C₄halogéno-alcoxy, C₁-C₁₀alcoxy-C₁-C₄alkyl-, C₃-C₇cycloalkyle, C₃-C₇-cycloalcoxy, C₃-C₇cycloalkyl-C₁-C₄alkyl-, C₃-C₇-cycloalkyl-C₁-C₄alcoxy-, C₁-C₆alkylcarbonyl-, formyle, C₁-C₄alcoxycarbonyl-, C₁-C₄alkylcarbonyloxy-, C₁-C₁₀-alkylthio-, C₁-C₄halogénoalkylthio-, C₁-C₁₀alkyl-sulfinyl-, C₁-C₄halogénoalkylsulfinyl-, C₁-C₁₀-alkylsulfonyl-, C₁-C₄halogénoalkylsulfonyl-, amino, C₁-C₁₀alkylamino-, di-C₁-C₁₀alkylamino-, C₁-C₁₀alkyl-carbonylamino-, aryle ou aryle substitué par de un à trois R¹³, pouvant être identiques ou différents, hétéroaryle ou hétéroaryle substitué par de un à trois R¹³, pouvant être identiques ou différents, aryl-C₁-C₄alkyl- ou aryl-C₁-C₄alkyl- où le motif aryle est substitué par de un à trois R¹³, pouvant être identiques ou différents, hétéroaryl-C₁-C₄alkyl- ou hétéroaryl-C₁-C₄alkyl- où le motif hétéroaryle est substitué par de un à trois R¹³, pouvant être identiques ou différents, aryloxy- ou aryloxy- substitué par de un à trois R¹³, pouvant être identiques ou différents, hétéroaryloxy-ou hétéroaryloxy- substitué par de un à trois R¹³, pouvant être identiques ou différents, arylthio- ou arylthio- substitué par de un à trois R¹³, pouvant être identiques ou différents, ou hétéroarylthio- ou hétéroarylthio- substitué par de un à trois R¹³, pouvant être identiques ou différents ; et
chaque R¹³ est indépendamment halogéno, cyano, nitro, C₁-C₆alkyle, C₁-C₆halogénoalkyle ou C₁-C₆alcoxy ;
ou un sel ou N-oxyde de celui-ci ;
et un composé de formule (IIf), 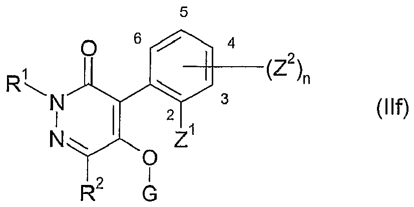 dans laquelle
R¹ est C₁-C₆ alkyle ou C₁-C₆alkyloxy-C₁-C₆alkyle ;
R² hydrogène ou C₁-C₆alkyle ;
G est hydrogène, -(C=L)R³, -(SO₂)R⁴ ou -(P=L)R⁵R⁶, où L est oxygène ou soufre ;
R³ est C₁-C₆alkyle, C₃-C₈cycloalkyle, C₂-C₆alcényle, C₂-C₆-alcynyle, C₆-C₁₀aryle, C₆-C₁₀aryl-C₁-C₆alkyl-, C₁-C₆-alkyloxy, C₃-C₈cycloalkyloxy, C₂-C₆alcényloxy, C₂-C₆-alcynyloxy, C₆-C₁₀aryloxy, C₆-C₁₀aryl-C₁-C₆alkyloxy-, amino, C₁-C₆alkylamino, C₂-C₆alcénylamino, C₆-C₁₀-arylamino, di (C₁-C₆alkyl)amino, di (C₂-C₆alcényl) amino, (C₁-C₆alkyl) (C₆-C₁₀aryl) amino ou un cycle hétérocyclique azoté de trois à huit chaînons ;
R⁴ est C₁-C₆alkyle, C₆-C₁₀aryle, un groupe C₁-C₆alkylamino ou di(C₁-C₆alkyl)amino ; et
R⁵ et R⁶ peuvent être identiques ou différents et sont indépendamment C₁-C₆alkyle, C₃-C₈cycloalkyle, C₂-C₆-alcényle, C₆-C₁₀aryle, C₁-C₆alkyloxy, C₃-C₈cycloalkyloxy, C₆-C₁₀aryloxy, C₆-C₁₀aryl-C₁-C₆alkyloxy, C₁-C₆alkylthio, C₁-C₆alkylamino ou di(C₁-C₆alkyl)amino ;
où tout groupement R³, R⁴, R⁵ et R⁶ peut être substitué par halogène, C₃-C₈cycloalkyle, C₆-C₁₀aryle, C₆-C₁₀aryl-C₁-C₆alkyl-, C₃-C₈cycloalkyloxy, C₆-C₁₀aryloxy, C₆-C₁₀aryl-C₁-C₆alkyloxy-, C₆-C₁₀arylamino, (C₁-C₆alkyl) (C₆-C₁₀aryl)-amino et un cycle hétérocyclique azoté de trois à huit chaînons pouvant être substitué par au moins un C₁-C₆alkyle ;
Z¹ est C₁-C₆alkyle ;
Z² est C₁-C₆alkyle ;
n vaut 0, 1, 2, 3 ou 4 ;
et chacun parmi Z² peut être identique ou différent lorsque n représente un nombre entier valant 2 ou plus,
et une somme du nombre d'atomes de carbone dans le groupement représenté par Z¹ et celui dans le groupement représenté par Z² est égale à 2 ou plus,
et/ou un herbicide inhibiteur de l'hydroxyphényl pyruvate dioxygénase (HPPD), où les plantes de culture comprennent au moins un polynucléotide hétérologue qui comprend une région qui code pour une HST et qui fournit une surexpression de ladite HST dans lesdites plantes de culture.

2. Méthode selon la revendication 1, **caractérisée en ce que** les plantes de culture contiennent un polynucléotide hétérologue supplémentaire qui comprend une région qui code pour une hydroxyphényl pyruvate dioxygénase (HPPD).

3. Méthode selon l'une quelconque des revendications précédentes, **caractérisée en ce que** la composition pesticide comprend un herbicide inhibiteur de l'homogentisate solanésyl transférase et un herbicide inhibiteur de l'hydroxyphényl pyruvate dioxygénase (HPPD).

4. Méthode selon l'une quelconque des revendications précédentes, **caractérisée en ce que** l'herbicide inhibiteur de l'hydroxyphényl pyruvate dioxygénase est choisi dans le groupe constitué par la mésotrione, la sulcotrione, l'isoxaflutole, la tembotrione, la topramézone, le benzofénap, le pyrazolate, le pyrazoxyfène, le pyrasulfotole, le kétospiradox ou l'acide libre de celui-ci, la 4-hydroxy-3-[[2-(2-méthoxyéthoxy)méthyl]-6-(trifluorométhyl)-3-pyridinyl]-carbonyl]-bicyclo[3.2.1]oct-3-èn-2-one, la [2-chloro-3-(2-méthoxyéthoxy)-4-(méthylsulfonyl)phényl](1-éthyl-5-hydroxy-1H-pyrazol-4-yl)-méthanone, l'a-(cyclopropyl-carbonyl)-2-(méthylsulfonyl)-β-oxo-4-(trifluorométhyl)-benzènepropanenitrile, et la (2,3-dihydro-3,3,4-triméthyl-1,1-dioxydobenzo[b]thién-5-yl)(5-hydroxy-1-méthyl-1H-pyrazol-4-yl)méthanone.

5. Méthode selon l'une quelconque des revendications précédentes, **caractérisée en ce que** l'enzyme homogentisate solanésyl transférase est dérivée d'*Arabidopsis thaliana, Glycine max, Oryza sativa* ou *Chlamydomonas reinhardtii.*

6. Méthode selon l'une quelconque des revendications précédentes, **caractérisée en ce que** l'enzyme homogentisate solanésyl transférase est choisie dans le groupe constitué par
(a) les enzymes HST telles qu'illustrées dans SEQ ID n° 1 à 10 ; et
(b) une HST qui comprend un ou plusieurs des motifs polypeptidiques suivants :
W-(R/K)-F-L-R-P-H-T-I-R-G-T ; et/ou
N-G-(Y/F)-I-V-G-I-N-Q-I-(Y/F)-D ; et/ou
I-A-I-T-K-D-L-P ; et/ou
Y-(R/Q)-(F/W)-(I/V)-W-N-L-F-Y.

7. Méthode selon l'une quelconque des revendications précédentes, **caractérisée en ce que** la plante de culture comprend un autre polynucléotide recombiné codant pour une autre enzyme de tolérance vis-à-vis d'herbicides.

8. Méthode selon la revendication 7, **caractérisée en ce que** l'autre enzyme de tolérance vis-à-vis d'herbicides est choisie dans le groupe constitué par la 5-énolpyruvylshikimate-3-phosphate synthase (EPSPS), la glyphosate acétyl transférase (GAT), le cytochrome P450, la phosphinothricine acétyltransférase (PAT), l'acétolactate synthase (ALS), la protoporphyrinogène oxydase (PPGO), la phytoène désaturase (PD), les enzymes de dégradation du dicamba, et les enzymes de dégradation d'herbicides à base d'aryloxy.

9. Méthode selon l'une quelconque des revendications précédentes, **caractérisée en ce que** la composition pesticide comprend un ou plusieurs autres herbicides.

10. Méthode selon la revendication 9, **caractérisée en ce que** les un ou plusieurs autres herbicides sont choisis dans le groupe constitué par le glyphosate (y compris les sels de celui-ci acceptables sur le plan agrochimique) ; le glufosinate (y compris les sels de celui-ci acceptables sur le plan agrochimique) ; les chloroacétanilides par exemple l'alachlor, l'acétochlor, le métolachlor, le S-métolachlor ; les inhibiteurs du photosystème II, par exemple les triazines telles que l'amétryne, l'atrazine, la cyanazine et la terbuthylazine, les triazinones telles que l'hexazinone et la métribuzine, et les urées telles que le chlorotoluron, le diuron, l'isoproturon, le linuron et le tébuthiuron ; les inhibiteurs d'ALS, par exemple les sulfonylurées telles que l'amidosulfuron, le chlorsulfuron, le flupyrsulfuron, l'halosulfuron, le nicosulfuron, le primisulfuron, le prosulfuron, le rimsulfuron, le triasulfuron, le trifloxysulfuron et le tritosulfuron ; les diphényléthers, par exemple l'acifluorfen et le fomésafen.

11. Méthode selon la revendication 10, **caractérisée en ce que** les un ou plusieurs autres herbicides sont le glyphosate et/ou un herbicide inhibiteur du PS-II.

12. Méthode selon l'une quelconque des revendications précédentes, comprenant en outre l'application au lieu d'un insecticide et/ou d'un fongicide.

13. Méthode de fourniture d'une plante transgénique qui est tolérante vis-à-vis d'herbicides inhibiteurs de l'homogentisate solanésyl transférase (HST) et/ou inhibiteurs de l'hydroxyphényl pyruvate dioxygénase (HPPD), comprenant la transformation d'un matériau végétal par un polynucléotide recombiné comprenant une région qui code pour une HST et, éventuellement, une région qui code pour une HPPD, la sélection du matériau végétal transformé à l'aide d'un inhibiteur de HST choisi dans le groupe constitué par les formules (IIa), (IIb), (IIc), (IId), (IIe) et (IIf) telles que définies selon la revendication 1 et/ou d'un inhibiteur de HPPD, et la régénération de ce matériau dans une plante fertile morphologiquement normale.

14. Méthode selon la revendication 13, **caractérisée en ce que** le polynucléotide recombiné comprend en outre une région codant pour la cible d'un herbicide non inhibiteur de l'homogentisate solanésyl transférase et/ou une région codant pour une protéine capable de conférer à un matériau végétal transformé par la région une résistance vis-à-vis d'insectes, de champignons et/ou de nématodes.
